(19) Europäisches Patentamt
European Patent Office
Office européen des brevets

(11) Veröffentlichungsnummer: **0 335 315 B1**

(12) **EUROPÄISCHE PATENTSCHRIFT**

(45) Veröffentlichungstag der Patentschrift: **22.06.94**

(51) Int. Cl.5: **C07D 311/24**, A61K 31/35

(21) Anmeldenummer: **89105427.2**

(22) Anmeldetag: **28.03.89**

Die Akte enthält technische Angaben, die nach dem Eingang der Anmeldung eingereicht wurden und die nicht in dieser Patentschrift enthalten sind.

(54) **Neue Alkanophenone.**

(30) Priorität: **29.03.88 CH 1186/88**
**14.10.88 CH 3857/88**

(43) Veröffentlichungstag der Anmeldung:
**04.10.89 Patentblatt 89/40**

(45) Bekanntmachung des Hinweises auf die Patenterteilung:
**22.06.94 Patentblatt 94/25**

(84) Benannte Vertragsstaaten:
**AT BE CH DE ES FR GB GR IT LI LU NL SE**

(56) Entgegenhaltungen:
**EP-A- 0 017 332**
**EP-A- 0 079 637**
**EP-A- 0 139 809**
**EP-A- 0 147 217**
**EP-A- 0 150 447**
**EP-A- 0 228 045**
**US-A- 4 546 194**

(73) Patentinhaber: **CIBA-GEIGY AG**
**Klybeckstrasse 141**
**CH-4002 Basel(CH)**

(72) Erfinder: **von Sprecher, Andreas, Dr.**
**Stallenmattstrasse 22**
**CH-4104 Oberwil(CH)**
Erfinder: **Beck, Andreas, Dr.**
**Reutebachgasse 40**
**D-7800 Freiburg(DE)**

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

In der EP-A-228 045 werden unsymmetrische alpha-Hydroxythioether offenbart, die als Leukotrien-Antagonisten wirksam sind und sich zur Behandlung von allergischen Zuständen eignen. In den Dokumenten EP-A-017 332, EP-A-079 637, US-A-4 546 194, EP-A-139 809 und EP-A-150 447 werden SRS-A- bzw. Leukotrien-Antagonisten beschrieben, die als gemeinsames Strukturmerkmal einen Alkanophenonrest enthalten, der über eine Kette mit einer 4-Oxo-4H-1-benzopyrangruppe verbunden ist.

Die Erfindung betrifft neue substituierte Alkanophenone der allgemeinen Formel I,

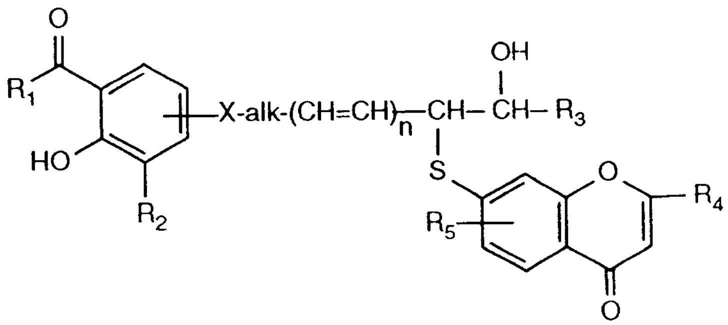

(I)

worin $R_1$ Niederalkyl oder Mono-, Di- oder Polyfluorniederalkyl bedeutet, $R_2$ Wasserstoff, Niederalkyl, Niederalkenyl oder Mono-, Di- oder Polyfluorniederalkyl darstellt, X $C_1$-$C_3$-Alkylen, Oxy oder Thio darstellt, alk Niederalkylen bedeutet, n für 1 oder 2 steht, $R_3$ für unsubstituiertes oder durch Niederalkyl, Niederalkoxy, Halogen, Carboxy, Niederalkoxycarbonyl, Amino, N-Mono- oder N,N-Diniederalkylamino, Carbamoyl, N-Mono- oder N,N-Diniederalkylcarbamoyl und/oder Trifluormethyl substituiertes Phenyl; oder für Niederalkyl, Mono-, Di- oder Trifluorniederalkyl, Carboxy-niederalkyl, Niederalkoxycarbonyl-niederalkyl, Carbamoyl-niederalkyl oder N-Mono- oder N,N-Diniederalkylcarbamoyl-niederalkyl steht, $R_4$ Carboxy, Niederalkoxycarbonyl, 5-Tetrazolyl, Carbamoyl, N-Mono- oder N,N-Diniederalkylcarbamoyl oder unsubstituiertes oder in Phenylteil durch Niederalkyl, Niederalkoxy, Halogen und/oder Trifluormethyl substituiertes N-(Benzolsulphonyl)carbamoyl bedeutet, und $R_5$ für Wasserstoff oder Niederalkyl steht; wobei mit "nieder" bezeichnete Reste nicht mehr als 7 Kohlenstoffatome aufweisen; und ihre Salze,
Verfahren zu ihrer Herstellung, sie als Wirkstoff enthaltende pharmazeutische Präparate und ihre Verwendung als Arzneimittelwirkstoffe.

Die Raumdarstellung in der obigen Formel I ist für die bevorzugten Verbindungen, in weichen das O-Atom der Hydroxylgruppe mit dem S-Atom in der relativen trans-Konfiguration sind, so zu verstehen, dass die Symbole der ersten Zeile oberhalb, die der dritten Zeile dann unterhalb der Darstellungsebene liegen (oder umgekehrt), was für die abgebildete Formel der entgegengesetzten Konfiguration, (RS)-(SR), gemäss der Kahn-Ingold-Prelog-Konvention an dem mit dem Schwefelatom verbundenen Kohlenstoffatom, (C-S-), und dem die Hydroxygruppe tragenden Kohlenstoffatom, (C-OH), entspricht. Dabei sind, wenn n für 2 steht, die Enantiomeren mit S(C-S-), R(C-OH)-Konfiguration und, wenn n für 1 steht, die Enantiomeren mit R(C-S-), S(C-OH)-Konfiguration besonders bevorzugt. In durch das Symbol $-(CH=CH)_n$ dargestellten Vinylen- bzw. Buta-1,3-dienylenrest liegt die Doppelbindung bzw. die von dem mit dem Rest alk verbundenen C-Atom ausgehende Doppelbindung des Butadienylenrestes vorzugs-, jedoch nicht notwendigerweise in cis-Konfiguration, üblicherweise mit (Z) bezeichnet, vor, wobei die andere Doppelbindung dann vorzugs-, jedoch ebenfalls nicht notwendigerweise trans-Konfiguration, üblicherweise mit (E) bezeichnet, besitzt.

Im Phenylteil gegebenenfalls wie angegeben substituiertes N-(Benzolsulphonyl)-carbamoyl ist beispielsweise unsubstituiert oder, vorzugsweise in ortho-Stellung, monosubstituiert.

Vor und nachstehend werden unter "niederen" Resten und Verbindungen solche verstanden, die nicht mehr als 7 und, wenn nicht anders angegeben, vorzugsweise nicht mehr als 4 Kohlenstoffatome (C-Atome) aufweisen. Ferner gilt:
Niederalkyl ist beispielsweise $C_1$-$C_7$-Alkyl, vor allem geradkettiges $C_1$-$C_4$-Alkyl, wie Methyl, Aethyl, Propyl, Isopropyl, Butyl oder Sekundärbutyl, kann aber auch ein verzweigtes $C_1$-$C_4$-Alkyl, wie Isobutyl oder Tertiärbutyl oder Pentyl-, Hexyl- oder Heptylrest sein. Niederalkyl $R_1$, $R_5$ bzw. als Substituent von Phenyl bzw. N-(Benzolsulphonyl)carbamoyl ist vorzugsweise $C_1$-$C_4$-Alkyl, z.B. Methyl, Niederalkyl $R_2$ vorzugsweise $C_2$-$C_5$-Alkyl, z.B. Propyl, und Niederalkyl $R_3$ vorzugsweise $C_3$-$C_7$-Alkyl, z.B. Propyl, Butyl oder Pentyl.

Mono-, Di- oder Polyfluorniederalkyl weist beispielsweise bis und mit 5 Fluoratome auf und ist beispielsweise Mono-, Di- oder Trifluor-$C_1$-$C_7$-alkyl, vor allem ω-Fluor- oder ω,ω,ω-Trifluor-$C_1$-$C_4$-alkyl, wie Trifluormethyl, 2,2,2-Trifluoräthyl oder 3,3,3-Trifluorpropyl. Fluoriertes Niederalkyl $R_1$ sowie solches als Substituent von Phenyl $R_3$ ist insbesondere Trifluormethyl und fluoriertes Niederalkyl $R_3$ vorzugsweise ω,ω,ω-Trifluor-$C_2$-$C_4$-alkyl, z.B. 3,3,3-Trifluorpropyl.

Niederalkenyl $R_2$ ist beispielsweise $C_2$-$C_4$-Alkenyl, wie Vinyl, Prop-1-enyl oder insbesondere Prop-2-enyl (Allyl).

Niederalkylen ist beispielsweise geradkettiges $C_1$-$C_7$-Alkylen, im Falle von X insbesondere $C_1$-$C_3$-Alkylen, wie Methylen oder Aethylen, und im Falle von alk insbesondere $C_2$-$C_6$-Alkylen, wie Aethylen, 1,3-Propylen, 1,4-Butylen, ferner 1,5-Pentylen oder 1,6-Hexylen.

Niederalkoxy ist beispielsweise $C_1$-$C_4$-Alkoxy, wie Methoxy.

Niederalkoxycarbonyl ist beispielsweise $C_1$-$C_4$-Alkoxycarbonyl, wie Methoxy-, Aethoxy-, Propyloxy- oder Butyloxycarbonyl.

Niederalkylamino ist beispielsweise $C_1$-$C_4$-Alkylamino, wie Methyl-, Aethyl-, Propyl- oder Isopropylamino.

Diniederalkylamino ist beispielsweise Di-$C_1$-$C_4$-alkylamino, wie Dimethylamino, Diäthylamino oder N-Aethyl-N-methyl-amino.

N-Mono- oder N,N-Diniederalkylcarbamoyl ist beispielsweise N-$C_1$-$C_4$-Alkyl- oder N,N-Di-$C_1$-$C_4$-alkylcarbamoyl, wie N-Methyl-, N-Aethyl-oder N,N-Dimethylcarbamoyl.

Halogen ist beispielsweise Halogen der Atomnummer bis und mit 35, wie Fluor, Chlor oder Brom.

Die meisten Verbindungen der Formel I können, ihrem individuellen Charakter nach, auch in Form von Salzen vorliegen. Diejenigen davon, welche eine genügende Acidität haben, wie insbesondere die mit Carboxyl-, Tetrazolyl- oder Sulphamoylgruppen, können Salze mit Basen, wie insbesondere anorganischen Basen, vorzugsweise physiologisch verträgliche Alkalimetall-Salze, vor allem Natrium- und Kalium-Salze, bilden. In Betracht kommen aber auch Ammoniumsalze mit Ammoniak oder physiologisch verträglichen organischen Aminen, wie Mono-, Di- oder Triniederalkylaminen, z.B. Diäthylamin, Mono-, Di- oder Tri-(hydroxyalkyl)aminen, wie Tris(hydroxymethyl)methylamin, oder D-Glucosamin.

Die Verbindungen der Formel I und ihre Salze weisen vorteilhafte pharmakologische Eigenschaften, insbesondere einen ausgeprägten Leukotrienantagonismus auf.

So hemmen sie z.B. in vitro im Konzentrationsbereich von etwa 0,001-1,0 µMol/l die durch Leukotrien-$D_4$ ($LTD_4$) induzierte Kontraktion eines glatten Muskels. Dieser sogenannte $LTD_4$-Antagonismus wird experimentell z.B. folgendermassen ermittelt: In Segmenten, die dem Ileum eines 300-400 g schweren Meerschweinchens entnommen wurden und in einem Organbad in Tyrode-Lösung bei 38°C und unter Begasung mit einem Gemisch von 95 % Sauerstoff und 5 % Kohlenstoffdioxid bei einer Belastung von 1 g inkubiert wurden, werden mit synthetischem Leukotrien $D_4$ (als Kaliumsalz) Kontraktionen ausgelöst und isotonisch registriert. Das Ausmass der Hemmung durch die Prüfsubstanz wird nach einer Vorinkubation von 2 Minuten ermittelt und als $IC_{50}$, d.h. die Konzentration, welche die Testkontraktion um 50 % reduziert, ausgewertet. Die Verbindungen der Formel I sind auch in vivo ausgezeichnet wirksam. Sie weisen zudem den spezifischen und therapeutisch sehr bedeutsamen Vorteil einer verhältnismässig langen Wirkungsdauer auf. So konnte im in vivo Bronchokonstriktions-Standardtest am Meerschweinchen bei Aerosol-Verabreichung einer Lösung, enthaltend 0,0001 bis 1 % Gew.-% der Prüfsubstanz ein deutlicher $LTD_4$-antagonisierender Effekt nachgewiesen werden. (Die Beschreibung der Testmethode befindet sich im Anhang nach den Beispielen).

Ueberraschenderweise üben viele Verbindungen der Formel I auch eine ausgeprägte Hemmwirkung auf andere physiologisch wichtige Enzymsysteme aus. So wurde die Hemmung von Phospholipase $A_2$ aus menschlichen Leukocyten im getesteten Konzentrationsbereich von etwa 0,5-50 µMol/l beobachtet. (Die experimentelle Anordnung für diese Bestimmung ist im Anhang nach den Beispielen näher beschrieben.) Ebenfalls wurde die Hemmung von Phospholipase C aus menschlichen Thrombocyten im getesteten Konzentrationsbereich von etwa 1-100 µMol/l beobachtet.

Dank dieser wertvollen pharmakologischen Eigenschaften können die erfindungsgemässen Verbindungen der Formel I überall dort therapeutische Anwendung finden, wo die Wirkung der Leukotriene zu krankhaften Zuständen führt, und diese mildern oder beheben. Demzufolge können sie beispielsweise zur Behandlung allergischer Zustände und Erkrankungen, wie insbesondere Asthma, aber auch Heufieber sowie obstruktiver Lungenkrankheiten einschliesslich zystischer Fibrose, verwendet werden. Ebenfalls sind sie, dank ihrer antiinflammatorischen Wirksamkeit, als entzündungshemmende Mittel, insbesondere als externe (topische) Hautphlogistatika für die Behandlung entzündlicher Dermatosen jeglicher Genese, wie bei leichten Hautirritationen, Kontaktdermatitis, Exanthemen und Verbrennungen, sowie als Schleimhautphlogostatika für die Behandlung von Mukosaentzündungen, z.B. der Augen, Nase, Lippen, Mund und Genital-,

bzw. Analregion, geeignet. Ferner können sie als Sonnenschutzmittel verwendet werden. Die hohe hemmende Wirksamkeit auf verschiedene Blutfaktoren weist zudem auf die Möglichkeit der therapeutischen Anwendung der Verbindungen der Formel I im Indikationsbereich Thrombose und Blutgerinnung hin.

Bevorzugt betrifft die Erfindung Verbindungen der Formel I, worin $R_1$ $C_1$-$C_4$-Alkyl oder ω,ω,ω-Trifluor-$C_1$-$C_4$-alkyl bedeutet, $R_2$ Wasserstoff, $C_1$-$C_4$-Alkyl, $C_2$-$C_4$-Alkenyl oder ω,ω,ω-Trifluor-$C_1$-$C_4$-alkyl darstellt, X $C_1$-$C_3$-Alkylen, Oxy oder Thio darstellt, alk geradkettiges $C_2$-$C_6$-Alkylen darstellt, n für 1 oder 2 steht, $R_3$ für unsubstituiertes oder durch $C_1$-$C_4$-Alkyl, $C_1$-$C_4$-Alkoxy, Halogen der Atomnummer bis und mit 35, Trifluormethyl, Carboxy und/oder $C_1$-$C_4$-Alkoxycarbonyl substituiertes Phenyl; oder für $C_1$-$C_7$-Alkyl, ω,ω,ω-Trifluor-$C_2$-$C_5$-alkyl, Carboxy-$C_2$-$C_5$-alkyl oder $C_1$-$C_4$-Alkoxycarbonyl-$C_2$-$C_5$-alkyl steht, $R_4$ Carboxy oder N-(Benzolsulphonyl)carbamoyl bedeutet, und $R_5$ für Wasserstoff steht, und ihre Salze, insbesondere ihre pharmazeutisch verwendbaren Salze.

Von den oben genannten Verbindungen der Formel I sind insbesondere diejenigen bevorzugt, worin $R_1$ $C_1$-$C_4$-Alkyl oder ω,ω,ω-Trifluor-$C_1$-$C_4$-alkyl bedeutet, $R_2$ $C_1$-$C_4$-Alkyl, $C_2$-$C_4$-Alkenyl oder ω,ω,ω-Trifluor-$C_1$-$C_4$-alkyl bedeutet, und die Gruppe X in <u>para</u>-Stellung zur $R_1$-C(=O)-Gruppe gebunden ist, und Salze davon, insbesondere ihre pharmazeutisch verwendbaren Salze.

Besonders bevorzugt sind die Verbindungen der Formel Ia,

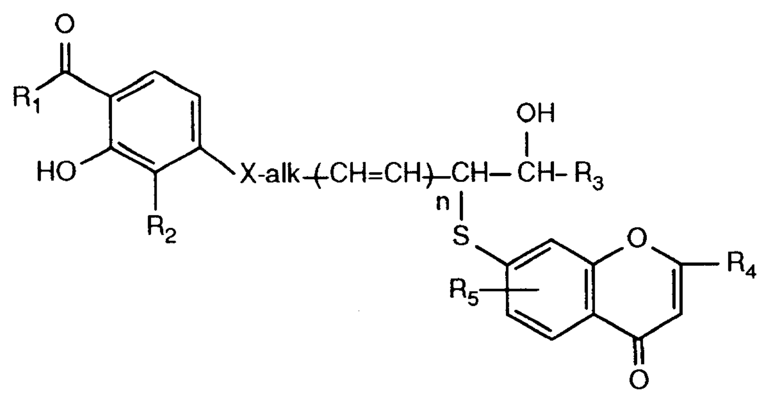

(Ia)

worin $R_1$ $C_1$-$C_4$-Alkyl bedeutet, $R_2$ $C_1$-$C_4$-Alkyl, $C_2$-$C_4$-Alkenyl oder ω,ω,ω-Trifluor-$C_1$-$C_4$-alkyl darstellt, X $C_1$-$C_3$-Alkylen, Oxy oder Thio darstellt, alk geradkettiges $C_2$-$C_6$-Alkylen darstellt, n für 1 oder 2 steht, $R_3$ eine Gruppe der Formel -A-$R_3$' darstellt, in der -A- für $C_1$-$C_4$-Alkylen, Phenylen oder eine direkte Bindung steht und $R_3$' $C_1$-$C_4$-Alkyl, Trifluormethyl, Carboxy oder $C_1$-$C_4$-Alkoxycarbonyl bedeutet, $R_4$ Carboxy oder N-(Benzolsulphonyl)carbamoyl bedeutet, und $R_5$ für Wasserstoff steht, und ihre Salze, insbesondere ihre pharmazeutisch verwendbaren Salze.

Vorzugsweise betrifft die Erfindung die Verbindungen der Formel Ia, worin $R_1$ $C_1$-$C_4$-Alkyl bedeutet, $R_2$ $C_1$-$C_4$-Alkyl darstellt, X für Oxy steht, alk $C_2$-$C_6$-Alkylen darstellt, n für 1 oder 2 steht, $R_3$ für durch $C_1$-$C_4$-Alkyl, $C_1$-$C_4$-Alkoxy, Halogen der Atomnummer bis und mit 35, Trifluormethyl oder $C_1$-$C_4$-Alkoxycarbonyl substituiertes Phenyl; oder für $C_2$-$C_8$-Alkyl, ω,ω,ω-Trifluor-$C_3$-$C_5$-alkyl oder $C_1$-$C_4$-Alkoxycarbonyl-$C_1$-$C_4$-alkyl steht, $R_4$ Carboxy bedeutet, und $R_5$ Wasserstoff ist, und ihre Salze, insbesondere ihre pharmazeutisch verwendbaren Salze.

Vor allen Dingen betrifft die Erfindung die Verbindungen der Formel Ia, worin $R_1$ $C_1$-$C_4$-Alkyl bedeutet, $R_2$ $C_1$-$C_4$-Alkyl darstellt, X für Oxy steht, alk $C_2$-$C_6$-Alkylen darstellt, n für 2 steht, $R_3$ eine Gruppe der Formel -A-$R_3$' darstellt, in der - A- für $C_1$-$C_4$-Alkylen oder Phenylen steht und $R_3$' $C_1$-$C_4$-Alkyl, Trifluormethyl oder $C_1$-$C_4$-Alkoxycarbonyl bedeutet, $R_4$ Carboxy oder N-(Benzolsulphonyl)carbamoyl bedeutet, und $R_5$ Wasserstoff ist, und ihre Salze, insbesondere ihre pharmazeutisch verwendbaren Salze.

Darüberhinaus sind generell die Verbindungen der Formel I bzw. Ia bevorzugt, worin X für Oxy steht, und ihre Salze, insbesondere ihre pharmazeutisch verwendbaren Salze.

Weiterhin sind generell die Verbindungen der Formel I bzw. Ia bevorzugt, worin n für 2 steht, und ihre Salze, insbesondere ihre pharmazeutisch verwendbaren Salze.

Ferner sind generell die Verbindungen der Formel I bzw. Ia bevorzugt, worin $R_3$ m-$C_1$-$C_4$-Alkylphenyl oder m-Trifluormethylphenyl, $R_4$ Carboxy und $R_5$ Wasserstoff bedeuten, und ihre Salze, insbesondere ihre pharmazeutisch verwendbaren Salze.

Weiterhin sind generell die Verbindungen der Formel I bzw. Ia bevorzugt, worin die dem Rest alk benachbarte Doppelbindung des Restes $-(CH=CH)_n-$ in (Z)-, d.h. cis-Konfiguration, und die gegebenenfalls

vorhandene zusätzliche Doppelbindung des Restes $-(CH=CH)_n-$ in (E)-, d.h. trans-Konfiguration, vorliegt, und ihre Salze, insbesondere ihre pharmazeutisch verwendbaren Salze.

Schliesslich sind generell die Verbindungen der Formel I bzw. Ia bevorzugt, worin das mit dem Schwefelatom verbundene Ketten-C-Atom (S)-Konfiguration und das die Hydroxygruppe tragende Ketten-C-Atom (R)-Konfiguration besitzt, und ihre Salze, insbesondere ihre pharmazeutisch verwendbaren Salze.

Die Erfindung betrifft namentlich die in den Beispielen genannten Verbindungen der Formel I und ihre Salze, insbesondere pharmazeutisch verwendbaren Salze.

Das erfindungsgemässe Verfahren zur Herstellung von Verbindungen der Formel I und ihrer Salze beruht auf an sich bekannten Methoden und ist dadurch gekennzeichnet, dass man ein Epoxid der Formel II,

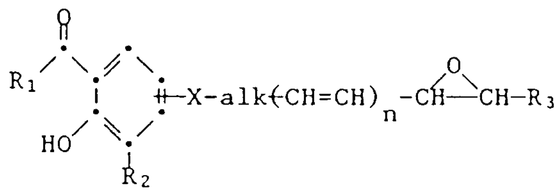

(II)

worin $R_1$, $R_2$, X, alk, n und $R_3$ obige Bedeutungen haben, mit einem Thiol der Formel III,

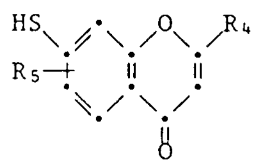

(III)

worin $R_4$ und $R_5$ obige Bedeutungen haben, oder einem Salz davon, umsetzt; und gewünschtenfalls eine verfahrensgemäss erhältliche Verbindung in eine andere Verbindung der Formel I umwandelt, ein verfahrensgemäss erhältliches Stereoisomerengemisch in die Komponenten auftrennt, und/oder eine verfahrensgemäss erhältliche freie Verbindung in ein Salz oder ein verfahrensgemäss erhältliches Salz in die freie Verbindung oder in ein anderes Salz überführt.

Die Umsetzung von Epoxiden II mit Thiolen III erfolgt unter Konfigurationsumkehr am die Bindung mit der Thiogruppe eingehenden C-Atom und unter Konfigurationserhaltung am die die Hydroxygruppe tragenden C-Atom. Um zu den bevorzugten Verbindungen mit entgegengesetzter Konfiguration an diesen beiden C-Atomen zu gelangen, geht man deshalb vorzugsweise von den entsprechenden trans-Epoxiden II aus. Dabei erhält man ausgehend von R,R-Epoxiden II Verbindungen I mit S(C-S-), R(C-OH)-Konfiguration bzw. ausgehend von S,S-Epoxiden II Verbindungen I mit R(C-S), S(C-OH)-Konfiguration. Die Umsetzung erfolgt unter an sich bekannten Bedingungen bei Temperaturen von etwa -20 °C bis etwa +50 °C, vorzugsweise bei Raumtemperatur, d.h. 18 °C bis 25 °C, und vornehmlich in einem basischen Milieu, beispielsweise in Gegenwart eines Amins, insbesondere eines tertiären aliphatischen, arylaliphatischen oder gesättigten heterocyclischen Amins, wie Trialkylamin (z.B. Triethylamin, oder Ethyl-diisopropylamin), Dialkyl-benzylamin (z.B. N,N-Dimethylbenzylamin), N,N-Dialkylanilin (z.B. N,N-Dimethylanilin) bzw. N-Methyl- oder N-Ethylpiperidin oder N,N'-Dimethylpiperazin. Ueblicherweise wird die Umsetzung in einem indifferenten organischen Lösungsmittel, wie einem Niederalkanol, z.B. Methanol oder Ethanol, durchgeführt.

In einer bevorzugten Ausführungsform geht man von Komponenten II und III aus, worin $R_4$ verestertes Carboxy oder Tetrazolyl und $R_3$ die angegebene Bedeutung hat und beispielsweise verestertes Carboxy oder gegebenenfalls fluoriertes Niederalkyl bedeutet, hydrolysiert $R_4$ (gegebenenfalls selektiv) zu Carboxy und überführt dieses gewünschtenfalls in amidiertes Carboxy.

Ausgangsstoffe für das erfindungsgemässe Verfahren sind entweder an sich bekannt oder nach bekannten Analogieverfahren in an sich bekannter Weise erhältlich.

Das als Ausgangsstoff verwendete Epoxid der oben definierten Formel II kann vornehmlich mittels derselben Verfahren hergestellt werden, welche auch bei der Synthese von Leukotrienen verwendet werden. Bei einem typischen allgemeinen Syntheseweg für Verbindungen II, worin n für 1 steht wird z.B. von einem Aldehyd der Formel

$O=CH-R_3$ (IV)

ausgegangen, worin A und $R_3$ die oben angegebenen Bedeutungen haben, wobei eine gegebenenfalls vorhandene freie Carboxylgruppe $R_3$ in einer als Ester geschützten Form, z.B. als Niederalkylester, vorliegt. Diese Verbindung wird mit Formylmethylentriphenylphosphoran (oder einem äquivalenten Reagens) kondensiert, wobei das entsprechende trans-3-$R_3$-Prop-2-enal der Formel

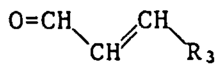

(V)

gebildet wird. Diese Verbindung wird anschliessend in an sich bekannter Weise, vorzugsweie unter schwach alkalischen Bedingungen (z.B. in Gegenwart von Alkalicarbonaten) mit wässrigem Wasserstoffperoxid epoxidiert, woraus ein trans, d.h. 2(RS),3(RS)-Epoxy-3-$R_3$-Propanal der Formel

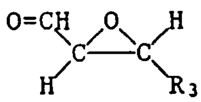

(VI)

resultiert. Der Epoxyaldehyd VI kann dann durch Kondensation mit einem Phosphoniumhalogenid

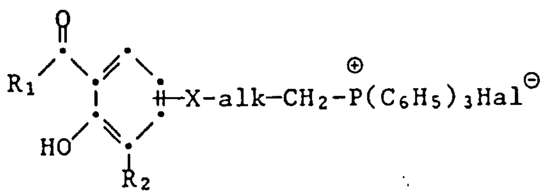

(VII)

worin $R_1$, $R_2$ und alk die angegebenen Bedeutungen haben und Hal ein Halogenatom vorzugsweise Brom, darstellt, und einer Base, z.B. Natriumamid, in Tetrahydrofuran zu dem entsprechenden Epoxid II, worin $R_4$ verestertes Carboxy bedeutet und n für 1 steht, umgesetzt werden.

Verbindungen VII werden insbesondere durch Umsetzung einer entsprechenden Verbindung der Formel

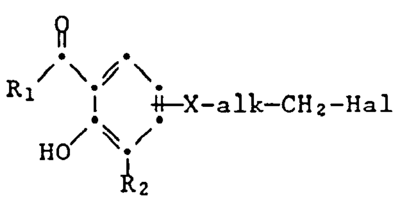

(VIII)

mit Triphenylphosphin in üblicher Weise hergestellt. Verbindungen VIII, worin X Oxy oder Thio bedeutet, werden beispielsweise erhalten, indem man entsprechende Verbindungen der Formeln

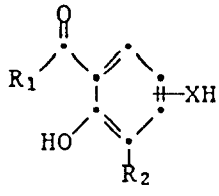

(IX)

und Hal-alk-$CH_2$-Hal (X)
in üblicher Weise miteinander kondensiert.

Eine andere Methode zur Herstellung von Verbindungen II besteht darin, dass man trans-3-$R_3$-Prop-2-enol der Formel

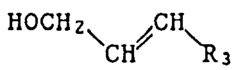 (XI),

worin $R_3$ obige Bedeutung hat, freies Carboxy als Substituent von $R_3$ aber vorzugsweise in einer Esterform geschützt ist, beispielsweise mittels Tertiärbutylhydroperoxid in Gegenwart von Titantetraisopropanolat und eines D- bzw. L-Weinsäurediniederalkylesters, epoxidiert, bei Verwendung eines D-Weinsäureesters erhält man dabei vorwiegend 2R,3R-Epoxy-3-$R_3$-propanol XIIa bzw. bei der Verwendung eines L-Weinsäureesters vorwiegend das entsprechende 2S,3S-Epoxy-3-$R_3$-propanol XIIb

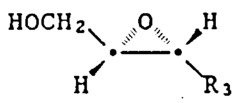 (XIIa) bzw. 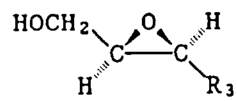 (XIIb).

Dieses wird dann, beispielsweise durch Behandeln mit Oxalylchlorid/Dimethylsulfoxid und anschliessend mit Triäthylamin, zum entsprechenden Epoxyaldehyd VI oxidiert, der dann mit dem entsprechenden Phosphoniumsalz VII in das entsprechende Epoxid II, worin $R_3$ verestertes Carboxy bedeutet und n für 1 steht, umgesetzt werden kann.

Dabei erhält man überwiegend Epoxide II, worin die Doppelbindung die bevorzugte cis-Stereotaxie besitzt. Verwendet man einen D-Weinsäureester erhält man, wie erwähnt, vorwiegend Verbindungen II, worin die Epoxygruppe R,R-Konfiguration aufweist, bzw. S,S-Enantiomere, wenn man die Umsetzung in Gegenwart von L-Weinsäureestern durchführt.

Die Herstellung von Epoxiden II, worin n für 2 steht, wird beispielsweise der Epoxyalkohol XIIa bzw. XIIb zunächst durch Behandlung mit N,N'-Dicyclohexylcarbodiimid und Dimethylsulfoxid in Gegenwart von Trifluoressigsäure und Pyridin und anschliessend mit Triphenylphosphoranylidenacetaldehyd zunächst in den entsprechende 4R,5R- bzw. 4S,5S-4,5-Epoxy-5-$R_3$-pent-2-enal der Formeln XIIIa bzw. XIIIb

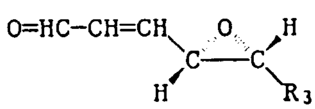 (XIIa) bzw. 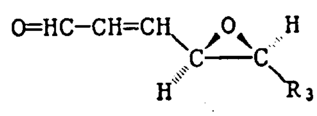 (XIIb)

überführt, der dann mit dem Phosphoniumhalogenid VII zur entsprechenden Epoxid II, worin n für 2 steht, weiter umgesetzt wird. Dabei werden vorzugsweise solche erhalten, in denen die mit dem Rest alk verbundene Doppelbindung cis-Stereotaxie und die mit dem Oxiranring verbundene Doppelbindung trans-Stereotaxie aufweist.

Verfahrensgemäss erhältliche Verbindungen kann man gewünschtenfalls in andere Verbindungen der Formel I überführen.

Beispielsweise kann man veresterte oder amidierte Carboxygruppen zu freiem Hydroxy hydrolisieren, vorzugsweise unter basischen Bedingungen, z.B. in Gegenwart von Natronlauge, und vorzugsweise in einem mit Wasser mischbaren organischen Lösungsmittel, wie Tetrahydrofuran, Dioxan oder einem Niederalkanol, wie Methanol oder Aethanol. Ausgehend von Verbindungen I, worin $R_4$ verestertes Carboxy, wie Niederalkoxycarbonyl bedeutet und $R_3$ solches als Substituenten enthält kann die Hydrolyse so gesteuert werden, dass selektiv nur $R_4$ oder sowohl $R_4$ als auch der Niederalkoxycarbonylsubstituent von $R_3$ zu Carboxy hydrolysiert werden. Verwendet man eine äquimolekulare Natronlauge und wählt milde Reaktionsbedingungen, z.B. etwa 0,5- bis 2-stündiges Rühren bei Raumtemperatur, wird praktisch nur Alkoxycarbonyl $R_4$ hydrolysiert, während unter drastischen Bedingungen, z.B. bei längeren Reaktionszeiten oder unter Erwärmen, sowohl $R_4$ als auch die Alkoxycarbonylgruppe in $R_3$ zu Carboxy hydrolysiert werden.

Umgekehrt kann man Carboxy $R_4$ sowie einen Carboxysubstituenten von $R_3$ in üblicher Weise verestern.

Freies oder verestertes Carboxy $R_4$ und solches als Substituent von $R_3$ kann ferner in üblicher Weise amidiert werden, beispielsweise durch Behandeln mit Ammoniak oder einem Mono- oder Diniederalkylamin. Beispielsweise kann man Carboxy $R_4$ in üblicher Weise, z.B. in Gegenwart eines Carbodiimidsalzes, z.B. von N-Aethyl-N'-(3-dimethylaminiopropyl)-carbodiimid-hydrochlorid, und 4-Dimethylaminopyridin, mit einem gegebenenfalls substituierten Benzolsulfonamid in die entsprechende N-Benzolsulfamidoylcarbamylgruppen überführen.

Selbstverständlich kann man auch erhaltene Diastereomerengemische auf Grund unterschiedlicher physikalischer Eigenschaften der Komponenten in die individuellen Komponenten auftrennen und/oder erhaltene Enantiomerengemische nach üblichen Racemattrennungsverfahren in die individuellen Enantiomeren auftrennen.

Wenn individuelle Diastereomere erwünscht sind, so kann vorteilhaft an beliebiger Stufe ein individuelles Diastereomeres eines Ausgangsstoffes eingesetzt werden oder aus einem in Form eines Diastereomeren vorliegenden Ausgangsstoff durch stereoselektive Reaktionsbedingungen oder optisch aktive Reagentien ein Diastereomeres bevorzugt gebildet werden, oder razemische Diastereomerengemische durch physikalische Trennmethoden, gegebenenfalls unter Anwendung optisch aktiver Hilfsstoffe, in die individuelle Diastereomere aufgetrennt werden.

In stereochemischer Hinsicht wird jedoch sowohl die erfindungsgemässe Kondensation der Komponenten II und III, wie auch die Herstellung der Ausgangsstoffe vornehmlich so durchgeführt, dass man jeweils stereotaktisch einheitliche Ausgangsstoffe einsetzt, die Reaktionen nach Möglichkeit stereoselektiv, z.B. durch Einsatz von stereotaktisch einheitlichen, optisch aktiven Reagentien und/oder Hilfsstoffen, durchführt und stereotaktisch einheitliche Produkte aus Reaktionsgemischen unmittelbar nach der Reaktion isoliert. So werden z.B. bei Herstellung der ungesättigten Ausgangsstoffe gegebenenfalls gebildete cis- und trans--Isomere sofort voneinander getrennt, wozu die üblichen physikalischen Trennungsmethoden, wie insbesondere Chromatographie, geeignet sind. In der Hauptreaktion wird vornehmlich das stereomere Epoxid II mit der im Endstoff bevorzugten Stereotaxie der Doppelbindung(en) und zwar in razemischer Form (wie es bei der Variante der Epoxydierung der Verbindung V mit Wasserstoffperoxid oft gebildet wird) oder vorzugsweise als individuelles Diastereomeres mit der im Endstoff I bevorzugten Konfiguration am (C-S-)-C-Atom entgegengesetzter Konfiguration am die Bindung mit dem S-Atom eingehenden Oxiran-C-atom verwendet.

Ebenso kann man erhaltene Salze, beispielsweise durch Säurebehandlung, in die freien Säuren bzw. erhaltene freie Säuren durch Basebehandlung in Salze überführen.

Infolge der engen Beziehung zwischen den neuen Verbindungen in freier Form und in Form ihrer Salze sind im voraussgegangenen und nachfolgend unter den freien Verbindungen oder ihren Salzen sinngemäss auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder unter den Reaktionsbedingungen bildet.

Die bei den erfindungsgemässen Verfahren und ihren Vorstufen auftretenden neuen Ausgangsstoffe und Zwischenprodukte bilden ebenfalls Gegenstand der Erfindung.

Vorzugsweise werden solche Ausgangsstoffe verwendet, und die Reaktionsbedingungen so gewählt, dass man zu den vorstehend als besonders bevorzugt aufgeführten Verbindungen gelangt.

Die vorliegende Erfindung betrifft ebenfalls pharmazeutische Zusammensetzungen und Arzneimittel, welche eine der erfindungsgemässen Verbindungen der Formel I oder ein pharmazeutisch verwendbares Salz davon enthalten. Bei den erfindungsgemässen pharmazeutischen Zusammensetzungen handelt es sich insbesondere um solche, die zur lokalen Verabreichung und vor allem zur Inhalationsverabreichung, z.B. in Form eines Aerosols, mikropulverisierten Pulvers oder einer feinversprühten Lösung, an Säuger, vor allem Menschen, bestimmt sind und den Wirkstoff allein oder zusammen mit einem pharmazeutisch anwendbaren Trägermaterial enthalten.

Pharmazeutische Präparate für topische und lokale Verwendung sind z.B. für die Behandlung der Haut Lotionen und Cremen, die eine flüssige oder semifeste Oel-in-Wasser- oder Wasser-in-Oel-Emulsion enthalten, und Salben (wobei solche vorzugsweise ein Konservierungsmittel enthalten). Für die Behandlung der Augen eignen sich Augentropfen, welche die aktive Verbindung in wässriger oder öliger Lösung enthalten, und Augensalben, die vorzugsweise in steriler Form hergestellt werden. Für die Behandlung der Nase sind Aerosole und Sprays (ähnlich den weiter unten beschriebenen für die Behandlung der Atemwege), grobe Puder, die durch schnelles Inhalieren durch die Nasenlöcher verabreicht werden, und vor allem Nasentropfen, welche die aktive Verbindung in wässriger oder öliger Lösung enthalten, geeignet; für die lokale Behandlung der Mundhöhle eignen sich auch Lutschbonbons, welche die aktive Verbindung in einer im allgemeinen aus Zucker und Gummiarabikum oder Tragaganth gebildeten Masse enthalten, welcher

Geschmacksstoffe beigegeben sein können, sowie Pastillen, die den Aktivstoff in einer inerten Masse, z.B. aus Gelatine und Glycerin oder Zucker und Gummiarabikum, enthalten.

Als pharmazeutische Zusammensetzungen für die Verabreichung in Form von Aerosolen oder Sprays sind geeignete z.B. Lösungen, Suspensionen oder Emulsionen des erfindungsgemässen Wirkstoffs der Formel I mit einem geeigneten pharmazeutisch annehmbaren Lösungsmittel, wie insbesondere Ethanol und Wasser, oder einem Gemisch solcher Lösungsmittel. Sie können nach Bedarf auch andere pharmazeutische Hilfsstoffe, wie nicht-ionische oder anionische oberflächenaktive Mittel, Emulgatoren und Stabilisatoren, sowie Wirkstoffe anderer Art enthalten und vor allem zweckmässig mit einem Treibgas, wie einem inerten Gas unter erhöhtem Druck oder insbesondere mit einer leicht flüchtigen, vorzugsweise unter normalem atmosphärischem Druck unterhalb der üblichen Raumtemperatur (z.B. zwischen etwa -30 und +10°C) siedenden Flüssigkeit, wie einem mindestens teilweise fluorierten polyhalogenierten Niederalkan, oder einem Gemisch solcher Flüssigkeiten, vermischt ist. Derartige pharmazeutische Zusammensetzungen, welche vorwiegend als Zwischenprodukte oder Vorratsgemische für die Herstellung der entsprechendenden Arzneimittel in fertiger Form verwendet werden, enthalten den Wirkstoff üblicherweise in einer Konzentration von etwa 0,1 bis etwa 10, insbesondere von etwa 0,3 bis etwa 3 Gewichts-%. - Zur Herstellung von Arzneimitteln in fertiger Form wird eine solche pharmazeutische Zusammensetzung in geeignete Behälter, wie Flacons und Druckflaschen, abgefüllt, welche mit einer für solche Zwecke geeigneten Versprühungseinrichtung bzw. Ventil versehen sind. Das Ventil ist vorzugsweise als ein Dosierungsventil konstruiert, welches bei der Betätigung eine vorbestimmte Menge der Flüssigkeit, entsprechend einer vorbestimmten Dosis des Wirkstoffs, freigibt. Bei Herstellung der fertigen Arzneimittelform kann man auch so vorgehen, dass entsprechende Mengen der als Vorratslösung vorliegenden pharmazeutischen Zusammensetzung und des Treibmittels separat in die Behälter abgefüllt werden und erst dort zur Vermischung kommen. Die Dosierung des zu verabreichenden Wirkstoffs der Formel I und die Häufigkeit der Verabreichung hängen ab von der jeweiligen Wirksamkeit bzw. der Länge der Wirkung jeder individueller dieser Verbindungen, von der Stärke der zu behandelnden Krankheit bzw. ihrer Symptome, sowie vom Geschlecht, Alter, Gewicht und individueller Ansprechbarkeit des zu behandelnden Säugers. Im Durchschnitt dürfte die empfohlene tägliche Dosis einer erfindungsgemässen Verbindung der Formel I bei einem 75 kg schweren Säuger (in erster Linie Menschen) im Bereich von etwa 10 bis etwa 500, vorzugsweise zwischen etwa 25 bis etwa 250 mg liegen, wobei die Verabreichung zweckmässig nach Bedarf in mehreren Dosen pro Tag erfolgen kann.

Die folgenden Beispiele illustrieren näher die vorliegende Erfindung, ohne sie in ihrem Umfang einzuschränken. Alle Temperaturen sind in Celsiusgraden angegeben.

Beispiel 1: (1R,2S)-1-Hydroxy-1-(3-trifluormethylphenyl)-8-(4-acetyl-3-hydroxy-2-propyl-phenoxy)-octa-3(E)-,5(Z)-dien-2-yl-7-thio-4-oxo-4H-1-benzopyran-2-carbonsäuremethylester

Die Lösung von 0.93 g (1R,2R)-1,2-Epoxy-1-(3-trifluormethylphenyl)-8-(4-acetyl-3-hydroxy-2-propyl-phenoxy)-octa-3(E),5(Z)-dien in 25 ml Methanol wird unter Argon mit 0.80 g Triäthylamin und 0.62 g 7-Mercaptochromon-2-carbonsäuremethylester 20 Stunden bei Raumtemperatur gerührt und eingedampft. Der Rückstand wird in Essigester gelöst und über Kieselgel filtriert. Das Filtrat wird 1-mal mit 2n-Salzsäure und 3-mal mit Sole gewaschen, über Magnesiumsulfat getrocknet und eingedampft Reinigung des Rückstandes durch Chromatographie an Kieselgel mit Hexan/Essigester (1:1) liefert die Titelverbindung vom Smp. 62-63°; $[\alpha]_D^{20}$ (Methanol, 0.135 %) = 103 ± 7.4° UV(Methanol): $\lambda_{max}$ ($\epsilon$) = 216 (50'000), 235/sh, 271 (27'940), 285/sh; 325 (12900).

Das Ausgangsmaterial wird z.B. wie folgt hergestellt:

a) (2R,3R)-2,3-Epoxy-3(3-trifluormethylphenyl)-propanol

Unter absolut wasserfreien Bedingungen und Argonatmosphäre wird die Lösung von 4.62 ml Tetraisopropyl-orthotitanat in 100 ml Methylenchlorid auf -70° gekühlt, mit 3,2 ml D(-) Weinsäurediäthylester und 5.45 g 3-(3-Trifluormethylphenyl)-prop-2(E)-enol in wenig Methylenchlorid versetzt. Nach 10-minütigem Rühren bei -70° werden 21,5 ml 3-molarer Tertiärbutylhydroperoxid-Lösung in Toluol zugegeben, wobei die Temperatur auf -60°C steigt. Man lässt die Temperatur innerhalb von 2 Stunden auf 0° steigen, giesst die resultierende gelbe Lösung langsam in eine Lösung von 14,5 g Eisen•II-sulfat und 5,8 g L(+)-Weinsäure in 60 ml Wasser (Kühlen!, exotherm) und rührt 30 Minuten bei 5-10°. Die wässrige Phase wird abgetrennt und mit Aether extrahiert. Die vereinigten organischen Extrakte werden über Natriumsulfat getrocknet und eingedampft. Der Rückstand wird in 90 ml Aether gelöst, auf 0-5° gekühlt, mit einer Suspension von 2.32 g Natriumhydroxid in 60 ml Sole versetzt und 1 Stunde bei 0-5° gerührt. Die wässrige Phase wird abgetrennt und mit Aether extrahiert. Die vereinigten Aetherphasen werden über Natriumsulfat getrocknet und eingedampft. Der Rückstand wir chromatographisch an Kieselgel mit Hexan/Essigester (3:2) gereinigt. Die Titelverbindung wird so als farbloses Oel erhalten; IR ($CH_2Cl_2$):

3550, 3430, 2950, 2880, 2830, 1310, 1150, 1110, 1050 $cm^{-1}$; $[\alpha]_D^{20}$ (Methanol, 0.175 %) = 42.3 ± 5.7°; $R_f$ = 0.30 (Hexan/Essigester 3:2).

b) (4R,5R)-4,5-Epoxy-5-(3-trifluormethylphenyl)-pent-2(E)-enal.

Die Lösung von 4.5 g (2R,3R)-2,3-Epoxy-3-(3-trifluormethylphenyl)-propanol in 105 ml Dimethylsulfoxid wird unter Argon mit 1.7 ml Pyridin, 0.77 ml Trifluoressigsäure und 12.75 g N,N-Dicyclohexylcarbodiimid 6 Stunden bei Raumtemperatur gerührt. Nach Zugabe von 8.25 g Formylmethylentriphenylphosphoran wird weitere 20 Stunden bei Raumtemperatur gerührt, mit 320 ml Essigester versetzt und nach 10 Minuten auf 320 ml Sole gegossen. Die entstandene Suspension wird 5 Minuten gerührt und filtriert. Im Filtrat wird die wässrige Phase 2-mal mit Essigester extrahiert. Die vereinigten organischen Phasen werden 3-mal mit Sole gewaschen, über Natriumsulfat getrocknet und eingedampft. Der Rückstand wird mit Aether/Hexan = (4:1) über Kieselgel filtriert. Das Filtrat wird eingedampft und der Rückstand durch Chromatographie an Kieselgel mit Hexan/Essigester (3:1) gereinigt. Die Titelverbindung wird so als hellgelbes Oel erhalten; IR ($CH_2Cl_2$): 2780, 2695, 1670, 1620, 1305, 1145, 1105 $cm^{-1}$ $R_f$ = 0.31 (Hexan/Essigester 3:1) $[\alpha]_D^{20}$ (Chloroform, 0.245 %) = 144.5 ± 4.1°

c) 3-(4-Acetyl-3-hydroxy-2-propylphenoxy)propyl-triphenylphosphoniumbromid

Die Lösung von 27 g 3-(4-Acetyl-3-hydroxy-2-propyl-phenoxy)propylbromid in 50 ml Toluol wird mit 21.85 g Triphenylphosphin 20 Stunden zum Rückfluss erhitzt. Die entstandene Suspension wird auf Raumtemperatur gekühlt, mit 200 ml Aether versetzt und 1 Stunde gerührt. Der farblose Niederschlag wird abgesaugt, mit Aether gewaschen und getrocknet. Die Titelverbindung zeigt einen Smp. von 211-212°.

d) (1R,2R)-1,2-Epoxy-1-(3-trifluormethylphenyl)-8-(4-acetyl-3-hydroxy-2-propylphenoxy)-octa-3(E),5(Z)-dien.

Die Suspension von 5.55 g 3-(4-Acetyl-3-hydroxy-2-propylphenoxy)propyltriphenylphosphoniumbromid in 80 ml Tetrahydrofuran wird mit 0.78 g $NaNH_2$ und 60 mg Kaliumtertiärbutanolat unter Argon 1 Stunde bei Raumtemperatur gerührt, auf 0-5° gekühlt, innerhalb von 5 Minuten mit 1.7 g (4R,5R)-4,5-Epoxy-5-(3-trifluormethylphenyl)-pent-2(E)-enal in 20 ml Tetrahydrofuran versetzt und anschliessend 2 Stunden bei Raumtemperatur gerührt. Die gebildete Suspension wird auf Phosphatpuffer (pH 7) gegossen und mit Aether extrahiert. Die vereinigten Aetherextrakte werden mit Phosphatpuffer pH 7 gewaschen, über Natriumsulfat getrocknet und eingedampft. Der Rückstand wird in Hexan/Essigester/Triäthylamin (24:71:5) aufgenommen und über mit diesem Lösungsmittelgemisch vorgewaschenes Kieselgel filtriert. Das Filtrat wird eingedampft und ergibt die Titelverbindung als hellgelbes Oel; $R_f$ = 0.75 (Hexan/Essigester 3:2).

Beispiel 2: (1R,2S)-1-Hydroxy-1-(3-trifluormethylphenyl)-8-(4-acetyl-3-hydroxy-2-propylphenoxy)-octa-3(E),5-(Z)-dien-2-yl-7-thio-4-oxo-4H-1-benzopyran-2-carbonsäure-Natriumsalz

0,7 g (1R,2S)-1-Hydroxy-1-(3-trifluormethylphenyl)-8-(4-acetyl-3-hydroxy-2-propylphenoxy)-octa-3(E),5-(Z)dien-2-yl-7-thio-4-oxo-4H-1-benzopyran-2-carbonsäuremethylester werden unter Argon in 20 ml Tetrahydrofuran gelöst, mit 5.1 ml 0,2n-Natronlauge versetzt und 1 Stunde bei Raumtemperatur gerührt. Eindampfen und chromatographische Reinigung des Rückstandes an einer "Reversed Phase"-Kieselgelsäule (z.B. Merck Lichroprep® RP-8) mit Methanol/Wasser (3:1) ergibt die Titelverbindung, Smp. 207-209°, $[\alpha]_D^{20}$ (0.54 %, Methanol) = 96.3 ± 1.9°

UV (Methanol): $\lambda_{max}$ ($\epsilon$) = 220 (488840), 235/sh, 267 (25940), 285 (22900), 324/sh.

Beispiel 3: (1S,2R)-1-Hydroxy-1-(3-trifluormethylphenyl)-8-(4-acetyl-3-hydroxy-2-propylphenoxy)-octa-3(E),5-(Z)-dien-2-yl-7-thio-4-oxo-4H-1-benzopyran-2-carbonsäuremethylester.

Die Titelverbindung wird analog zu Beispiel 1) aus (1S,2S)-1,2-Epoxy-1-(3-trifluormethylphenyl)-8-(4-acetyl-3-hydroxy-2-propylphenoxy)-octa-3(E),5(Z)-dien hergestellt; Smp. 68-69°.

Das Ausgangsmaterial wird z.B. wie folgt hergestellt:

a) (2S,3S)-2,3-Epoxy-3-(3-trifluormethylphenyl)-propanol

Die Titelverbindung wird wie in Beispiel 1a) beschrieben, aber unter Verwendung von L(+) Weinsäurediäthylester, hergestellt; farbloses Oel; IR ($CH_2Cl_2$): 3590, 3480, 2920, 2870, 1330, 1165, 1125, 1070 $cm^{-1}$; $[\alpha]_D^{20}$ (Methanol, 0.175 %) = -41.7 ± 5.7°; $R_f$ = 0.34 (Hexan/Essigester 1:1).

b) (4S,5S)-4,5-Epoxy-5-(3-trifluormethylphenyl)-pent-2(E)-enal.

Die Titelverbindung wird analog zu Beispiel 1b) aus dem Epoxyalkohol gemäss a) hergestellt; hellgelbes Oel; IR($CH_2Cl_2$): 2780, 2695, 1670, 1620, 1305, 1145, 1110 $cm^{-1}$; $[\alpha]_D^{20}$ (Chloroform, 0.15 %) = -158.0 ± 6.7°; $R_f$ = 0.4 (Hexan/Essigester 4:1).

c) (1S,2S)-1,2-Epoxy-1-(3-trifluormethylphenyl)-8-(4-acetyl-3-hydroxy-2-propylphenoxy)-octa-3(E),5(Z)-dien.
Die Titelverbindung wird analog zu Beispiel 1d) aus dem Epoxyaldehyd gemäss b) hergestellt; hellbraunes Oel; $R_f$ = 0.61 (Hexan/Essigester 3:2).

Beispiel 4: (1S,2R)-1-Hydroxy-1-(3-trifluormethylphenyl)-8-(4-acetyl-3-hydroxy-2-propylphenoxy)-octa-3(E),5-(Z)-dien-2-yl-7-thio-4-oxo-4H-1-benzopyran-2-carbonsäure-Natriumsalz

Die Titelverbindung wird analog zu Beipiel 2 aus dem entsprechenden Methylester gemäss Beispiel 3 hergestellt; Smp. 210-212°, $[\alpha]_D^{20}$ (MeOH, 0.18 %) = -86.1 ± 5.6° UV(MeOH): $\lambda_{max}$ ($\epsilon$) = 220 (42820); 235/sh; 267 (26660); 285 (23760); 320 (15800).

Beispiel 5: (1R,2S)-1-Hydroxy-1-(3-trifluormethylphenyl)-6-(4-acetyl-3-hydroxy-2-propylphenoxy-hex-3(Z)-en-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-trifluormethylphenyl)-6-(4-acetyl-3-hydroxy-2-propylphenoxy)-hex-3(Z)-en hergestellt; hellgelbes zähes Oel; $[\alpha]_D^{20}$ (MeOH, 0.115 %) = 57.4 ± 8.7°; UV(MeOH): $\lambda_{max}$($\epsilon$) = 220/sh; 271 (5280); 285/sh; 320 (2800).

Das Ausgangsmaterial wird z.B. wie folgt hergestellt:

a) (2S,3R)-2,3-Epoxy-3-(3-trifluormethylphenyl)-propanal
Die Lösung von 1.1 g Oxalylchlorid in 15 ml Methylenchlorid wird unter Argon auf -65-70° gekühlt und innerhalb von 2 Minuten mit 1.5 g Dimethylsulfoxid in 5 ml Methylendichlorid versetzt. Nach 10-minütigem Rühren bei -65-70° werden 1.7 g (2R,3R)-2,3-Epoxy-3-(3-trifluormethylphenyl)-propanol in 15 ml Methylenchlorid zugetropft. Nach weiteren 30 Minuten Rühren werden 4 g Triäthylamin zugetropft, wobei die Temperatur auf - 40° steigt. Man lässt die Temperatur auf 0° steigen und giesst das Reaktionsgemisch auf Phosphatpuffer (pH 8). Die organische Phase wird abgetrennt und die wässrige Phase mit Methylenchlorid extrahiert. Die vereinigten organischen Extrakte werden 2-mal mit Sole gewaschen, über Natriumsulfat getrocknet und eingedampft. Chromatographie des Rückstandes an Kieselgel mit Hexan/Essigester (7:3) ergibt die Titelverbindung als farbloses Oel; IR (Methylenchlorid): 2820, 1730, 1330, 1165, 1125, 1070 $cm^{-1}$. $R_f$ = 0.36 (Hexan/Essigester 3:2). $[\alpha]_D^{20}$ (Chloroform, 0.20 %) = -17.5 ± 5°

b) (1R,2R)-1,2-Epoxy-1-(3-trifluormethylphenyl)-6-(4-acetyl-3-hydroxy-2-propylphenoxy)-hex-3(Z)-en.
Die Titelverbindung wird analog zu Beispiel 1d) aus dem Epoxyaldehyd gemäss a) hergestellt; hellgelbes Oel; $R_f$ = 0.69 (Hexan/Essigester 3:2).

Beispiel 6: (1R,2S)-1-Hydroxy-1-(3-trifluormethylphenyl)-6-(4-acetyl-3-hydroxy-2-propylphenoxy)-hex-3(Z)-en-2-yl-7-thio-4-oxo-4H-1-benzopyran-2-carbonsäure-Natriumsalz

Die Titelverbindung wird analog zu Beipiel 2 aus dem entsprechenden Methylester gemäss Beispiel 5 hergestellt; Smp. 222-224°; $[\alpha]_D^{20}$ (MeOH, 0.135 %) = 62.2 ± 7.4°. UV(MeOH): $\lambda_{max}$ ($\epsilon$) = 267 (22060); 285 (21140); 318/sh.

Beispiel 7: (1S,2R)-1-Hydroxy-1-(3-trifluormethylphenyl)-6-(4-acetyl-3-hydroxy-2-propylphenoxy)-hex-3(Z)-en-2-yl-7-thio-4-oxo-4H-1-benzopyran-2-carbonsäuremethylester

Die Titelverbindung wird analog zu Beispiel 1 aus (1S,2S)-1,2-Epoxy-1-(3-trifluormethylphenyl)-6-(4-acetyl-3-hydroxy-2-propylphenoxy)-hex-3(Z)-en hergestellt; farbloses Pulver vom Smp. 69-71°.

Das Ausgangsmaterial wird z.B. wie folgt hergestellt:

a) (2R,3S)-2,3-Epoxy-3-(3-trifluormethylphenyl)-propanal
Die Titelverbindung wird analog zu Beispiel 5a) aus (2S,3S)-2,3-Epoxy-3-(3-trifluormethylphenyl)-propanol hergestellt. Hellgelbe Flüssigkeit; IR ($CH_2Cl_2$): 2820, 1730, 1330, 1165, 1130, 1070 $cm^{-1}$; $[\alpha]_D^{20}$ (Chloroform, 0.20 %) = 0.0 ± 5;

$$[\alpha]_{365}^{20} \text{ (Chloroform, 0,20 \%) } = 475.0 \pm 5.0°;$$

$R_f$ = 0.44 (Hexan/Essigester 7:3).

b) (1S,2S)-1,2-Epoxy-1-(3-trifluormethylphenyl)-6-(4-acetyl-3-hydroxy-2-propylphenoxy)-hex-3(Z)-en.
Die Titelverbindung wird analog zu Beispiel 1d) aus dem entsprechenden Epoxyaldehyd gemäss a) hergestellt. Hellgelbes Oel; $R_f$ = 0.43 (Hexan/Essigester 3:2).

Beispiel 8: (1S,2R)-1-Hydroxy-1-(3-trifluormethvlphenyl)-6-(4-acetyl-3-hydroxy-2-propylphenoxy)-hex-3(Z)-en-2-yl-7-thio-4-oxo-4H-1-benzopyran-2-carbonsäure-Natriumsalz

Die Titelverbindung wird analog zu Beipiel 2 aus dem entsprechenden Methylester gemäss Beispiel 7 hergestellt; Smp. 239-241°;
$[\alpha]_D^{20}$ (Methanol, 0.15 %) = -60.7 ± 6.7°; UV(Methanol): $\lambda_{max}$ ($\epsilon$) = 216 (44080); 268 (22520); 285 (21640) 320/sh.

Beispiel 9: (1R,2S)-1-Hydroxy-1-(3-methylphenyl)-8-(4-acetyl-3-hydroxy-2-propylphenoxy)-octa-3(E),5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-methylphenyl)-8-(4-acetyl-3-hydroxy-2-propylphenoxy)-octa-3-(E),5(Z)-dien hergestellt; hellgelbes Pulver vom Smp. 71-72°; $[\alpha]_D^{20}$ = 81.7 ± 8.7° (MeOH, 0.115 %); UV(MeOH): $\lambda_{max}(\epsilon)$ = 217 (53680); 235/sh; 271 (29120); 285/sh; 325 (13200).
Das Ausgangsmaterial wird z.B. wie folgt hergestellt:
a) (2R,3R)-2,3-Epoxy-3-(3-methylphenyl)-propanol
Die Titelverbindung wird analog zu in Beispiel 1a) aus 3-(3-Methylphenyl)-prop-2(E)-enol hergestellt; farbloses, zähes Oel; IR ($CH_2Cl_2$): 3560, 3410, 2880, 2830, 1590, 1050 $cm^{-1}$; $R_f$ = 0.39 (Hexan/Essigester 1:1); $[\alpha]_D^{20}$ = 38.9 ± 5.3° (Chloroform, 0.19 %)
b) (4R,5R)-4,5-Epoxy-5-(3-methylphenyl)-pent-2(E)-enal.
Die Titelverbindung wird analog zu Beispiel 1b) aus dem Epoxyalkohol gemäss a) hergestellt; hellgelbes Pulver, Smp. 60-61°; IR ($CH_2Cl_2$): 2920, 2820, 2740, 1690, 1640, 1610, 1155, 1130 $cm^{-1}$ $R_f$ = 0.34 (Hexan/Essigester 4:1)
c) (1R,2R)-1,2-Epoxy-1-(3-methylphenyl)-8-(4-acetyl-3-hydroxy-2-propylphenoxy)-octa-3(E),5(Z)-dien.
Die Titelverbindung wird analog zu Beispiel 1d) aus dem entsprechenden Epoxyaldehyd gemäss a) hergestellt; hellgelbes Oel; $R_f$ = 0.63 (Hexan/Essigester 3:2).

Beispiel 10: (1R,2S)-1-Hydroxy-1-(3-methylphenyl)-8-(4-acetyl-3-hydroxy-2-propylphenoxy)-octa-3(E),5(Z)-dien-2-yl-7-thio-4-oxo-4H-1-benzopyran-2-carbonsäure-Natriumsalz

Die Titelverbindung wird analog zu Beispiel 2 aus dem entsprechenden Methylester gemäss Beispiel 9 hergestellt; beiges Pulver vom Smp. 217 (Zers.); $[\alpha]_D^{20}$ (Methanol, 0.15 %) = 71.3 ± 6.7°; UV (Methanol): $\lambda_{max}(\epsilon)$ = 219 (51520); 234/sh; 267 (26460); 284 (23280); 322/sh.

Beispiel 11: (1S,2R)-1-Hydroxy-1-(3-methylphenyl)-8-(4-acetyl-3-hydroxy-2-propylphenoxy)-octa-3(E),5(Z)-dien-2-yl-7-thio-4-oxo-4H-1-benzopyran-2-carbonsäuremethylester.

Die Titelverbindung wird analog zu Beispiel 1 aus (1S,2S)-1,2-Epoxy-1-(m-tolyl)-8-(4-acetyl-3-hydroxy-2-propylphenoxy)octa-3(E),5(Z)-dien hergestellt; $[\alpha]_D^{20}$ (MeOH, 0.148 %) = -75.7 ± 6.8°; UV(MeOH): $\lambda_{max}(\epsilon)$ = 217 (51760); 240/sh; 271 (27860); 290/sh; 328 (12600).
Das Ausgangsmaterial wird z.B. wie folgt hergestellt:
a) (2S,3S)-2,3-Epoxy-3-(3-methylphenyl)-propanol
Die Titelverbindung wird analog zu Beispiel 1a) aus 3-(3-Methylphenyl)-prop-2(E)-enol, aber unter Verwendung von L(+) Weinsäurediäthylester, hergestellt; farbloses Oel; IR ($CH_2Cl_2$): 3550, 3470, 2940, 2880, 2830, 1590, 1050 $cm^{-1}$; $R_f$ = 0.31 (Hexan/Essigester 3:2)
b) (4s,5S)-4,5-Epoxy-5-(3-methylphenyl)-pent-2(E)-enal.
Die Titelverbindung wird analog zu Beispiel 1b) aus dem Epoxyalkohol gemäss a) hergestellt; hellgelbes Oel; IR ($CH_2Cl_2$): 2920, 2820, 2740, 1690, 1640, 1610, 1155, 1130, 1085 $cm^{-1}$. $R_f$ = 0.29 (Hexan/Essigester 4:1).
c) (1S,2S)-1,2-Epoxy-1-(3-methylphenyl)-8-(4-acetyl-3-hydroxy-2-propyl-phenoxy)-octa-3(E),5(Z)-dien.
Die Titelverbindung wird analog zu Beispiel 1d) aus dem Epoxyaldehyd gemäss b) hergestellt; hellgelbes Oel; $R_f$ = 0.51 (Hexan/Essigester 7:3).

Beispiel 12: (1S,2R)-1-Hydroxy-1-(3-methylphenyl)-8-(4-acetyl-3-hydroxy-2-propylphenoxy)-octa-3(E),5(Z)-dien-2-yl-7-thio-4-oxo-4H-1-benzopyran-2-carbonsäure-Natriumsalz.

Die Titelverbindung wird analog zu Beipiel 1 aus dem entsprechenden Methylester gemäss Beispiel 11 hergestellt; Smp. 197-198°; $[\alpha]_D^{20}$ (0.14 % Methanol) = -72.1 ± 7.1°, UV (MeOH): $\lambda_{max}$ ($\epsilon$) = 218 (50700); 235/sh; 267 (25780); 285 (22600); 321 (15000).

Beispiel 13: (1R,2S)-1-Hydroxy-1-(3-methylphenyl)-6-(4-acetyl-3-hydroxy-2-propylphenoxy)-hex-3(Z)-en-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-tolyl)-6-(4-acetyl-3-hydroxy-2-propylphenoxy)-hex-3(Z)-en hergestellt; farbloses Pulver vom Smp. 136-138° $[\alpha]_D^{20}$ = 28.1 ± 7.4° (Methanol, 0.135 %) UV (Methanol): $\lambda_{max}$ ($\epsilon$) = 271 (26020; 282/sh; 323 (13700).

Das Ausgangsmaterial wird z.B. wie folgt hergestellt:

a) (2S,3R)-2,3-Epoxy-3-(3-methylphenyl)-propanal.

Die Titelverbindung wird analog zu Beispiel 5a) aus (2R,3R)-2,3-Epoxy-3-(3-methylphenyl)-propanol hergestellt; hellgelbes Oel. IR (Methylenchlorid): 2920, 2820, 1730, 1610, 1140, 1070 $cm^{-1}$ ; $R_f$ = 0.49 (Hexan/Essigester 3:2).

b) (1R,2R)-1,2-Epoxy-1-(3-methylphenyl)-6-(4-acetyl-3-hydroxy-2-propylphenoxy)-hex-3(Z)-en.

Die Titelverbindung wird analog zu Beispiel 1d) aus dem entsprechenden Epoxyaldehyd gemäss a) hergestellt; hellgelbes Oel; $R_f$ = 0.73 (Hexan/Essigester 3:2).

Beispiel 14: (1R,2S)-1-Hydroxy-1-(3-methylphenyl)-6-(4-acetyl-3-hydroxy-2-propylphenoxy)-hex-3(Z)-en-2-yl-7-thio-4-oxo-4H-1-benzopyran-2-carbonsäure-Natriumsalz.

Die Titelverbindung wird analog zu Beipiel 2 aus dem entsprechenden Methylester gemäss Beispiel 13 hergestellt; beiges Pulver vom Smp. 238-240°; $[\alpha]_D^{20}$ (Methanol 0.135 %) = 31.1 ± 7.4°; UV (Methanol): $\lambda_{max}$ ($\epsilon$) = 268 (21800); 285 (20860); 322/sh.

Beispiel 15: (1R,2S)-1-Hydroxy-1-(3-methoxycarbonylphenyl)-8-(4-acetyl-3-hydroxy-2-propylphenoxy)-octa-3(E),5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-methoxycarbonylphenyl)-8-(4-acetyl-3-hydroxy-2-propylphenoxy)-octa-3(E),5(Z)dien hergestellt; $[\alpha]_D^{20}$ (Methanol, 0.14 %) = 27.9 ± 7.1° ; UV (Methanol): $\lambda_{max}$ ($\epsilon$) = 221 (53560); 234/sh; 270 (28980); 285/sh; 326 (13860).

Das Ausgangsmaterial wird z.B. wie folgt hergestellt:

a) (2R,3R)-2,3-Epoxy-3-(3-methoxycarbonylphenyl)-propanol-

Die Titelverbindung wird analog zu Beispiel 1a) aus (E)-3-Methoxcarbonyl-zimtalkohol hergestellt; schwach gelbliches Oel; $R_f$ = 0.3 (Hexan/Essigester 1:1).

b) (4R,5R)-4,5-Epoxy-5-(3-methoxycarbonylphenyl)pent-2(E)-enal.

Die Titelverbindung wird analog zu Beispiel 1b) aus dem entsprechenden Epoxyalkohl hergestellt; hellgelbes Oel; $R_f$ = 0.35 (Hexan/Essigester 3:2).

c) (1R,2R)-1,2-Epoxy-1-(3-methoxycarbonylphenyl)-8-(4-acetyl-3-hydroxy-2-propylphenoxy)-octa-3(E),5-(Z)dien.

Die Titelverbindung wird analog zu Beispiel 1d) aus dem entsprechenden Epoxyaldehyd gemäss b) hergestellt; zähes gelbes Oel; $R_f$ = 0.50 (Hexan/Essigester 3:2).

Beispiel 16: (1R,2S)-1-Hydroxy-1-(3-methoxycarbonylphenyl)-8-(4-acetyl-3-hydroxy-2-propylphenoxy)-octa-3(E),5(Z)-dien-2-yl-7-thio-4-oxo-4H-1-benzopyran-2-carbonsäure-Natriumsalz.

Die Titelverbindung wird analog zu Beipiel 2 aus (1R,2S)-1-Hydroxy-1-(3-methoxycarbonylphenyl)-8-(4-acetyl-3-hydroxy-2-propylphenoxy)-octa-3(E),5(Z)-dien-2-yl-7-thio-4-oxo-4H-1-benzopyran-2-carbonsäuremethylester gemäss Beispiel 15 hergestellt; hellbraunes Pulver vom Smp. 181 (Zers.); $[\alpha]_D^{20}$ (Methanol, 0.15 %) = 32.0 ± 6.7°; UV (Methanol): $\lambda_{max}$ ($\epsilon$) = 222 (51600); 232/sh; 267 (24160); 284 (22940); 320/sh.; 400/sh.

Beispiel 17: (1S,2R)-1-Hydroxy-1-(3-methoxycarbonylphenyl)-8-(4-acetyl-3-hydroxy-2-propylphenoxy)-octa-3(E)-5(Z)-dien-2-yl-7-thio-4-oxo-4H-1-benzopyran-2-carbonsäuremethylester.

Die Titelverbindung wird analog zu Beispiel 1 aus dem (1S,2S)-1,2-Epoxy-1-(3-methoxycarbonylphenyl)-8-(4-acetyl-3-hydroxy-2-propylphenoxy)-octa-3(E),5(Z)-dien hergestellt; Smp. 77-78° (farbloses Pulver) $[\alpha]_D^{20}$ (Methanol, 0,15 %) = -52.7 ± 6.7°; UV (Methanol): $\lambda_{max}(\epsilon)$ = 221 (57420); 235/sh; 271 (29980); 288/sh; 326 (14320).

Das Ausgangsmaterial wird z.B. wie folgt hergestellt:

a) (2S,3S)-2,3-Epoxy-3-(3-methoxycarbonylphenyl)-propanol

Die Titelverbindung wird analog zu Beispiel 1a) aus dem (E)-3-Methox-carbonylzimtalkohol, aber unter Verwendung von L(+) Weinsäurediäthylester, hergestellt; farbloses Oel; $R_f$ = 0.48 (Hexan/Essigester 1:1).

b) (4S,5S)-4,5-Epoxy-5-(3-methoxycarbonylphenyl)-pent-2-(E)-enal.

Die Titelverbindung wird analog zu Beispiel 1b) aus dem entsprechenden Epoxyalkohl hergestellt; farbloses Oel; IR (Methylenchlorid): 3050, 2990, 2945, 2820, 2730, 1725, 1695, 1640, 1290, 1255 $cm^{-1}$; $R_f$ = 0.34 (Hexan/Essigester 3:2).

c) (1S,2S)-1,2-Epoxy-1-(3-methoxycarbonylphenyl)-8-(4-acetyl-3-hydroxy-2-propylphenoxy)-octa-3(E),5-(Z)-dien.

Die Titelverbindung wird analog zu Beispiel 1d) aus dem entsprechenden Epoxyaldehyd gemäss b) hergestellt; hellgelbes Oel; $R_f$ = 0.54 (Hexan/Essigester 3:2).

Beispiel 18: (1S,2R)-1-Hydroxy-1-(3-methoxycarbonylphenyl)-8-(4-acetyl-3-hydroxy-2-propylphenoxy)-octa-3(E),5(Z)-dien-2-yl-7-thio-4-oxo-4H-1-benzopyran-2-carbonsäure-Natriumsalz.

Die Titelverbindung wird analog zu Beipiel 2 aus dem entsprechenden Methylester gemäss Beispiel 17 hergestellt; beiges Pulver vom Smp. 174-176°; $[\alpha]_D^{20}$ (Methanol, 0.155 %) = -80.6 ± 6.5°; UV(Methanol): $\lambda_{max}(\epsilon)$ = 223 (59300); 235 sh; 267 (27300); 284 (24800); 321 (16200).

Beispiel 19: (1R,2S)-1-Hydroxy-1-(3-methoxycarbonylphenyl)-6-(4-acetyl-3-hydroxy-2-propylphenoxy)-hex-3-(Z)-en-2-yl-7-thio-4-oxo-4H-1-benzopyran-2-carbonsäuremethylester

Die Titelverbindung wird analog zu Beispiel 1 aus (1R,2R)-1,2-Epoxy-1-(3-methoxycarbonylphenyl)-6-(4-acetyl-3-hydroxy-2-propylphenoxy)-hex-3(Z)-en hergestellt; farbloses Oel; $[\alpha]_D^{20}$ = (Methanol, 0.11 %) = 24.5 ± 9.1°; UV (Methanol): $\lambda_{max}(\epsilon)$ = 270 (22400); 322 (12000).

Das Ausgangsmaterial wird z.B. wie folgt hergestellt:

a) (2S,3R)-2,3-Epoxy-3-(3-methoxycarbonylphenyl)-propanal

Die Titelverbindung wird analog zu Beispiel 5a) aus (2R,3R)-2,3-Epoxy-3-(3-methoxycarbonylphenyl)-propanol hergestellt; hellgelbes Oel; IR (Methylenchlorid): 2950, 2820, 1725, 1610, 1590, 1430, 1290, 1255 $cm^{-1}$; $R_f$ = 0.33 (Hexan/Essigester 3:2).

b) (1R,2R)-1,2-Epoxy-1-(3-methoxycarbonylphenyl)-6-(4-acetyl-3-hydroxy-2-propylphenoxy)-hex-3(Z)-en.

Die Titelverbindung wird analog zu Beispiel 1d) aus dem Epoxyaldehyd gemäss a) hergestellt; hellgelbes Oel; $R_f$ = 0.39 (Hexan/Essigester 3:2).

Beispiel 20: (1R,2S)-1-Hydroxy-1-(3-methoxycarbonylphenyl)-6-(4-acetyl-3-hydroxy-2-propylphenoxy)-hex-3-(Z)en-2-yl-7-thio-4-oxo-4H-1-benzopyran-2-carbonsäure-Natriumsalz (A), und (1R,2S)-1-Hydroxy-1-(3-carboxyphenyl)-6-(4-acetyl-3-hydroxy-2-propylphenoxy)-hex-3(Z)-en-2-yl-7-thio-4-oxo-4H-1-benzopyran-2-carbonsäure-Dinatriumsalz (B).

Die Lösung von 0,5 g (1R,2S)-1-Hydroxy-1-(3-methoxycarbonylphenyl)-6-(4-acetyl-3-hydroxy-2-propylphenoxy)-hex-3(Z)-en-2-yl-7-thio-4-oxo-4H-1-benzopyran-2-carbonsäuremethylester gemäss Beispiel 19 in 20 ml Tetrahydrofuran wird unter Argon 40 Stunden mit 7.5 ml 0.2n Natronlauge bei Raumtemperatur gerührt und dann eingedampft. Der Rückstand wird durch Chromatographie an Lichroprep® RP-8, Merck mit Methanol/Wasser (3:1) gereinigt und liefert in den Fraktionen 2-5 die Titelverbindung B; Smp. 262-264°; $[\alpha]_D^{20}$ (Methanol, 0.105 %) = 17.1 ± 9.5°; UV (Methanol): $\lambda_{max}$ $(\epsilon)$ = 268 (19680); 284 (19060); 325/sh; und in den Fraktionen 8-12 die Titelverbindung A; Smp. 155 (Zers.); $[\alpha]_D^{20}$ (Methanol, 0.12 %) = 22.5 ± 8.3°; UV (Methanol): $\lambda_{max}$ $(\epsilon)$ = 268 (26200); 286 (29220); 325/sh.

Beispiel 21: (1S,2R)-1-Hydroxy-1-(3-methoxycarbonylphenyl)-6-(4-acetyl-3-hydroxy-2-propylphenoxy)-hex-3-(Z)-en-2-yl-7-thio-4-oxo-4H-1-benzopyran-2-carbonsäuremethylester

Die Titelverbindung wird analog zu Beispiel 1 aus dem (1S,2S)-1,2-Epoxy-1-(3-carboxymethylphenyl)-6-(4-acetyl-3-hydroxy-2-propylphenoxy)-hex-3(Z)-en hergestellt; farbloses Oel, das im Kühlschrank erstarrt, Smp. 90-91°; $[\alpha]_D^{20}$ = -41.5±7.7° (0.13 % Methanol); UV (Methanol): $\lambda_{max}$ ($\epsilon$) = 271 (25120); 322 (13280).

Das Ausgangsmaterial wird z.B. wie folgt hergestellt:

a) (2R,3S)-2,3-Epoxy-3-(3-methoxycarbonylphenyl)-propanal

Die Titelverbindung wird analog zu Beispiel 5 aus (2S,3S)-2,3-Epoxy-3-(3-methoxycarbonylphenyl)-propanol hergestellt; farbloses Oel; IR (Methylenchlorid): 2910, 2780, 1705, 1590, 1570, 1415, 1270, 1235 $cm^{-1}$; $R_f$ = 0.36 (Hexan/Essigester 3:2).

b) (1S,2S)-1,2-Epoxy-1-(3-methoxycarbonylphenyl)-6-(4-acetyl-3-hydroxy-2-propylphenoxy)-hex-3(Z)-en.

Die Titelverbindung wird analog zu Beispiel 1d) aus dem entsprechenden Epoxyaldehyd gemäss a) hergestellt; farbloses Oel, nicht näher charakterisiert.

Beispiel 22: (1S,2R)-1-Hydroxy-1-(3-methoxycarbonylphenyl)-6-(4-acetyl-3-hydroxy-2-propylphenoxy)-hex-3-(Z)-en-2-yl-7-thio-4-oxo-4H-1-benzopyran-2-carbonsäure-Natriumsalz.

Die Titelverbindung wird analog zu Beispiel 2 aus dem entsprechenden Methylester gemäss Beispiel 21 hergestellt; beiges Pulver vom Smp. 208-210°; $[\alpha]_D^{20}$ (Methanol, 0.12 %) = -41.7 ± 8.3°, UV (Methanol): $\lambda_{max}(\epsilon)$ = 268 (21060); 285 (21540); 325/sh.

Beispiel 23: (4R,5S)-1,1,1-Trifluor-4-hydroxy-11-(4-acetyl-3-hydroxy-2-propylphenoxy)-undeca-6(E),8(Z)-dien-5-yl-7-thio-4-oxo-4H-1-benzopyran-2-carbonsäuremethylester.

Die Titelverbindung wird analog zu Beispiel 1) aus (4R,5R)-4,5-Epoxy-1,1,1-trifluor-11-(4-acetyl-3-hydroxy-2-propylphenoxy)-undeca-6(E),8(Z)-dien hergestellt; beiges Pulver vom Smp. 64-66°, $[\alpha]_D^{20}$ (Methanol 0.15 %) = 147.3 ± 6.7°. UV (Methanol): $\lambda_{max}(\epsilon)$ = 220 (48960); 270 (28500); 283/sh; 325 (13400).

Das Ausgangsmaterial wird z.B. wie folgt hergestellt:

a) (2R,3R)-2,3-Epoxy-6,6,6-trifluor-hexanol.

Die Titelverbindung wird analog zu Beispiel 1a) aus dem 6,6,6-Trifluor-hex-2(E)-enol hergestellt; farbloses Oel; $[\alpha]_D^{20}$ (Chloroform, 0.17 %) = 35.9 ± 5.9°; IR (Methylenchlorid): 3550, 3420, 2950, 2890, 2830, 1125 $cm^{-1}$.

b) (4R,5R)-4,5-Epoxy-8,8,8-trifluor-oct-2(E)-enal.

Die Titelverbindung wird analog zu Beispiel 1b) aus dem entsprechenden Epoxyalkohol gemäss a) hergestellt; hellgelbes Oel; IR ($CH_2Cl_2$): 3050, 2980, 2930, 2820, 2730, 1695, 1645, 1450, 1150, 1100 $cm^{-1}$; $R_f$ = 0.41 (Hexan/Essigester = 3:2).

c) (4R,5R)-4,5-Epoxy-1,1,1-trifluor-11-(4-acetyl-3-hydroxy-2-propyl-phenoxy)-undeca-6(E),8(Z)-dien.

Die Titelverbindung wird analog zu Beispiel 1d) aus dem Epoxyaldehyd gemäss b) hergestellt; hellgelbes Oel; $R_f$ = 0.48 (Hexan/Essigester 3:2).

Beispiel 24: (4R,5S)-1,1,1-Trifluor-4-hydroxy-11-(4-acetyl-3-hydroxy-2-propylphenoxy)-undeca-6(E),8(Z)-dien-5-yl-7-thio-4-oxo-4H-1-benzopyran-2-carbonsäure-Natriumsalz.

Die Titelverbindung wird analog zu Beispiel 2 aus dem entsprechenden Methylester gemäss Beispiel 23 hergestellt; beiges Pulver vom Smp. 198-200°, $[\alpha]_D^{20}$ (Methanol, 0.15 %) = 127.3 ± 6.7°; UV (Methanol): $\lambda_{max}(\epsilon)$ = 221 (48820); 230/sh.; 267 (25440); 285 (23080); 322/sh.

Beispiel 25: (4S,5R)-1,1,1-Trifluor-4-hydroxy-11-(4-acetyl-3-hydroxy-2-propylphenoxy)-undeca-6(E),8(Z)-dien-5-yl-7-thio-4-oxo-4H-1-benzopyran-2-carbonsäuremethylester.

Die Titelverbindung wird analog zu Beispiel 1 aus (4S,5S)-4,5-Epoxy-1,1,1-trifluor-11-(4-acetyl-3-hydroxy-2-propylphenoxy)-undeca-6(E),8(Z)-dien hergestellt; farbloses Pulver vom Smp. 69-71°; $[\alpha]_D^{20}$ = -153.3 ± 6.7° (Methanol, 0.15 %). UV (Methanol): $\lambda_{max}(\epsilon)$ = 220 (49560); 235/sh; 270 (29220); 285/sh; 325 (12990).

Das Ausgangsmaterial wird z.B. wie folgt hergestellt:

a) (2S,3S)-2,3-Epoxy-6,6,6-trifluor-hexanol

Die Titelverbindung wird analog zu Beispiel 1a) aus 6,6,6-Trifluorhex-2(E)-enol, aber unter Verwendung

von L(+) Weinsäurediäthylester, hergestellt; farbloses Oel; $[\alpha]_D^{20}$ (Chloroform 0.17 %) = -25.9 ± 5.9°; IR (Methylenchlorid): 3550, 3430, 2940, 2880, 2830, 1125 $cm^{-1}$.

b) (4S,5S)-4,5-Epoxy-8,8,8-trifluor-oct-2(E)-enal.

Die Titelverbindung wird analog zu Beispiel 1b) aus dem entsprechenden Epoxyalkohol gemäss a) hergestellt, hellgelbes Oel; IR (Methylenchlorid): 3050, 2990, 2930, 2820, 2730, 1695, 1645, 1450, 1150, 1100 $cm^{-1}$; $R_f$ = 0.36 (Hexan/Essigester 3:2).

c) (4S,5S)-4,5-Epoxy-1,1,1-trifluor-11-(4-acetyl-3-hydroxy-2-propyl-phenoxy)-undeca-6(E),8(Z)-dien.

Die Titelverbindung wird analog zu Beispiel 1d) aus dem entsprechenden Epoxyaldehyd gemäss b) hergestellt; hellgelbes Oel.

Beispiel 26: (4S,5R)-1,1,1-Trifluor-4-hydroxy-11-(4-acetyl-3-hydroxy-2-propylphenoxy)-undeca-6(E),8(Z)dien-5-yl-7-thio-4-oxo-4H-1-benzopyran-2-carbonsäure-Natriumsalz.

Die Titelverbindung wird analog zu Beispiel 2 aus dem entsprechenden Methylester gemäss Beispiel 25 hergestellt; beiges Pulver vom Smp. 201-203°; $[\alpha]_D^{20}$ (Methanol, 0.15 %) = -117.3 ± 6.7°; UV(Methanol): $\lambda_{max}(\epsilon)$ = 222 (48320); 233/sh.; 267 (26440); 285 (24440); 330/sh.

Beispiel 27: (4R,5S)-1,1,1-Trifluor-4-hydroxy-9-(4-acetyl-3-hydroxy-2-propylphenoxy)-non-6(Z)-en-5-yl-7-thio-4-oxo-4H-1-benzo-pyran-2-carbonsauremethylester.

Die Titelverbindung wird analog zu Beispiel 1 aus dem (4R,5R)-4,5-Epoxy-1,1,1-trifluor-9-(4-acetyl-3-hydroxy-2-propylphenoxy)-non-6(Z)-en hergestellt; farbloses, zähes Oel; IR (Methylenchlorid): 3530, 2920, 2890, 2820, 1720, 1640, 1605, 1585 $cm^{-1}$.

Das Ausgangsmaterial wird z.B. wie folgt hergestellt:

a) (2S,3R)-2,3-Epoxy-6,6,6-trifluor-hexanal

Die Titelverbindung wird analog zu Beispiel 5a) aus dem (2R,3R)-2,3-Epoxy-6,6,6-trifluor-hexanol hergestellt, farblose Flüssigkeit vom Sdp. 80-81°/26 mbar.; $[\alpha]_D^{20}$ (Chloroform, 0.15 %) = -10.7 ± 6.7°.

b) (4R,5R)-4,5-Epoxy-1,1,1-trifluor-9-(4-acetyl-3-hydroxy-2-propyl-phenoxy)-non-6(Z)-en.

Die Titelverbindung wird analog zu Beispiel 1d) aus dem Epoxyaldehyd gemäss a) hergestellt; hellgelbes Oel.

Beispiel 28: (4R,5S)-1,1,1-Trifluor-4-hydroxy-9-(4-acetyl-3-hydroxy-2-propylphenoxy)-non-6(Z)-en-5-yl-7-thio-4-oxo-4H-1-benzo-pyran-2-carbonsäure-Natriumsalz.

Die Titelverbindung wird analog zu Beispiel 2 aus dem entsprechenden Methylester gemäss Beispiel 27 hergestellt; hellgelbes Pulver vom Smp. 204-206°; $[\alpha]_D^{20}$ (Methanol, 0.15 %) = 42.7 ± 6.7°, UV (Methanol): $\lambda_{max}(\epsilon)$ = 218 (36920); 268 (20280); 285 (20180); 325/sh.

Beispiel 29: (5R,6S)-5-Hydroxy-12-(4-acetyl-3-hydroxy-2-propylphenoxy)-dodec-7(Z)-en-6-yl-7-thio-4-oxo-4H-1-benzopyran-2-carbon-säuremethylester.

Die Titelverbindung wird analog zu Beispiel 1 aus (5R,6R)-5,6-Epoxy-12-(4-acetyl-3-hydroxy-2-propylphenoxy)-dodec-7-(Z)-en hergestellt; hellgelbes Oel; $R_f$ = 0.37 (Hexan/Essigester 1:1).

Das Ausgangsmaterial wird z.B. wie folgt hergestellt:

a) 5-(4-Acetyl-3-hydroxy-2-propylphenoxy)pentyl-triphenylphosphonium-bromid

Die Titelverbindung wird wie in Beispiel 1c) aus 5-(4-Acetyl-3-hydroxy-2-propylphenoxy)pentylbromid hergestellt; farbloses Kristalle vom Smp. 82-85°.

b) (2S,3R)-2,3-Epoxy-heptanal.

Die Titelverbindung wird analog zu Beispiel 5a) aus dem (2R,3R)-2,3-Epoxyheptanolhergestellt; $[\alpha]_D^{20}$ = -99.4 ± 0.1°.

c) (5R,6R)-5,6-Epoxy-12-(4-acetyl-3-hydroxy-2-propylphenoxy)-dodec-7-(Z)-en.

Die Titelverbindung wird analog zu Beispiel 1d) aus dem Epoxyaldehyd gemäss a) hergestellt, hellgelbes Oel.

Beispiel 30: (5R,6S)-5-Hydroxy-12-(4-acetyl-3-hydroxy-2-propylphenoxy)-dodec-7(Z)-en-6-yl-7-thio-4-oxo-4H-1-benzopyran-2-carbon-säure-Natriumsalz.

Die Titelverbindung wird analog zu Beipiel 2 aus dem entsprechenden Methylester gemäss Beispiel 29 hergestellt; Smp. 192-194°; $[\alpha]_D^{20}$ (Methanol, 0.125 %) = +5.6 ± 8.0°; UV (Methanol): $\lambda_{max}(\epsilon)$ = 218 (38000); 268 (22040); 285 (21120); 325 sh.

Beispiel 31: (5S,6R)-5-Hydroxy-12-(4-acetyl-3-hydroxy-2-propylphenoxy)-dodec-7(Z)-en-6-yl-7-thio-4-oxo-4H-1-benzopyran-2-carbon-säuremethylester.

Die Titelverbindung wird analog zu Beispiel 1 aus (5S,6S)-5,6-Epoxy-12-(4-acetyl-3-hydroxy-2-propylphenoxy)-dodec-7-(Z)-en hergestellt; hellgelbes Oel; $R_f$ = 0.41 (Hexan/Essigester 1:1).

Das Ausgangsmaterial wird z.B. wie folgt hergestellt:

a) (2R,3S)-2,3-Epoxy-heptanal.

Die Titelverbindung wird analog zu Beispiel 5a) aus (2S,3S)-2,3-Epoxy-heptanol hergestellt; $[\alpha]_D^{20}$ = +104.3 ± 0.4°.

b) (5S,6S)-5,6-Epoxy-12-(4-acetyl-3-hydroxy-2-propylphenoxy)-dodec-7(Z)-en.

Die Titelverbindung wird analog zu Beispiel 1d) aus dem Epoxyaldehyd gemäss a) hergestellt; hellgelbes Oel, $R_f$ = 0.42 (Hexan/Essigester 3:2).

Beispiel 32: (5S,6R)-5-Hydroxy-12-(4-acetyl-3-hydroxy-2-propylphenoxy)-dodec-7(Z)-en-6-yl-7-thio-4-oxo-4H-1-benzopyran-2-carbon-säure-Natriumsalz.

Die Titelverbindung wird analog zu Beipiel 2 aus dem entsprechenden Methylester gemäss Beispiel 31 hergestellt; Smp. 192-194°; $[\alpha]_D^{20}$ (Methanol, 0.145 %) = 0 ± 6.9°; UV (Methanol): $\lambda_{max}(\epsilon)$ = 218 (37060); 268 (21760); 286 (20520); 325 sh.

Beispiel 33: (4R,5S)-1,1,1-Trifluor-4-hydroxy-11-(4-acetyl-3-hydroxy-2-propylphenoxy)-undec-6(Z)-en-5-yl-7-thio-4-oxo-4H-1-benzo-pyran-2-carbonsäuremethylester

Die Titelverbindung wird analog zu Beispiel 1 aus (4R,5R)-4,5-Epoxy-1,1,1-trifluor-11-(4-acetyl-3-hydroxy-2-propylphenoxy)-undec-6(Z)-en hergestellt, hellgelbes Oel; $R_f$ = 0.47 (Hexan/Essigester 1:1).

Das Ausgangsmaterial wird z.B. analog zu Beispiel 1d) aus (2S,3R)-2,3-Epoxy-6,6,6-trifluor-hexanal hergestellt; hellgelbes Oel.

Beispiel 34: (4R,5S)-1,1,1-Trifluor-4-hydroxy-11-(4-acetyl-3-hydroxy-2-propylphenoxy)-undec-6(Z)en-5-yl-7-thio-4-oxo-4H-1-benzopyran-2-carbonsäure-Natriumsalz.

Die Titelverbindung wird analog zu Beispiel 2 aus dem entsprechenden Methylester gemäss Beispiel 33 hergestellt; Smp. 193-195°; $[\alpha]_D^{20}$ (Methanol, 0.135 %) = +16.3 ± 7,40°; UV (MeOH): $\lambda_{max}$ $(\epsilon)$ = 218 (37380). 267 (21380), 286 (21020), 325/sh.

Beispiel 35: (5R,6S)-5-Hydroxy-10-(4-acetyl-3-hydroxy-2-Propylphenoxy)-dec-7(Z)en-6-yl-7-thio-4-oxo-4H-1-benzopyran-2-carbon-säuremethylester.

Die Titelverbindung wird analog zu Beispiel 1 aus (5R,6R)-5,6-Epoxy-10-(4-acetyl-3-hydroxy-2-propylphenoxy)-dec-7(Z)-en hergestellt, hellgelbes Oel; $[\alpha]_D^{20}$ (Methanol, 0.135 %) = +48.9 ± 7.4°, UV (Methanol): $\lambda_{max}(\epsilon)$ = 216 (38140); 271 (24040); 285 sh, 322 (12700).

Das Ausgangsmaterial wird z.B. analog zu Beispiel 1d) aus (2S,3R)-2,3-Epoxy-heptanal hergestellt; hellgelbes Oel. $R_f$ = 0.45 (Hexan/Essigester 7:3).

Beispiel 36: (5R,6S)-5-Hydroxy-10-(4-acetyl-3-hydroxy-2-propylphenoxy)-dec-7(Z)-en-6-yl-7-thio-4-oxo-4H-1-benzopyran-2-carbonsäure.

Die Titelverbindung wird analog zu Beispiel 2 aus dem entsprechenden Methylester gemäss Beispiel 35 hergestellt und mit Chlorwasserstoffsäure zur freien Säure umgewandelt; Smp. 58-60°; $[\alpha]_D^{20}$ (Methanol, 0.130 %) = +36.2 ± 7.7°; UV (Methanol): $\lambda_{max}(\epsilon)$ = 218 (35240); 269 (20760); 283 (19800); 330 sh.

Beispiel 37: (5S,6R)-5-Hydroxy-10-(4-acetyl-3-hydroxy-2-Propylphenoxy)-dec-7(Z)-en-6-yl-7-thio-4-oxo-4H-1-benzopyran-2-carbonsäuremethylester

Die Titelverbindung wird analog zu Beispiel 1 aus (5S,6S)-5,6-Epoxy-10-(4-acetyl-3-hydroxy-2-propylphenoxy)-dec-7(Z)-en hergestellt, hellgelbes Oel; $[\alpha]_D^{20}$ (Methanol, 0.115 %) = -50.4 ± 8.7°; UV (MeOH): $\lambda_{max}(\epsilon)$ = 217 (38000); 271 (24100); 285 sh, 321 (12800).

Das Ausgangsmaterial wird z.B. analog zu Beispiel 1d) aus (2R,3S)-2,3-Epoxy-heptanal hergestellt; hellbraunes Oel; $R_f$ = 0.52 (Hexan/Essigester 7:3).

Beispiel 38: (5S,6R)-5-Hydroxy-10-(4-acetyl-3-hydroxy-2-propylphenoxy)-dec-7(Z)-en-6-yl-7-thio-4-oxo-4H-1-benzopyran-2-carbonsäure-Natriumsalz.

Die Titelverbindung wird analog zu Beispiel 2 aus dem entsprechenden Methylester gemäss Beispiel 37 hergestellt; Smp. 224-226°, $[\alpha]_D^{20}$ (Methanol, 0.145 %) = -29.0 ± 6.9°; UV (Methanol): $\lambda_{max}(\epsilon)$ = 219 (38760); 268 (21880); 285 (21260); 325 sh.

Beispiel 39: (5S,6R)-5-Hydroxy-10-(4-acetyl-3-hydroxy-2-propylphenoxy)-dec-7(Z)-en-6-yl-7-thio-4-oxo-4H-1-benzopyran-2-(N-benzolsulfonamidyl)carboxamid.

Die Lösung von 0.20 g (5S,6R)-5-Hydroxy-10-(4-acetyl-3-hydroxy-2-propylphenoxy)-dec-7(Z)-en-6-yl-7-thio-4-oxo-4H-1-benzopyran-2-carbonsäure in 10 ml Methylenchlorid wird unter Argon mit 60 mg Benzolsulfonamid, 44 mg 4-Dimethylaminopyridin und 70 mg N-Aethyl-N'-(3-dimethylaminopropyl)-carbodiimid-hydrochlorid 24 Stunden bei Raumtemperatur gerührt. Die gebildete Lösung wird mit 30 ml Methylenchlorid verdünnt, 2-mal mit 1n-Salzsäure und 2-mal mit Sole gewaschen, über Magnesiumsulfat getrocknet und eingedampft. Chormatographie des Rückstandes an Kieselgel mit Methylenchlorid/Methanol (9:1) liefert die Titelverbindung vom Smp. 140-142°.

Beispiel 40: (4RS,5SR)-1-Methoxycarbonyl-4-hydroxy-9-(4-acetyl-3-hydroxy-2-propylphenoxy)-non-6(Z)-en-5-yl-7-thio-4-oxo-4H-1-benzopyran-2-carbonsäuremethylester.

Die Titelverbindung wird analog zu Beispiel 1 aus (4RS,5RS)-4,5-Epoxy-1-methoxycarbonyl-9-(4-acetyl-3-hydroxy-2-propylphenoxy)-non-6(Z)-en hergestellt; farbloses Oel; $[\alpha]_D^{20}$ (Methanol, 0.125 %) = 0.0 ± 8.0°, UV (Methanol): $\lambda_{max}(\epsilon)$ = 270 (24000); 340 (13200).

Das Ausgangsmaterial wird z.B. analog zu Beispiel 1 d) aus 5,6-Epoxy-6-formylhexansäuremethylester hergestellt; hellgelbes Oel; $R_f$ = 0.35 (Hexan/Essigester 3:2).

Beispiel 41: (4RS,5SR)-1-Carboxy-4-hydroxy-9-(4-acetyl-3-hydroxy-2-propylphenoxy)-non-6(Z)-en-5-yl-7-thio-4-oxo-4H-1-benzopyran-2-carbonsäure-Dinatriumsalz.

0,24 g des Methylester gemäss Beispiel 40 werden unter Argon in 15 ml Tetrahydrofuran gelöst, mit 3,8 ml 0,2n-Natronlauge versetzt und 20 Stunden bei Raumtemperatur gerührt. Eindampfen und chromatographische Reinigung des Rückstandes an einer "Reversed Phase"-Kieselgelsäule (z.B. Merck Lichroprep® RP-8) mit Methanol/Wasser (7:3) ergibt die Titelverbindung von Smp. 248-250° (Zers.); UV (Methanol): $\lambda_{max}(\epsilon)$ = 218 (33900); 268 (18580); 284 (18240); 330 sh.

Beispiel 42: (1R,2S)-1-Hydroxy-1-(3-trifluormethylphenyl)-10-(4-acetyl-3-hydroxy-2-propylphenoxy)-deca-3-(E),5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-trifluormethylphenyl)-10-(4-acetyl-3-hydroxy-2-propylphenoxy)-deca-3(E),5(Z)-dien hergestellt; hellgelbes Oel. $[\alpha]_D^{20}$ ($CHCl_3$, 0.363 %) = 46.6 ± 2.8° UV ($CHCl_3$): $\lambda_{max}(\epsilon)$ = 270 (26500); 285 (24240); 322 (15200).

Das Ausgangsmaterial wird z.B. wie folgt hergestellt:

a) (1R,2R)-1,2-Epoxy-1-(3-trifluormethylphenyl)-10-(4-acetyl-3-hydroxy-2-propylphenoxy)-deca-3(E),5(Z)-dien. Die Titelverbindung wird analog zu Beispiel 1d aus 5-(4-Acetyl-3-hydroxy-2-propylphenoxy)-pentyltriphenyl-phosphoniumbromid (Beispiel 29a) und (4R,5R)-4,5-Epoxy-5-(3-trifluormethylphenyl)-pent-2(E)-enal (Beispiel 1b) hergestellt; hellbraunes Oel; $[\alpha]_D^{20}$ ($CHCl_3$, 0.224 %) = 70.8 ± 10° $R_f$ = 0.50 (Hexan/Essigester = 1:1) IR ($CH_2Cl_2$): 2960, 2930, 2865, 1735, 1625, 1330, 1125 $cm^{-1}$.

Beispiel 43: (1R,2S)-1-Hydroxy-1-(3-trifluormethylphenyl)-10-(4-acetyl-3-hydroxy-2-propylphenoxy)-deca-3-(E) ,5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 42) hergestellt; Smp. 217-219°; $[\alpha]_D^{20}$ (MeOH; 0.160 %) = 145.6 ± 6.3° UV (MeOH): $\lambda_{max}(\epsilon)$ = 220 (50480), 230 (sh), 267 (26240), 284 (23000), 320 (sh).

Beispiel 44: (1R,2S)-1-Hydroxy-1-(3-methylphenyl)-10-(4-acetyl-3-hydroxy-2-propylphenoxy)-deca-3(E),5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-methylphenyl)-10-(4-acetyl-3-hydroxy-2-propylphenoxy)-deca-3(E),5(Z)-dien hergestellt; Smp. 59-60°; $[\alpha]_D^{20}$ ($CHCl_3$, 0.163 %) = 31.9 ± 6.1°; UV ($CHCl_3$): $\lambda_{max}$ ($\epsilon$) = 241 (31420); 286 (23060).

Das Ausgangsmaterial wird z.B. wie folgt hergestellt:

a) (1R,2R)-1,2-Epoxy-1-(3-methylphenyl)-10-(4-acetyl-3-hydroxy-2-propyl-phenoxy)-deca-3(E) ,5(Z)-dien.
Die Titelverbindung wird analog zu Beispiel 1d aus 5-(4-Acetyl-3-hydroxy-2-propylphenoxy)-pentyltriphenylphosphoniumbromid (Beispiel 29a) und (4R,5R)-4,5-Epoxy-5-(3-methylphenyl)-pent-2(E)-enal (Beispiel 9b) hergestellt; hellgelbes Oel; $[\alpha]_D^{20}$ ($CHCl_3$, 0.273 %) = 118.7° ± 3.7. $R_f$ = 0.62 (Hexan/Essigester = 3:2).

Beispiel 45: (1R,2S)-1-hydroxy-1-(3-methylphenyl)-10-(4-acetyl-3-hydroxy-2-propylphenoxy)-deca-3(E),5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 44) hergestellt; Smp. 208-210°; $[\alpha]_D^{20}$ (MeOH, 0.30 %) = 50.7 ± 3.3°. UV (MeOH): $\lambda_{max}(\epsilon)$ = 219 (52420), 230 (sh), 267 (26620), 285 (23520), 325 (sh).

Beispiel 46: (1S,2R)-1-Hydroxy-1-(3-trifluormethylphenyl)-10-(4-acetyl-3-hydroxy-2-propylphenoxy)-deca-3-(E) ,5(Z)-dien-2-yl-7-thio-4-oxo-4H-1-benzopyran-2-carbonsäuremethylester.

Die Titelverbindung wird analog zu Beispiel 1 aus (1S,2S)-1,2-Epoxy-1-(3-trifluormethylphenyl)-10-(4-acetyl-3-hydroxy-2-propylphenoxy)-deca-(3E),5(Z)-dien hergestellt; zähe Masse;
$R_f$ = 0.48 (Hexan/Essigester = 1:1).

Das Ausgangsmaterial wird z.B. wie folgt hergestellt:

a) (1S,2S)-1,2-Epoxy-1-(3-trifluormethylphenyl)-10-(4-acetyl-3-hydroxy-2-propylphenoxy)-deca-3(E),5(Z)-dien.
Die Titelverbindung wird analog zu Beispiel 1d aus 5-(4-Acetyl-3-hydroxy-2-propylphenoxy)-pentyltriphenylphosphoniumbromid (Beispiel 29a) und (4S,5S)-4,5-Epoxy-5-(3-trifluormethylphenyl)-pent-2(E)-enal (Beispiel 3b) hergestellt; hellgelbes Oel; $[\alpha]_D^{20}$ (Chloroform, 0.454 % = -86.8 ± 2.2°; $R_f$ = 0.46 (Hexan/Essigester = 1:1). IR (Methylenchlorid): 2960, 2930, 1730, 1625, 1330, 1130 $cm^{-1}$.

Beispiel 47: (1S,2R)-1-Hydroxy-1-(3-trifluormethylphenyl)-10-(4-acetyl-3-hydroxy-2-propylphenoxy)-deca-3-(E),5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 46) hergestellt; Smp. 168-170°; $[\alpha]_D^{20}$ (Methanol, 0.150 %) = -66.7 ± 6.7°; UV (MeOH): $\lambda_{max}(\epsilon)$ = 220 (49640), 230 (sh), 266 (25640), 285 (22140), 320 (sh).

Beispiel 48: (1R,2S)-1-Hydroxy-1-(3-trifluormethylphenyl)-8-[4-acetyl-3-hydroxy-2-(3,3,3-trifluorpropyl)-phenoxy]-octa-3(E),5(Z)-dien-2-yl-7-thio-4-oxo-4H-1-benzopyran-2-carbonsäuremethyl-ester.

Die Titelverbindung wird analog zu Beispiel 1 aus (1R,2R)-1,2-Epoxy-1-(3-trifluormethylphenyl)-8-[4-acetyl-3-hydroxy-2-(3,3,3-trifluorpropyl)-phenoxy]-octa-3(E),5(Z)-dien hergestellt; hellgelbes Oel; $R_f$ = 0.34 (Hexan/Essigester = 1:1).

Das Ausgangsmaterial wird z.B. wie folgt hergestellt:

a) (1R,2R)-1,2-Epoxy-1-(3-trifluormethylphenyl)-8-[4-acetyl-3-hydroxy-2-(3,3,3-trifluorpropyl)-phenoxy]-octa-3(E),5(Z)-dien.
Die Titelverbindung wird analog zu Beispiel 1d aus 3-[4-Acetyl-3-hydroxy-2-(3,3,3-trifluorpropyl)-phenoxy]-propyltriphenylphosphoniumbromid und (4R,5R)-4,5-Epoxy-5-(3-trifluormethylphenyl)-pent-2(E)-enal (Beispiel 1b) hergestellt; hellgelbes Oel; $[\alpha]_D^{20}$ (Chloroform, 0.406 %) = -58.6 ± 2.5°; $R_f$ = 0.45 (Hexan/Essigester = 7:3). IR (Methylenchlorid: 2945, 1670, 1610, 1310, 1055 $cm^{-1}$.

b) 3-[4-Acetyl-3-hydroxy-2-(3,3,3-trifluorpropyl)-phenoxy]propyl-triphenylphosphoniumbromid.

Die Titelverbindung wird analog zu Beispiel 1c aus 3-[4-Acetyl-3-hydroxy-2-(3,3,3-trifluorpropyl)-phenoxy]propylbromid hergestellt. Smp. 184-185°.

Beispiel 49: (1R,2S)-1-Hydroxy-1-(3-trifluormethylphenyl)-8-[4-acetyl-3-hydroxy-2-(3,3,3-trifluorpropyl)-phenoxy]-octa-3(E),5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 48) hergestellt; Smp. 238-240°; $[\alpha]_D^{20}$ (Methanol, 0.150 %) = 208 ± 6.6°; UV (MeOH): $\lambda_{max}(\epsilon)$ = 216 (50040), 230 (sh), 267 (28960), 280 (sh), 320 (16020).

Beispiel 50: (1R,2S)-1-Hydroxy-1-(3-methylphenyl)-8-[4-acetyl-3-hydroxy-2-(3,3,3-trifluorpropyl)-phenoxy]-octa-3(E),5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-methylphenyl)-8-[4-acetyl-3-hydroxy-2-(3,3,3-trifluorpropyl)-phenoxy]-octa-3(E),5(Z)-dien hergestellt; hellgelbes zähes Oel; $R_f$ = 0.41 (Hexan/Essigester = 1:1). $[\alpha]_D^{20}$ (Chloroform, 0.155 %) = 32.3 ± 6.5°. UV (Chloroform): $\lambda_{max}(\epsilon)$ = 271 (32320), 318 (16100).

Das Ausgangsmaterial wird z.B. wie folgt hergestellt:

a) (1R,2R)-1,2-Epoxy-1-(3-methylphenyl)-8-[4-acetyl-3-hydroxy-2-(3,3,3-trifluorpropyl)-phenoxy]-octa-3(E)-,5(Z)-dien.

Die Titelverbindung wird analog zu Beispiel 1d aus 3-[4-Acetyl-3-hydroxy-2-(3,3,3-trifluorpropyl)-phenoxy]-propyltriphenylphosphoniumbromid (Beispiel 48b) und (4R,5R)-4,5-Epoxy-5-(3-methylphenyl)-pent-2(E)-enal (Beispiel 9b) hergestellt, hellgelbes Oel; $R_f$ = 0.38 (Hexan/Essigester = 3:2).

Beispiel 51: (1R,2S)-1-Hydroxy-1-(3-methylphenyl)-8-[4-acetyl-3-hydroxy-2-(3,3,3-trifluorpropyl)-phenoxy]-octa-3(E)5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 50) hergestellt; Smp. 233-235°; $[\alpha]_D^{20}$ (Methanol, 0.195 %) = 69.7 ± 5.1°; UV (MeOH): $\lambda_{max}(\epsilon)$ = 218 (52320), 230 (sh), 267 (29040), 280 (sh), 320 (16000).

Beispiel 52: (1R,2S)-1-Hydroxy-1-(2-trifluormethylphenyl)-8-(4-acetyl-3-hydroxy-2-propyl-phenoxy)-octa-3(E)-,5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(2-trifluormethylphenyl)-8-(4-acetyl-3-hydroxy-2-propylphenoxy)-octa-3(E),5(Z)-dien hergestellt; Smp. 66-68° $R_f$ = 0.23 (Hexan/Essigester = 3:2). $[\alpha]_D^{20}$ (Methanol, 0.150 %) = 22.0 ± 6.7°; UV (MeOH): $\lambda_{max}(\epsilon)$ = 216 (50000), 238 (sh), 271 (27860), 285 (sh), 324 (13900).

Das Ausgangsmaterial wird z.B. wie folgt hergestellt:

a) (1R,2R)-1,2-Epoxy-1-(2-trifluormethylphenyl)-8-(4-acetyl-3-hydroxy-2-propyl-phenoxy)-octa-3(E),5(Z)-dien.

Die Titelverbindung wird analog zu Beispiel 1d aus 3-(4-Acetyl-3-hydroxy-2-propyl-phenoxy)-propyltriphenylphosphoniumbromid (Beispiel 1c) und (4R,5R)-4,5-Epoxy-5-(2-trifluormethylphenyl)-pent-2(E)-enal hergestellt; hellbraunes Oel; $[\alpha]_D^{20}$ (Chloroform, 0.207 %) = -5.8 ± 4.8° $R_f$ = 0.25 (Hexan/Essigester = 4:1).

b) (4R,5R)-4.5-Epoxy-5-(2-trifluormethylphenyl)-pent-2(E)-enal.

Die Titelverbindung wird analog zu Beispiel 1b aus (2R,3R)-2,3-Epoxy-3-(2-trifluormethylphenyl)-propanol hergestellt; gelbe Kristalle; $R_f$ = 0.36 (Hexan/Essigester = 4:1). $[\alpha]_D^{20}$ (Methanol, 0.165 %) = 23.0 ± 6.1; UV (MeOH): $\lambda_{max}(\epsilon)$ = 216 (14400), 235 (17240).

c) (2R,3R)-2,3-Epoxy-3-(2-trifluormethylphenyl)-propanol.

Die Titelverbindung wird analog zu Beispiel 1a aus 3-(2-Trifluormethylphenyl)-prop-2(E)-enol hergestellt; farblose Kristalle; $R_f$ = 0.38 (Hexan/Essigester = 3:2); IR (Methylenchlorid]: 3600, 3050, 2990, 2920, 2870, 1610, 1585, 1320, 1170, 1125 $cm^{-1}$.

Beispiel 53: (1R,2S)-1-Hydroxy-1-(2-trifluormethylphenyl)-8-(4-acetyl-3-hydroxy-2-propyl-phenoxy)-octa-3(E)-,5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 52) hergestellt; Smp. 155-157°; $[\alpha]_D^{20}$ (Methanol, 0.180 %) = 12.8 ± 5.6°; UV (MeOH): $\lambda_{max}(\epsilon)$ = 219 (48400), 230 (sh), 266 (25480), 284 (22540), 325 (sh).

Beispiel 54: (1S,2R)-1-Hydroxy-1-(2-trifluormethylphenyl)-8-(4-acetyl-3-hydroxy-2-propyl-phenoxy)-octa-3(E)-,5(Z)-dien-2-yl-7-thio-4-oxo-4H-1-benzopyran-2-carbonsäuremethylester.

Die Titelverbindung wird analog zu Beispiel 1 aus (1S,2S)-1,2-Epoxy-1-(2-trifluormethylphenyl)-8-(4-acetyl-3-hydroxy-2-propyl-phenoxy)-octa-3(E),5(Z)-dien hergestellt; Smp. 71-73°; $R_f$ = 0.25 (Hexan/Essigester = 3:2). $[\alpha]_D^{20}$ (Methanol, 0.170 % = -27.1 ± 5.9°; UV (MeOH): $\lambda_{max}(\epsilon)$ = 216 (51040), 235 (sh), 271 (28140), 285 (sh), 324 (13500).

Das Ausgangsmaterial wird z.B. wie folgt hergestellt:

a) (1S,2S)-1,2-Epoxy-1-(2-trifluormethylphenyl)-8-(4-acetyl-3-hydroxy-2-propyl-phenoxy)-octa-3(E),5(Z)-dien.

Die Titelverbindung wird analog zu Beispiel 1d aus 3-(4-Acetyl-3-hydroxy-2-propyl-phenoxy)propyl-triphenylphosphoniumbromid (Beispiel 1c) und (4S,5S)-4,5-Epoxy-5(2-trifluormethylphenyl)-pent-2(E)-enal hergestellt; rötliches Oel; $[\alpha]_D^{20}$ (Chloroform, 0.207 %) = 5.4 ± 4.8°;

$R_f$ = 0.29 (Hexan/Essigester = 4:1).

b) (4S,5S)-4,5-Epoxy-5-(2-trifluormethylphenyl)-pent-2(E)-enal. Die Titelverbindung wird analog zu Beispiel 1b aus (2S,3S)-2,3-Epoxy-3-(2-trifluormethylphenyl)-propanol hergestellt; gelbe Kristalle; $R_f$ = 0.38 (Hexan/Essigester = 4:1); $[\alpha]_D^{20}$ (Methanol, 0,180 %) = -25.0 ± 5.6°; UV (MeOH): $\lambda_{max}(\epsilon)$ = 215 (13960), 236 (17060).

c) (2S,3S)-2,3-Epoxy-3-(2-trifluormethylphenyl)-propanol.

Die Titelverbindung wird analog zu Beispiel 1a aus 3-(2-Trifluormethyl-phenyl)-prop-2(E)-enol hergestellt; farblose Kristalle; $R_f$ = 0,35 (Hexan/Essigester = 3:2). IR (Methylenchlorid): 3600, 3050, 2990, 2920, 2870, 1610, 1585, 1320, 1170, 1125 $cm^{-1}$.

Beispiel 55: (1S,2R)-1-Hydroxy-1-(2-trifluormethylphenyl)-8-(4-acetyl-3-hydroxy-2-propyl-phenoxy)-octa-3(E)-,5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 54) hergestellt; Smp. 182-184°; $[\alpha]_D^{20}$ (Methanol, 0.205 %) = -16.6 ± 4.9°; UV (MeOH): $\lambda_{max}(\epsilon)$ = 219 (47680), 235 (sh), 266 (24960), 284 (22100), 330 (sh).

Beispiel 56: (1R,2S)-1-Hydroxy-1-(4-trifluormethylphenyl)-8-(4-acetyl-3-hydroxy-2-propyl-phenoxy)-octa-3(E)-,5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(4-trifluormethylphenyl)-8-(4-acetyl-3-hydroxy-2-propyl-phenoxy)-octa-3(E),5(Z)-dien hergestellt; Smp. 68-70° $R_f$ = 0.16 (Hexan/Essigester = 3:2). $[\alpha]_D^{20}$ (Methanol, 0.155 %) = 110.3 ± 6.5°; UV (MeOH): $\lambda_{max}(\epsilon)$ = 217 (52880), 236 (sh), 270 (28880), 285 (sh), 326 (13700).

Das Ausgangsmaterial wird z.B. wie folgt hergestellt:

a) (1R,2R)-1,2-Epoxy-1-(4-trifluormethylphenyl)-8-(4-acetyl-3-hydroxy-2-propyl-phenoxy)-octa-3(E),5(Z)-dien.

Die Titelverbindung wird analog zu Beispiel 1d aus 3-(4-Acetyl-3-hydroxy-2-propyl-phenoxy)propyl-triphenylphosphoniumbromid (Beispiel 1c) und (4R,5R)-4,5-Epoxy-5-(4-trifluormethylphenyl)-pent-2-(E)-enal hergestellt; gelbes Oel; $[\alpha]_D^{20}$ (Chloroform, 0.220 %) = 6.5 ± 4.5°;

$R_f$ = 0.35 (Hexan/Essigester = 4:1).

b) (4R,5R)-4,5-Epoxy-5-(4-trifluormethylphenyl)-pent-2(E)-enal.

Die Titelverbindung wird analog zu Beispiel 1b aus (2R,3R)-2,3-Epoxy-3-(4-trifluormethylphenyl)-propanol hergestellt; gelbe Kristalle; Smp. 67-70°. $R_f$ = 0.24 (Hexan/Essigester = 4:1). $[\alpha]_D^{20}$ (Methanol, 0.150 %) = 171.3 ± 6.7°; UV(MeOH): $\lambda_{max}(\epsilon)$ = 237 (19660).

c) (2R,3R)-2,3-Epoxy-3-(4-trifluormethylphenyl)-propanol.

Die Titelverbindung wird analog zu Beispiel 1a aus 3-(4-Trifluormethylphenyl)-prop-2(E)-enol hergestellt; farblose Kristalle; $R_f$ = 0.25 (Hexan/Essigester = 3:2). IR (Methylenchlorid): 3550, 3010, 2950, 2880,

2830, 1605, 1310, 1150, 1110, 1050 $cm^{-1}$.

Beispiel 57: (1R,2S)-1-Hydroxy-1-(4-trifluormethylphenyl)-8-(4-acetyl-3-hydroxy-2-propyl-phenoxy)-octa-3(E)-,5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 56) hergestellt; Smp. 229-231°; $[\alpha]_D^{20}$ (Methanol, 0.160 %) = 118.8 ± 6.3°; UV (MeOH): $\lambda_{max}(\epsilon)$ = 220 (53060), 235 (sh), 267 (27280), 284 (23880), 320 (16300).

Beispiel 58: (1S,2R)-1-Hydroxy-1-(4-trifluormethylphenyl)-8-(4-acetyl-3-hydroxy-3-propyl-phenoxy)-octa-3(E)-,5(Z)-dien-2-yl-7-thio-4-oxo-4H-1-benzopyran-2-carbonsäuremethylester.

Die Titelverbindung wird analog zu Beispiel 1 aus (1S,2S)-1,2-Epoxy-1-(4-trifluormethylphenyl)-8-(4-acetyl-3-hydroxy-2-propyl-phenoxy)-octa-3(E),5(Z)-dien hergestellt; Smp. 67-69°; $R_f$ = 0.13 (Hexan/Essigester = 3:2). $[\alpha]_D^{20}$ (Methanol, 0.155 %) = -109.7 ± 6.5°; UV (MeOH): $\lambda_{max}(\epsilon)$ = 217 (52640), 235 (sh), 270 (28660), 285 (sh), 326 (13680).

Das Ausgangsmaterial wird z.B. wie folgt hergestellt:

a) (1S,2S)-1,2-Epoxy-1-(4-trifluormethylphenyl)-8-(4-acetyl-3-hydroxy-2-propyl-phenoxy)-octa-3(E),5(Z)-dien.
Die Titelverbindung wird analog zu Beispiel 1d aus 3-(4-Acetyl-3-hydroxy-2-propyl-phenoxy)propyl-triphenylphosphoniumbromid (Beispiel 1c) und (4S,5S)-4,5-Epoxy-5-(4-trifluormethylphenyl)-pent-2(E)-enal hergestellt; rötliches Oel; $[\alpha]_D^{20}$ (Chloroform, 0.199 %) = -5.4 ± 5.0°
$R_f$ = 0.23 (Hexan/Essigester = 4:1).

b) (4S,5S)-4,5-Epoxy-5-(4-trifluormethylphenyl)-pent-2(E)-enal.
Die Titelverbindung wird analog zu Beispiel 1b aus (2S,3S)-2,3-Epoxy-3-(4-trifluormethylphenyl)-propanol hergestellt; gelbe Kristalle; Smp. 67-69°. $R_f$ = 0.18 (Hexan/Essigester = 4:1). $[\alpha]_D^{20}$ (Methanol, 0.150 %) = -180.0 ± 6.7°; UV (MeOH): $\lambda_{max}(\epsilon)$ = 236 (19740).

c) (2S,3S)-2,3-Epoxy-3-(4-trifluormethylphenyl)-propanol.
Die Titelverbindung wird analog zu Beispiel 1a aus 3-(4-Trifluormethylphenyl)-prop-2(E)-enol hergestellt; farblose Kristalle; $R_f$ = 0.23 (Hexan/Essigester = 3:2). IR (Methylenchlorid): 3550, 3010, 2950, 2880, 2830, 1605, 1310, 1150, 1110, 1050 $cm^{-1}$.

Beispiel 59: (1S,2R)-1-Hydroxy-1-(4-trifluormethylphenyl)-8-(4-acetyl-3-hydroxy-2-propyl-phenoxy)-octa-3(E)-,5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 58) hergestellt; Smp. 228-230°; $[\alpha]_D^{20}$ (Methanol, 0.175 %) = -99.4 ± 5.7°; UV (MeOH): $\lambda_{max}(\epsilon)$ = 220 (49800), 235 (sh), 267 (25320), 284 (22200), 320 (15600).

Beispiel 60: (1R,2S)-1-Hydroxy-1-(3-trifluormethylphenyl)-8-(4-acetyl-3-hydroxy-2-propyl-phenoxy)-octa-3(Z)-en-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-trifluormethylphenyl)-8-(4-acetyl-3-hydroxy-2-propyl-phenoxy)-octa-3(Z)-en hergestellt; farbloses Oel; $R_f$ = 0.41 (Hexan/Essigester = 1:1). $[\alpha]_D^{20}$ (Chloroform, 0.150 %) = 46.7 ± 6.7°; UV (MeOH): $\lambda_{max}(\epsilon)$ = 271 (22760), 288 (20060), 270 (28880), 324 (14460).

Das Ausgangsmaterial wird z.B. wie folgt hergestellt:

a) (1R,2R)-1,2-Epoxy-1-(3-trifluormethylphenyl)-8-(4-acetyl-3-hydroxy-2-propyl-phenoxy)-octa-3(Z)-en.
Die Titelverbindung wird analog zu Beispiel 1d aus 3-(4-Acetyl-3-hydroxy-2-propyl-phenoxy)pentyl-triphenylphosphoniumbromid (Beispiel 29a) und (2S,3R)-2,3-Epoxy-3-(3-trifluormethylphenyl)-propanal (Beispiel 5a) hergestellt; hellgelbes Oel; $[\alpha]_D^{20}$ (Chloroform, 0.221 %) = 25.3 ± 4.5°; $R_f$ = 0.56 (Hexan/Essigester = 3:2).

Beispiel 61: (1R,2S)-1-Hydroxy-1-(3-trifluormethylphenyl)-8-(4-acetyl-3-hydroxy-2-propyl-phenoxy)-octa-3(Z)-en-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 60) hergestellt; Smp. 231-233°; $[\alpha]_D^{20}$ (Methanol, 0,190 %) = 51 1 ± 5.3°; UV (MeOH): $\lambda_{max}(\epsilon)$ = 215 (42320),

267 (22220), 286 (20800), 320 (sh).

Beispiel 62: (1R,2S)-1-Hydroxy-1-(3-trifluormethylphenyl)-9-(4-acetyl-3-hydroxy-2-propylphenoxy)-nona-3(E)-,5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-trifluormethylphenyl)-9-(4-acetyl-3-hydroxy-2-propylphenoxy)-nona 3(E),5(Z)-dien hergestellt; hellgelbes zähes Oel; $[\alpha]_D^{20}$ (Chloroform, 0.155 %) = 44.5 ± 6.50°; $R_f$ = 0.50 (Hexan/Essigester = 1:1); UV($CHCl_3$): $\lambda_{max}(\epsilon)$ = 270 (26800), 284 (23100), 323 (14480)

Das Ausgangsmaterial wird z.B. wie folgt hergestellt:

a) 3-(4-Acetyl-3-hydroxy-2-propyl-phenoxy)butyl-triphenylphosphoniumbromid.

Die Titelverbindung wird analog zu Beispiel 1c aus 3-(4-Acetyl-3-hydroxy-2-propyl-phenoxy)butylbromid hergestellt. Smp. 167-169°.

b) (1R,2R)-1,2-Epoxy-1-(3-trifluormethylphenyl)-9-(4-acetyl-3-hydroxy-2-propylphenoxy)-nona-3(E),5(Z)-dien.

Die Titelverbindung wird analog zu Beispiel 1d aus 5-(4-Acetyl-3-hydroxy-2-propylphenoxy)butyl-triphenylphosphoniumbromid und (4R,5R)-4,5-Epoxy-5-(3-trifluormethylphenyl)-pent-2(E)-enal (Beispiel 1b) hergestellt; hellgelbes Oel; $[\alpha]_D^{20}$ (Chloroform, 0.308 %) = 40.7 ± 3.2°; $R_f$ = 0.70 (Hexan/Essigester = 3:2); IR (Methylenchlorid): 2950, 2860, 1625, 1325, 1120 $cm^{-1}$.

Beispiel 63: (1R,2S)-1-Hydroxy-1-(3-trifluormethylphenyl)-9-(4-acetyl-3-hydroxy-2-propylphenoxy)-nona-3(E)-,5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 62) hergestellt; Smp. 204-206°; $[\alpha]_D^{20}$ (Chloro-form, 0.289 %) = 8.0 ± 3.5°; $[\alpha]_D^{20}$ = 55.5 ± 6.5° (Methanol, 0.155 %); UV (Methanol): $\lambda_{max}(\epsilon)$ = 219 (49320), 232 (sh), 266 (25800), 285 (22060), 330 (sh).

Beispiel 64: (1R,2S)-1-Hydroxy-1-(3-trifluormethylphenyl)-11-(4-acetyl-3-hydroxy-2-propylphenoxy)-undeca-3(E),5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-trifluormethylphenyl)-11-(4-acetyl-3-hydroxy-2-propylphenoxy)-undeca-3(E),5(Z)-dien hergestellt, hellgelbes zähes Oel;$[\alpha]_D^{20}$ (Chloroform, 0.160 %) = 48.1 ± 6.3°; $R_f$ = 0.50 (Hexan/Essigester = 1:1). UV ($CHCl_3$): $\lambda_{max}(\epsilon)$ = 270 (27120), 286 (23300), 323 (14740).

Das Ausgangsmaterial wird z.B. wie folgt hergestellt:

a) 3-(4-Acetyl-3-hydroxy-2-propyl-phenoxy)hexyl-triphenylphosphoniumbromid.

Die Titelverbindung wird analog zu Beispiel 1c aus 3-(4-Acetyl-3-hydroxy-2-propyl-phenoxy)hexylbromid hergestellt; Verbindung kristallisiert sehr langsam.

b) (1R,2R)-1,2-Epoxy-1-(3-trifluormethylphenyl)-11-(4-acetyl-3-hydroxy-2-propylphenoxy)-undeca-3(E),5-(Z)-dien.

Die Titelverbindung wird analog zu Beispiel 1d aus 5-(4-Acetyl-3-hydroxy-2-propylphenoxy)hexyl-triphenylphosphoniumbromid und (4R,5R)-4,5-Epoxy-5-(3-trifluormethylphenyl)-pent-2(E)-enal (Beispiel 1b) hergestellt; hellgelbes Oel; $[\alpha]_D^{20}$ (Chloroform, 0.450 %) = 41.1 ± 2.2°; $R_f$ = 0.66 (Hexan/Essigester = 3:2); IR (Methylenchlorid): 2960, 2860, 1625, 1325, 1120 $cm^{-1}$.

Beispiel 65: (1R,2S)-1-Hydroxy-1-(3-trifluormethylphenyl)-11-(4-acetyl-3-hydroxy-2-propylphenoxy)-undeca-3(E),5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 64) hergestellt; Smp. 216-218°; $[\alpha]_D^{20}$ (Chloroform, 0.258 %) = 34.9 ± 3.9°; $[\alpha]_D^{20}$ = 73.8 ± 6.3° (Methanol, 0.160 %); UV (Methanol): $\lambda_{max}(\epsilon)$ = 219 (50140), 232 (sh), 266 (26120), 286 (22460), 320 (15600).

Beispiel 66: (1R,2S)-1-Hydroxy-1-phenyl-8-(4-acetyl-3-hydroxy-2-propyl-phenoxy)-octa-3(E),5(Z)-dien-2-yl-7-thio-4-oxo-4H-1-benzo-pyran-2-carbonsauremethylester.

Die Titelverbindung wird analog zu Beispiel 1 aus (1R,2R)-1,2-Epoxy-1-phenyl-8-(4-acetyl-3-hydroxy-2-propyl-phenoxy)-octa-3 (E),5(Z)-dien hergestellt; hellgelber Schaum; $R_f$ = 0.41 (Hexan/Essigester = 1:1); $[\alpha]_D^{20}$ = 47.3 ± 2.6° (Chloroform, 0.385 %).

Das Ausgangsmaterial wird z.B. wie folgt hergestellt:

a) (1R,2R)-1,2-Epoxy-1-phenyl-8-(4-acetyl-3-hydroxy-2-propyl-phenoxy)-octa-3 (E),5(Z)-dien.

Die Titelverbindung wird analog zu Beispiel 1d aus 3-(4-Acetyl-3-hydroxy-2-propyl-phenoxy)propyl-triphenylphosphoniumbromid (Beispiel 1c) und (4R,5R)-4,5-Epoxy-5-phenyl-pent-2(E)-enal hergestellt; hellgelbes Oel; $R_f$ = 0.60 (Hexan/Essigester = 3:2).

b) (4R,5R)-4,5-Epoxy-5-phenyl-pent-2(E)-enal.

Die Titelverbindung wird analog zu Beispiel 1b aus (2R,3R)-2,3-Epoxy-3-phenyl-propanol hergestellt; gelbes Oel, das beim Stehenlassen kristallisiert; $R_f$ = 0.38 (Hexan/Essigester = 3:2); $[\alpha]_D^{20}$ = 185 ± 5.0° (Chloroform, 0.200 %)

c) (2R,3R)-2,3-Epoxy-3-phenyl-propanol.

Die Titelverbindung wird analog zu Beispiel 1a aus 3-Phenyl-prop-2(E)-enol hergestellt; farbloses Oel, das in der Kälte kristallisiert. $R_f$ = 0.49 (Hexan/Essigester = 1:1); IR (Methylenchlorid): 3590, 3040, 2980, 2920, 2870, 1605, 1080, 1070 $cm^{-1}$. $[\alpha]_D^{20}$ (Chloroform, 0.279 %) = 47.7 ± 3.6°;

Beispiel 67: (1R,2S)-1-Hydroxy-1-phenyl-8-(4-acetyl-3-hydroxy-2-propyl-phenoxy)-octa-3(E),5(Z)-dien-2-yl-7-thio-4-oxo-4H-1-benzo-pyran-2-carbonsäure-Natriumsalz.

Die Titelverbindung wird analog zu Beispiel 2 aus dem entsprechenden Methylester (Beispiel 66) hergestellt; Smp. 219-221°; $[\alpha]_D^{20}$ (Chloroform, 0.160 %) = 103.1 ± 6.3°; UV (Methanol): $\lambda_{max}(\epsilon)$ = 221 (51180), 232 (sh), 267 (27040), 284 (23840), 321 (16200); UV (Chloroform):
$\lambda_{max}(\epsilon)$ = 274 (26080), 286 (sh), 330 (sh).

Beispiel 68: (1R,2S)-1-Hydroxy-1-(3-trifluormethylphenyl)-8-(3-acetyl-4-hvdroxy-5-propyl-phenoxy)-octa-3(E)-,5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-trifluormethylphenyl)-8-(3-acetyl-4-hydroxy-5-propyl-phenoxy)-octa-3(E),5(Z)-dien hergestellt; $R_f$ = 0.21 (Hexan/Aethylacetat, 3:2).

Das Ausgangsmaterial wird z.B. wie folgt hergestellt:

a) 3-(3-Acetyl-4-hydroxy-5-propyl-phenoxy)-propylbromid

Zu einer Lösung von 5.8 g 2,5-Dihydroxy-3-propyl-acetophenon und 6.1 ml 1,3-Dibrompropan in 60 ml Methyläthylketon gibt man 6.2 g Kaliumcarbonat und 0.5 g Kaliumjodid Das Reaktionsgemisch wird 24 Stunden zum Rückfluss erhitzt. Dann wird auf 300 ml Eiswasser gegossen, mit Salzsäure sauer gestellt und mit Dichlormethan (3 x 150 ml) extrahiert. Die vereinigten Extrakte werden mit 50 ml Wasser gewaschen und über Natriumsulfat getrocknet. Nach Filtration und Eindampfen im Vakuum wird der Rückstand an 400 g Kieselgel mit Dichlormethan chromatographiert. In der ersten Fraktion eluiert man die Titelverbindung, die nach Eindampfen in Form hellgelber Kristalle vom Smp. 69-70° anfällt.

b) 3-(3-Acetyl-4-hydroxy-5-propyl-phenoxy)propyl-triphenylphosphoniumbromid

Die Titelverbindung wird analog zu Beispiel 1c) aus 3-(3-Acetyl-4-hydroxy-5-propyl-phenoxy)-propylbromid und Triphenylphosphin hergestellt; Smp. 103-105°.

c) (1R,2R)-1,2-Epoxy-1-(3-trifluormethylphenyl)-8-(3-acetyl-4-hydroxy-5-propyl-phenoxy)-octa-3(E),5(Z)-dien

Die Titelverbindung wird analog zu Beispiel 1d) aus 3-(3-Acetyl-4-hydroxy-5-propyl-phenoxy)propyl-triphenylphosphoniumbromid und (4R,5R)-4,5-Epoxy-5-(3-trifluormethylphenyl)-pent-2(E)-enal hergestellt; $R_f$ = 0.54 (Hexan/Ethylacetat, 2:1).

Beispiel 69: (1R,2S)-1-Hydroxy-1-(3-trifluormethylphenyl)-8-(3-acetyl-4-hydroxy-5-propyl-phenoxy)-octa-3(E)-,5(Z)-dien-2-yl-7-thio-4-oxo-4H-1-benzopyran-2-carbonsäure-Natriumsalz.

Die Titelverbindung wird analog zu Beispiel 2 aus (1R,2S)-1-Hydroxy-1-(3-trifluormethylphenyl)-8-(3-acetyl-4-hydroxy-5-propyl-phenoxy)-octa-3(E),5(Z)-dien-2-yl-7-thio-4-oxo-4H-1-benzopyran-2-carbonsäuremethylester hergestellt; gelber Schaum; 1H-NMR ($CD_3OD$): $\delta$ = 7.91, 7.76-7.56, 7.50, 7.36, 7.05, 6.82, 6.41, 6.00, 5.75, 5.47, 5.12, 4.45, 3.78, 2.65-2.35, 1.88, 1.58, 0.94 ppm.

Beispiel 70: (1R,2S)-1-Hydroxy-1-(3-chlorphenyl)-8-(4-acetyl-3-hvdroxy-2-propyl-phenoxy)-octa-3(E),5(Z)-dien-2-yl-7-thio-4-oxo-4H-1-benzopyran-2-carbonsäuremethylester.

Die Titelverbindung wird analog Zu Beispiel 1 aus (1R,2R)-1,2-Epoxy-1-(3-chlorphenyl)-8-(4-acetyl-3-hydroxy-2-propyl-phenoxy)-octa-3-(E),5(Z)-dien hergestellt; Smp. 76-77°; $[\alpha]_D^{20}$ (Chloroform, 0.215 %) =

51.2 ± 4.7°; UV (Chloroform): $\lambda_{max}(\epsilon)$ = 271 (28160), 285 (sh), 321 (15380).

Das Ausgangsmaterial wird z.B. wie folgt hergestellt:

a) (1R,2R)-1,2-Epoxy-1-(3-chlorphenyl)-8-(4-acetyl-3-hydroxy-2-propyl-phenoxy)-octa-3(E),5(Z)-dien.

Die Titelverbindung wird analog zu Beispiel 1d aus 3-(4-Acetyl-3-hydroxy-2-propyl-phenoxy)propyltriphenylphosphoniumbromid (Beispiel 1c) und (4R,5R)-4,5-Epoxy-5-(3-chlorphenyl)-pent-2(E)-enal hergestellt; hellgelbes Oel; $[\alpha]_D^{20}$ (Chloroform, 0.541 %) = 63.9 ± 1.8°.

b) (4R,5R)-4,5-Epoxy-5-(3-chlorphenyl)-pent-2(E)-enal.

Die Titelverbindung wird analog zu Beispiel 1b aus (2R,3R)-2,3-Epoxy-3-(3-chlorphenyl)-propanol hergestellt; dunkelgelbes Oel; $R_f$ = 0.23 (Hexan/Essigester = 4:1). $[\alpha]_D^{20}$ (Chloroform, 0.30 %) = 184.7 ± 3.3.

c) (2R,3R)-2,3-Epoxy-3-(3-chlorphenyl)-propanol.

Die Titelverbindung wird analog zu Beispiel 1a aus 3-(3-Chlorphenyl)-prop-2(E)-enol hergestellt; hellgelbes Oel; $R_f$ = 0.22 (Hexan/Essigester = 7:3). IR (Methylenchlorid): 3580, 3040, 2980, 2910, 2860, 1600, 1570, 1070 $cm^{-1}$. $[\alpha]_D^{20}$ (Chloroform, 0.334 %) = 47.3 ± 3.0°.

Beispiel 71: (1R,2S)-1-Hydroxy-1-(3-chlorphenyl)-8-(4-acetyl-3-hydroxy-2-propyl-phenoxy)-octa-3(E),5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 70) hergestellt; Smp. 217-9°; $[\alpha]_D^{20}$ = (Methanol, 0,160 %) = 107.5 ± 6.3°; UV (Methanol): $\lambda_{max}(\epsilon)$ = 219 (55060), 235 (sh), 267 (27340), 284 (23920), 320 (16500).

Beispiel 72: (1R,2S)-1-Hydroxy-1-(3-chlorphenyl)-10-(4-acetyl-3-hydroxy-2-propyl-phenoxy)-deca-3(E),5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-chlorphenyl)-10-(4-acetyl-3-hydroxy-2-propyl-phenoxy)-deca-3(E),5(Z)-dien hergestellt; Smp. 61-62°.
$[\alpha]_D^{20}$ (Chloroform, 0.170 %) = 52.9 ± 5.9°; UV (Chloroform): $\lambda_{max}(\epsilon)$ = 271 (27120), 286 (24800), 322 (15400).

Das Ausgangsmaterial wird z.B. wie folgt hergestellt:

a) (1R,2R)-1,2-Epoxy-1-(3-chlorphenyl)-10-(4-acetyl-3-hydroxy-2-propyl-phenoxy)-deca-3(E),5(Z)-dien.

Die Titelverbindung wird analog zu Beispiel 1d aus 3(4-Acetyl-3-hydroxy-2-propyl-phenoxy)pentyl-triphenylphosphoniumbromid (Beispiel 29a) und (4R,5R)-4,5-Epoxy-5-(3-chlorphenyl)-pent-2(E)-enal (Beispiel 70b) hergestellt; hellgelbes Oel; $[\alpha]_D^{20}$ (Chloroform, 0.391 %) = 61.4 ± 2.5°.

Beispiel 73: (1R,2S)-1-Hydroxy-1-(3-chlorphenyl)-10-(4-acetyl-3-hydroxy-2-propyl-phenoxy)-deca-3(E),5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 72) hergestellt; Smp. 204-206°; $[\alpha]_D^{20}$ (Methanol, 0.205 %) = 58.5 ± 4.9°; UV (MeOH): $\lambda_{max}(\epsilon)$ = 218 (27940), 267 (13140), 285 (11600), 320 (sh).

Beispiel 74: (1R,2S)-1-Hydroxy-1-(3-methoxyphenyl)-8-(4-acetyl-3-hydroxy-2-propyl-phenoxy)-octa-3(E),5(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-methoxyphenyl)-8-(4-acetyl-3-hydroxy-2-propyl-phenoxy)-octa 3(E),5(Z)-dien hergestellt; Smp. 65-67°, UV (Chloroform: $\lambda_{max}(\epsilon)$ = 271 (30360), 285 (sh), 323 (16600).

Das Ausgangsmaterial wird z.B. wie folgt hergestellt:

a) (1R,2R)-1,2-Epoxy-1-(3-methoxyphenyl)-8-(4-acetyl-3-hydroxy-2-propyl-phenoxy)-octa-3(E),5(Z)-dien.

Die Titelverbindung wird analog zu Beispiel 1d aus 3-(4-Acetyl-3-hydroxy-2-propyl-phenoxy)propyltriphenylphosphoniumbromid (Beispiel 1c) und (4R,5R)-4,5-Epoxy-5-(3-methoxyphenyl)-pent-2(E)-enal hergestellt; hellgelbes Oel; $[\alpha]_D^{20}$ (Chloroform, 0.315 %) = 78.4 ± 3.2.

b) (4R,5R)-4,5-Epoxy-5-(3-methoxyphenyl)-pent-2(E)-enal.

Die Titelverbindung wird analog zu Beispiel 1b aus (2R,3R)-2,3-Epoxy-3-(3-methoxyphenyl)-propanol hergestellt; gelbes Oel; $R_f$ = 0.41 (Hexan/-Essigester = 3:2); $[\alpha]_D^{20}$ (Chloroform, 0.567 %) = 168.9 ± 1.8°.

c) (2R,3R)-2,3-Epoxy-3-(3-methoxyphenyl)-propanol.

Die Titelverbindung wird analog zu Beispiel 1a aus 3-(3-Methoxyphenyl)-prop-2(E)-enol hergestellt;

hellgelbes Oel; $R_f$ = 0.31 (Hexan/Essigester = 3:2). IR (Methylenchlorid): 3550, 2890, 1580, 1565, 1465, 1445, 1130 $cm^{-1}$.

Beispiel 75: (1R,2S)-1-Hydroxy-1-(3-methoxyphenyl)-8-(4-acetyl-3-hydroxy-2-propyl-phenoxy)-octa-3(E),5(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 74) hergestellt; Smp. 206-208°; $[\alpha]_D^{20}$ (Methanol, 0.293 %) = 99.7 ± 3.4°; UV (Methanol): $\lambda_{max}(\epsilon)$ = 221 (57840), 235 (sh), 268 (29360), 282 (26400), 320 (16800).

Beispiel 76: (1R,2S)-1-Hydroxy-1-(3-methoxyphenyl)-10-(4-acetyl-3-hydroxy-2-propyl-phenoxy)-deca-3(E),5-(Z)-dien-2-yl-7-thio-4-oxo-4H-1-benzopyran-2-carbonsäuremethylester.

Die Titelverbindung wird analog zu Beispiel 1 aus (1R,2R)-1,2-Epoxy-1-(3-methoxyphenyl)-10-(4-acetyl-3-hydroxy-2-propyl-phenoxy)-deca-3(E),5(Z)-dien hergestellt; Smp. 53° (Sublimiert zum Teil); UV (Chloroform): $\lambda_{max}(\epsilon)$ = 272 (29920), 285 (sh), 323 (15540); $[\alpha]_D^{20}$ (Chloroform, 0,180 %) = 44.4 ± 5.6°.

Das Ausgangsmaterial wird z.B. wie folgt hergestellt: a) (1R,2R)-1,2-Epoxy-1-(3-methoxyphenyl)-10-(4-acetyl-3-hydroxy-2-propyl-phenoxy)-deca-3(E),5(Z)-dien.

Die Titelverbindung wird analog zu Beispiel 1d aus 3-(4-Acetyl-3-hydroxy-2-propyl-phenoxy)pentyl-triphenylphosphoniumbromid (Beispiel 29a) und (4R,5R)-4,5-Epoxy-5-(3-methoxyphenyl)-pent-2(E)-enal (Beispiel 74b) hergestellt; hellgelbes Oel; $[\alpha]_D^{20}$ (Chloroform, 0.375 %) = 72.5 ± 2.7.

Beispiel 77: (1R,2S)-1-Hydroxy-1-(3-methoxyphenyl)-10-(4-acetyl-3-hydroxy-2-propyl-phenoxy)-deca-3(E),5-(Z)-dien-2-yl-7-thio-4-oxo-4H-1-benzopyran-2-carbonsäure-Natriumsalz.

Die Titelverbindung wird analog zu Beispiel 2 aus dem entsprechenden Methylester (Beispiel 76) hergestellt; Smp. 187-8°; $[\alpha]_D^{20}$ (Methanol, 0.150 %) = 72.0 ± 6.7°; UV (Methanol): $\lambda_{max}(\epsilon)$ = 222 (55780), 268 (27820), 282 (25200), 321 (16200).

Beispiel 78: (1R,2S)-1-Hydroxy-1-(3-trifluormethylphenyl)-8-(4-trifluor-acetyl-3-hydroxy-phenoxy)-octa-3(E),5-(Z)-dien-2-yl-7-thio-4-oxo-4H-1-benzopyran-2-carbonsäuremethylester

Die Titelverbindung wird analog zu Beispiel 1 aus (1R,2R)-1,2-Epoxy-1-(3-trifluormethylphenyl)-8-(4-trifluoracetyl-3-hydroxy-phenoxy)-octa-3(E),5(Z)-dien hergestellt; $R_f$ = 0.23 (Hexan/Essigester 3:2).

Das Ausgangsmaterial wird z.B. wie folgt hergestellt:

a) 3-(4-Trifluoracetyl-3-hydroxy-phenoxy)propylbromid.

Die Titelverbindung wird analog zu Beispiel 68a aus 2,4-Dihydroxy-trifluoracetophenon hergestellt und fällt als hellgelbes Oel an; IR (Dichlormethan): 3150, 2940, 1645, 1625, 1380, 1210, 1150, 1125, 1020, 940 $cm^{-1}$.

b) 3-(4-Trifluoracetyl-3-hydroxy-phenoxy)propyl-triphenylphosphonium-bromid.

Die Titelverbindung wird analog zu Beispiel 1c aus 3-(4-Trifluoracetyl-3-hydroxy-phenoxy)propylbromid und Triphenylphosphin hergestellt; Smp. 125-130°C.

c) (1R,2R)-1,2-Epoxy-1-(3-trifluormethylphenyl)-8-(4-trifluoracetyl-3-hydroxy-phenoxy)-octa-3(E),5(Z)-dien.

Die Titelverbindung wird analog zu Beispiel 1d aus 3-(4-Trifluoracetyl-3-hydroxy-phenoxy)propyl-triphenylphosphoniumbromid und (4R,5R)-4,5-Epoxy-5-(3-trifluormethylphenyl)-pent-2(E)-enal hergestellt; $R_f$ = 0,56 (Hexan/Essigester 2:1).

Beispiel 79: (1R,2S)-1-Hydroxy-1-(3-trifluormethylphenyl)-8-(4-trifluor-acetyl-3-hydroxyphenoxy)-octa-3(E),5-(Z)-dien-2-yl-7-thio-4-oxo-4H-1-benzopyran-2-carbonsaure-Natriunsalz

Zu einer auf 10°C gekühlten Lösung von 708 mg des Methylesters der Titelverbindung (vgl. Beispiel 78) in 25 ml Tetrahydrofuran gibt man 10 ml 0,1 N wässrige Natronlauge. Das Reaktionsgemisch wird 24 h bei Raumtemperatur gerührt, im Vakuum vom Lösungsmittel befreit und der Rückstand in Wasser aufgenommen. Nach Klarfiltration wird die Lösung lyophylisiert. Man erhält die Titelverbindung als olivgrünes, amorphes Pulver;

$^1$H-NMR ($CD_3OD$): $\delta$ = 7.94, 7.78-7.46, 7.36, 6.90, 6.36, 6.04, 5.74, 5.42, 5.12, 4.42, 3.86 ppm.

Beispiel 80: (1R,2S)-1-Hydroxy-1-(3-trifluormethylphenyl)-8-(4-trifluor-acetyl-3-hydroxy-2-propyl-phenoxy)-octa-3(E),5(Z)-dien-2-yl-7-thio-4-oxo-4H-1-benzopyran-2-carbonsäuremethylester

Die Titelverbindung wird analog zu Beispiel 1) aus (1R,2R)-1,2-Epoxy-1-(3-trifluormethylphenyl)-8-(4-trifluoracetyl-3-hydroxy-2-propyl-phenoxy)-octa-3(E),5(Z)-dien hergestellt; $R_f$ = 0.18 (Toluol/Essigester 5:1).

Das Ausgangsmaterial wird z.B. wie folgt hergestellt:

a) 3-(4-Trifluoracetyl-3-hydroxy-2-propyl-phenoxy)propylbromid.

Die Titelverbindung wird analog zu Beispiel 68a aus 2,4-Dihydroxy-3-propyl-trifluoracetophenon hergestellt und fällt als gelbes Oel an; IR (Dichlormethan): 3150, 2950, 2860, 1640, 1620, 1500, 1295, 1210, 1150, 1120, 1070 $cm^{-1}$.

b) 3-(4-Trifluoracetyl-3-hydroxy-2-propyl-phenoxy)propyl-triphenylphosphoniumbromid.

Die Titelverbindung wird analog zu Beispiel 1c aus 3-(4-Trifluoracetyl-3-hydroxy-2-propyl-phenoxy)-propylbromid und Triphenylphosphin hergestellt; Smp. 170-190 °C.

c) (1R,2R)-1,2-Epoxy-1-(3-trifluormethylphenyl)-8-(4-trifluoracetyl-3-hydroxy-2-propyl-phenoxy)-octa-3(E)-,5(Z)-dien.

Die Titelverbindung wird analog zu Beispiel 1d aus 3-(4-Trifluoracetyl-3-hydroxy-2-propyl-phenoxy)-propyl-triphenylphosphoniumbromid und (4R,5R)-4,5-Epoxy-5-(3-trifluormethylphenyl)-pent-2(E)-enal hergestellt;

$R_f$ = 0.55 (Hexan/Essigester 2:1).

Beispiel 81: (1R,2S)-1-Hydroxy-1-(3-trifluormethylphenyl)-8-(4-trifluor-acetyl-3-hydroxy-2-propyl-phenoxy)-octa-3(E),5(Z)-dien-2-yl-7-thio-4-oxo-4H-1-benzopyran-2-carbonsäure-Natriumsalz

Zu einer auf 10 °C gekühlten Lösung von 500 mg des Methylesters der Titelverbindung (vgl. Beispiel 80) in 20 ml Tetrahydrofuran gibt man 6,6 ml 0,1 N wässrige Natronlauge. Das Reaktionsgemisch wird 1 h bei 10 °C gerührt, im Vakuum von Tetrahydrofuran befreit und die verbleibende Lösung lyophylisiert. Man erhält die Titelverbindung als gelbgrünes, amorphes Pulver; $^1$H-NMR ($CD_3OD$): $\delta$ = 7.93, 7.77-7.60, 7.50, 7.36, 6.92, 6.43, 6.04, 5.73, 5.47, 5.13, 4.47, 4.00, 2.50, 2.38, 0.72 ppm.

Beispiel 82: (1R,2S)-1-Hydroxy-1-(3-trifluormethylphenyl)-8-(4-acetyl-3-hydroxy-2-propyl-phenylthio)-octa-3-(E),5(Z)-dien-2-yl-7-thio-4-oxo-4H-1-benzopyran-2-carbonsäuremethylester

Die Titelverbindung wird analog Beispiel 1 aus (1R,2S)-1,2-Epoxy-1-(3-trifluormethylphenyl)-8-(4-acetyl-3-hydroxy-2-propyl-phenylthio)-octa-3(E),5(Z)-dien hergestellt; $R_f$ = 0,19 (Hexan/Essigester 3:2).

Das Ausgangsmaterial wird z.B. wie folgt hergestellt:

a) 3-(4-Acetyl-3-hydroxy-2-propyl-phenylthio)propylbromid

Die Titelverbindung wird analog Beispiel 68a aus 2-Hydroxy-4-mercaptoacetophenon hergestellt: hellgelbes Oel.

b) 3-(4-Acetyl-3-hydroxy-2-propyl-phenylthio)propyl-triphenylphosphoniumbromid

Die Titelverbindung wird analog Beispiel 1 aus 3-(4-Acetyl-3-hydroxy-2-propyl-phenylthio)propylbromid und Triphenylphosphin hergestellt.

c) (1R,2S)-1,2-Epoxy-1-(3-trifluormethylphenyl)-8-(4-acetyl-3-hydroxy-2-propyl-phenylthio)-octa-3(E),5(Z)-dien

Die Titelverbindung wird analog Beispiel 1d aus 3-(4-Acetyl-3-hydroxy-2-propyl-phenylthio)propyl-triphenylphosphoniumbromid und (4R,5R)-4,5-Epoxy-5-(3-trifluormethylphenyl)-pent-2(E)-enal hergestellt.

Beispiel 83: (1R,2S)-1-Hydroxy-1-(3-trifluormethylphenyl)-8-(4-acetyl-3-hydroxy-2-propyl-phenylthio)-octa-3-(E),5(Z)-dien-2-yl-7-thio-4-oxo-4H-benzopyran-2-carbonsäure-Natriumsalz

Die Titelverbindung wird analog Beispiel 2 aus dem entsprechenden Methylester (Beispiel 82) hergestellt.

Beispiel 84: (1R,2S)-1-Hydroxy-1-(3-trifluormethylphenyl)-10-(4-acetyl-3-hydroxy-2-propyl-phenylthio)-deca-3(E),5(Z)-dien-2-yl-7-thio-4-oxo-4H-benzopyran-2-carbonsäuremethylester

Die Titelverbindung wird analog Beispiel 1 aus (1R,2S)-1,2-Epoxy-1-(3-trifluormethylphenyl)-10-(4-acetyl-3-hydroxy-2-propyl-phenylthio)-deca-3(E),5(Z)-dien hergestellt; $R_f$ = 0.17 (Hexan/Essigester 3:2).

Das Ausgangsmaterial kann z.B. wie folgt hergestellt werden:

a) 5-(4-Acetyl-3-hydroxy-2-propyl-phenylthio)pentylbromid

Die Titelverbindung wird analog Beispiel 68a aus 2-Hydroxy-4-mercaptoacetophenon hergestellt; hellgelbes Oel.

b) 5-(4-Acetyl-3-hydroxy-2-propyl-phenylthio)pentyl-triphenylphosphoniumbromid

Die Titelverbindung wird analog Beispiel 1 aus 5-(4-Acetyl-3-hydroxy-2-propyl-phenylthio)pentylbromid und Triphenylphosphin hergestellt.

c) (1R,2S)-1,2-Epoxy-1-(3-trifluormethylphenyl)-10-(4-acetyl-3-hydroxy-2-propyl-phenylthio)-deca-3(E),5-(Z)-dien

Die Titelverbindung wird analog Beispiel 1d aus 5-(4-Acetyl-3-hydroxy-2-propyl-phenylthio)pentyl-triphenylphosphoniumbromid und (4R,5R)-4,5-Epoxy-5-(3-trifluormethylphenyl)-pent-2-(E)-enol hergestellt.

Beispiel 85: (1R,2S)-1-Hydroxy-1-(3-trifluormethylphenyl)-10-(4-acetyl-3-hydroxy-2-propyl-phenylthio-deca-3-(E),5(Z)-dien-2-yl-7-thio-4-oxo-4H-benzopyran-2-carbonsäure-Natriumsalz

Die Titelverbindung wird analog Beispiel 2 aus dem entsprechenden Methylester (Beispiel 84) hergestellt.

Beispiel 86: (5R,6S)-1,1,1-Trifluor-5-hydroxy-12-(4-acetyl-3-hydroxy-2-propyl-phenoxy)-dodeca-7(E),9(Z)-dien-6-yl-7-thio-4-oxo-4H-1-benzopyran-2-carbonsäuremethylester

Die Titelverbindung wird analog zu Beispiel 1 aus (5R,6R)-5,6-Epoxy-1,1,1-trifluor-12-(4-acetyl-3-hydroxy-2-propyl-phenoxy)-dodeca-7(E),9(Z)-dien hergestellt; hellgelber Schaum; $R_f$ = 0.24 (Hexan/Essigester = 3:2)

Das Ausgangsmaterial wird z.B. wie folgt hergestellt:

a) (2R,3R)-2,3-Epoxy-7,7,7-trifluor-heptanol

Die Titelverbindung wird analog Beispiel 1a aus 7,7,7-Trifluor-hept-2(E)-enol hergestellt; hellgelbes Oel; $R_f$ = 0.38 (Hexan/Essigester = 3:2). $[\alpha]_D^{20}$ (Chloroform, 0.490 %) = 15.3 ± 2.0°. IR (Methylenchlorid: 3550. 3430, 2900, 1180, 1125 $cm^{-1}$.

b) (4R,5R)-4,5-Epoxy-9,9,9-trifluor-non-2(E)-enal

Die Titelverbindung wird analog zu Beispiel 1b aus (2R,3R)-2,3-Epoxy-7,7,7-trifluor-heptanol hergestellt; Oel, das im Kühlschrank kristallisiert. $R_f$ = 0.63 (Hexan/Essigester = 1:1). $[\alpha]_D^{20}$ (Chloroform, 0.210 %) = 19.5 ± 4.8°.

c) (5R,6R)-5,6-Epoxy-1,1,1-trifluor-12-(4-acetyl-3-hydroxy-2-propyl-phenoxy)-dodeca-7(E) ,9(Z)-dien

Die Titelverbindung wird analog zu Beispiel 1d aus 3-(4-Acetyl-3-hydroxy-2-propyl-phenoxy)-propyltriphenyl-phosphoniumbromid (Beispiel 1c) und (4R,5R)-4,5-Epoxy-9,9,9-trifluor-non-2(E)-enal hergstellt; gelbes Oel; $R_f$ = 0.56 (Hexan/Essigester = 3:2).

Beispiel 87: (5R,6S)-1,1,1-Trifluor-5-hydroxy-12-(4-acetyl-3-hydroxy-2-propyl-phenoxy)-dodeca-7(E),9(Z)-dien-6-yl-7-thio-4-oxo-4H-1-benzopyran-2-carbonsäure-Natriumsalz

Die Titelverbindung wird analog zu Beispiel 2 aus dem entsprechenden Methylester (Beispiel 86) hergestellt; Smp. 190-191°; $[\alpha]_D^{20}$ (Methanol, 0.268 % = 105.2 ± 3.7°; UV (Methanol): $\lambda_{max}(\epsilon)$ = 222 (48800), 235 (sh), 267 (25920), 285 (22920), 320 (16000).

Beispiel 88: (4R,5S)-1,1,1-Trifluor-4-hydroxy-13-(4-acetyl-3-hydroxy-2-propyl-phenoxy)-trideca-6(E),8(Z)-dien-5-yl-7-thio-4-oxo-4H-1-benzopyran-2-carbonsäuremethylester

Die Titelverbindung wird analog zu Beisipiel 1 aus (4R,5R)-4,5-Epoxy-1,1,1-trifluor-13-(4-acetyl-3-hydroxy-2-propyl-phenoxy)-trideca-6(E),8(Z)-dien hergestellt; gelbes Oel.

Das Ausgangsmaterial wird z.B. wie folgt hergestellt:

a) (4R,5R)-4,5-Epoxy-8,8,8-trifluor-oct-2(E)-enal

Die Titelverbindung wird analog zu Beispiel 1b aus (2R,3R)-2,3-Epoxy-6,6,6-trifluor-hexanol hergestellt; hellgelbes Oel, das im Kühlschrank kristallisiert; $R_f$ = 0.53 (Hexan/Essigester = 3:2). $[\alpha]_D^{20}$ (Chloroform, 0.290 %) = 21.7 ± 3.4°. IR (Methylenchlorid): 3050, 2980, 2930, 2810, 2730, 1690, 1640, 1145 $cm^{-1}$.

b) (4R,5R)-4,5-Epoxy-1,1,1-trifluor-13-(4-acetyl-3-hydroxy-2-propyl-phenoxy)-trideca-6(E),8(Z)-dien.

Die Titelverbindung wird analog zu Beispiel 1d aus 3-(4-acetyl-3-hydroxy-2-propyl-phenoxy)pentyl-triphenylphosphoniumbromid (Beispiel 29a) und (4R,5R)-4,5-Epoxy-8,8,8-trifluor-oct-2(E)-enal hergestellt; gelbes Oel; $R_f$ = 0.69 (Hexan/Essigester = 3:2).

Beispiel 89: (4R,5R)-1,1,1-Trifluor-4-hydroxy-13-(4-acetyl-3-hydroxy-2-propylphenoxy)-trideca-6(E),8(Z)-dien-5-yl-7-thio-4-oxo-4H-1-benzopyran-2-carbonsäure-Natriumsalz

Die Titelverbindung wird analog zu Beispiel 2 aus dem entsprechenden Methylester (Beispiel 88) hergestellt; Smp. 150-152°; $[\alpha]_D^{20}$ (Methanol, 0.265 %) = 72.5 ± 3.8°; UV (Methanol): $\lambda_{max}(\epsilon)$ = 221 (44680), 231 (sh), 266 (22560), 285 (20560), 330 (sh).

Beispiel 90: (4R,5S)-1,1,1-Trifluor-4-hydroxy-12-(4-acetyl-3-hydroxy-2-propyl-phenoxy)-dodeca-6(E),8(Z)-dien-5-yl-7-thio-4-oxo-4H-1-benzopyran-2-carbonsäuremethylester

Die Titelverbindung wird analog zu Beispiel 1 aus (4R,5R)-4,5-Epoxy-1,1,1-trifluor-12-(4-acetyl-3-hydroxy-2-propylphenoxy)dodeca-6(E),8(Z)-dien hergestellt; gelbes Oel; $R_f$ = 0.31 (Hexan/Essigester = 3:2).

Das Ausgangsmaterial wird z.B. wie folgt hergestellt:

a) (4R,5R)-4,5-Epoxy-1,1,1-trifluor-12-(4-acetyl-3-hydroxy-2-propyl-phenoxy)-dodeca-6(E),8(Z)-dien

Die Titelverbindung wird analog zu Beispiel 1d aus 3-(4-Acetyl-3-hydroxy-2-propyl-phenoxy)butyl-triphenylphosphoniumbromid (Beispiel 62b) und (4R,5R)-4,5-Epoxy-8,8,8-trifluor-oct-2(E)-enal (Beispiel 88a) hergestellt; gelbes Oel; $R_f$ = 0.64 (Hexan/Essigester = 3:2).

Beispiel 91: (4R,5S)-1,1,1-Trifluor-4-hydroxy-12-(4-acetyl-3-hydroxy-2-propyl-phenoxy)-dodeca-6(E),8(Z)-dien-5-yl-7-thio-4-oxo-4H-1-benzopyran-2-carbonsäure-Natriumsalz

Die Titelverbindung wird analog zu Beispiel 2 aus dem entsprechenden Methylester (Beispiel 90) hergestellt; Smp. 180-182°; $[\alpha]_D^{20}$ (Methanol), 0.292 %) = 77.1 ± 3.4°; UV (Methanol): $\lambda_{max}(\epsilon)$ = 222 (47480), 231 (sh), 267 (24840), 285 (22120), 321 (15800).

Beispiel 92: (5R,6S)-5-Hydroxy-12-(4-acetyl-3-hydroxy-2-propyl-phenoxy)-dodeca-7(E),9(Z)dien-5-yl-7-thio-4-oxo-4H-1-benzopyran-2-carbonsäuremethylester

Die Titelverbindung wird analog zu Beispiel 1 aus, (5R,6R)-5,6-Epoxy-12-(4-acetyl-3-hydroxy-2-propyl-phenoxy)-dodeca-7(E),9(Z)-dien hergestellt; hellgelber Schaum; $R_f$ = 0.43 (Hexan/Essigester = 1:1); $[\alpha]_D^{20}$ (Methanol), 0.150 %) = 22.0 ± 6.7°; IR (Methylenchlorid): 3580, 2950, 1745, 1655, 1625, 1600 $cm^{-1}$.

Das Ausgangsmaterial wird z.B. wie folgt hergestellt:

a) (4R,5R)-4,5-Epoxy-non-2(E)-enal

Die Titelverbindung wird analog zu Beispiel 1b aus (2R,3R)-2,3-Epoxy-heptanol hergestellt; gelbes Oel; $R_f$ = 0.29 (Hexan/Essigester = 4:1); $[\alpha]_D^{20}$ (Chloroform, 0.390 %) = 21.3 ± 2.6°; IR (Methylenchlorid): 2950, 2920, 2860, 1690, 1640, 1100, 970 $cm^{-1}$.

b) (5R,6R)-5,6-Epoxy-12-(4-acetyl-3-hydroxy-2-propyl-phenoxy)-dodeca-7(E),9(Z)-dien

Die Titelverbindung wird analog zu Beispiel 1d aus 3-(4-acetyl-3hydroxy-2-propyl-phenoxy)propyl-triphenylphosphoniumbromid (Beispiel 1c) und (4R,5R)-4,5-Epoxy-non-2(E)-enal hergestellt; hellgelbes Oel; $[\alpha]_D^{20}$ (Chloroform, 0.650 %) = 23.7 ± 1.5°.

Beispiel 93: (5R,6S)-5-hydroxy-12-(4-acetyl-3-hydroxy-2-propyl-phenoxy)-dodeca-7(E),9( Z)-dien-6-yl-7-thio-4-oxo-4H-1-benzopyran-2-carbonsäure-Natriumsalz

Die Titelverbindung wird analog zu Beispiel 2 aus dem entsprechenden Methylester (Beispiel 92) hergestellt; Smp. 205-207°; $[\alpha]_D^{20}$ (Methanol, 0.278 %) = 115.1 ± 3.6°; UV (Methanol): $\lambda_{max}(\epsilon)$ = 222 (50960), 232 (sh), 267 (27400), 285 (24000), 321 (16400).

Beispiel 94: (5R,6S)-5-hydroxy-12-(4-acetyl-3-hydroxy-2-propyl-phenoxy)-dodeca-7(E),9(Z)-dien-6-yl-7-thio-4-oxo-4H-1-benzopyran-2-carbonsäuremethylester

Die Titelverbindung wird analog zu Beispiel 1 aus (5S,6S)-5,6-Epoxy-12(4-acetyl-3-hydroxy-2-propyl-phenoxy)-dodeca-7(E),9(Z)-dien hergestellt; farbloser Schaum; $[\alpha]_D^{20}$ (Methanol, 0.260 %) = 136.2 ± 3.8°; UV (Methanol): $\lambda_{max}(\epsilon)$ = 221 (48040), 271 (28320), 327 (13200).

Das Ausgangsmaterial wird z.B. wie folgt hergestellt:

a) (4S,5S)-4,5-Epoxy-non-2(E)-enal

Die Titelverbindung wird analog zu Beispiel 1b aus (2S,3S)-2,3-Epoxy-heptanol hergestellt; gelbes Oel: $R_f$ = 0.27 (Hexan/Essigester = 5:1; $[\alpha]_D^{20}$ (Chloroform, 0.325 %) = 23.1 ± 3.0°.

b) (5S,6S)-5,6-Epoxy-12-(4-acetyl-3-hydroxy-2-propyl-phenoxy)-dodeca-7(E),9(Z)-dien

Die Titelverbindung wird analog zu Beispiel 1d aus 3-(4-Acetyl-3-hydroxy-2-propyl-phenoxy)-propyltriphenylphosphoniumbromid (Beispiel 1c) und (4S,5S)-4,5-Epoxy-non-2(E)-enal hergestellt; hellgelbes Oel; $[\alpha]_D^{20}$ (Chloroform, 0.600 %) = 24.8 ± 1.6°.

Beispiel 95: (5S,6R)-5-Hydroxy-12-(4-acetyl-3-hydroxy-2-propyl-phenoxy)-dodeca-7(E),9(Z )dien-6-yl-7-thio-4-oxo-4H-1-benzopyran-2-carbonsäure-Natriumsalz

Die Titelverbindung wird analog zu Beispiel 2 aus dem entsprechenden Methylester (Beispiel 94) hergestellt; Smp. 204-206°; $[\alpha]_D^{20}$ (Methanol, 0.570 %) = 121.1 ± 1.8°; UV (Methanol): $\lambda_{max}(\epsilon)$ = 222 (51240), 235 (sh), 267 (27360), 284 (21400), 320 (16400).

Beispiel 96: (5R,6S)-5-Hydroxy-14-(4-acetyl-3-hydroxy-2-propyl-phenoxy)-tetradeca-7(E),9(Z)-dien-6-yl-7-thio-4-oxo-4H-1-benzopyran-2-carbonsäuremethylester

Die Titelverbindung wird analog zu Beispiel 1 aus (5R,6R)-5,6-Epoxy-14-(4-acetyl-3-hydroxy-2-propyl-phenoxy)-tetradeca-7(E),9(Z)-dien hergestellt; hellgelbes zähes Oel; $[\alpha]_D^{20}$ (Chloroform, 0.424 %) = 66.5 ± 2.4°; UV (Chloroform): $\lambda_{max}(\epsilon)$ = 270 (28560), 288 (sh), 325 (15240).

Das Ausgangsmaterial wird z.B. wie folgt hergestellt:

a) (5R,6R)-5,6-Epoxy-14-(4-acetyl-3-hydroxy-2-propyl-phenoxy)-tetradeca-7(E),9(Z)-dien

Die Titelverbindung wird analog zu Beispiel 1d aus 3-(4-Acetyl-3-hydroxy-2-propyl-phenoxy)pentyl-triphenylphosphoniumbromid (Beispiel 29a) und (4R,5R)-4,5-Epoxy-non-2(E)-enal (Beispiel 92a) hergestellt; hellgelbes Oel; $[\alpha]_D^{20}$ (Chloroform, 0.441 %) = 20.6 ± 2.3°.

Beispiel 97: (5R,6S)-5-Hydroxy-14-(4-acetyl-3-hydroxy-2-propyl-phenoxy)-tetradeca-7(E),9(Z )-dien-6-yl-7-thio-4-oxo-4H-1-benzopyran-2-carbonsäure-Natriumsalz

Die Titelverbindung wird analog zu Beispiel 2 aus dem entsprechenden Methylester (Beispiel 96) hergestellt; Smp. 193-195°; $[\alpha]_D^{20}$ (Methanol, 0.284 %) = 71.1 ± 3.5°. UV (Methanol): $\lambda_{max}(\epsilon)$ = 222 (49320), 232 (sh), 267 (25520), 286 (22680), 321 (16000).

Beispiel 98: (5S,6R)-5-Hydroxy-14-(4-acetyl-3-hydroxy-2-propyl-phenoxy)-tetradeca-7(E),9(Z )-dien-6-yl-7-thio-4-oxo-4H-1-benzopyran-2-carbonsäuremethylester

Die Titelverbindung wird analog zu Beispiel 1 aus (5S,6S)-5,6-Epoxy-14-(4-acetyl-3-hyeroxy-2-propyl-phenoxy)-tetradeca-7(E),9(Z)-dien hergestellt; hellgelbe zähe Masse; $[\alpha]_D^{20}$ (Chloroform, 0.463 %) = 79.0 ± 2.2°. UV (Chloroform): $\lambda_{max}(\epsilon)$ = 270 (27120), 288 (sh), 326 (14960).

Das Ausgangsmaterial wird z.B. wie folgt hergestellt:

a) (5S,6S)-5,6-Epoxy-14-(4-acetyl-3-hydroxy-2-propyl-phenoxy)-tetradeca-7(E)9(Z)-dien

Die Titelverbindung wird analog zu Beispiel 1d aus 3-(4-Acetyl-3-hydroxy-2-propyl-phenoxy)pentyl-triphenylphosphoniumbromid (Beispiel 29a) und (4S,5S)-4,5-Epoxy-non-2(E)-enal (Beispiel 94a) hergestellt; hellgelbes Oel; $[\alpha]_D^{20}$ (Chloroform, 0.472 %) = 18.8 ± 2.1°.

Beispiel 99: (5S,6R)-5-Hydroxy-14-(4-acetyl-3-hydroxy-2-propyl-phenoxy)-tetradeca-7(E),9(Z)-dien-6-yl-7-thio-4-oxo-4H-1-benzopyran-2-carbonsäure-Natriumsalz

Die Titelverbindung wird analog zu Beispiel 2 aus dem entsprechenden Methylester (Beispiel 98) hergestellt; Smp. 193-195°; $[\alpha]_D^{20}$ (Methanol, 0.296 %) = 65.9 ± 3.4°. UV (Methanol): $\lambda_{max}(\epsilon)$ = 222 (49760), 232 (sh), 267 (25800), 285 (22520), 320 (16000).

Beispiel 100: (5R,6S)-1,1,1-Trifluor-5-hydroxy-14-(4-acetyl-3-hydroxy-2-propyl-phenoxy)-tetradeca-7(E),9(Z)-dien-6-yl-7-thio-4-oxo-4H-1-benzopyran-2-carbonsäuremethylester

Die Titelverbindung wird analog zu Beispiel 1 aus (5R,6R)-5,6-Epoxy-1,1,1-trifluor-14-(4-acetyl-3-hydroxy-2-propylphenoxy)-tetradeca-7(E),9(Z)-dien hergestellt; hellgelbes Oel; $R_f$ = 0.32 (Hexan/Essigester = 3:2).

Das Ausgangsmaterial wird z.B. wie folgt hergestellt:

a) (5R,6R)-5,6-Epoxy-1,1,1-trifluor-14-(4-acetyl-3-hydroxy-2-propyl-phenoxy)-tetradeca-7(E)9(Z)-dien
Die Titelverbindung wird analog zu Beispiel 1d aus 3-(4-Acetyl-3-hydroxy-2-propyl-phenoxy)pentyl-triphenylphosphoniumbromid (Beispiel 29a) und (4R,5R)-4,5-Epoxy-9,9,9-trifluor-non-2(E)-enal (Beispiel 86b) hergestellt; gelbes Oel; $R_f$ = 0.72 (Hexan/Essigester = 3:2).

Beispiel 101: (5R,6S)-1,1,1-Trifluor-5-hydroxy-14-(4-acetyl-3-hydroxy-2-propyl-phenoxy)-tetradeca-7(E),9(Z)-dien-6-yl-7-thio-4-oxo-4H-1-benzopyran-2-carbonsäure-Natriumsalz

Die Titelverbindung wird analog zu Beispiel 2 aus dem entsprechenden Methylester (Beispiel 100) hergestellt; UV (Methanol): $\lambda_{max}(\epsilon)$ = 222 (48800), 235 (sh), 267 (25920), 285 (22920), 320 (16000).

Beispiel 102: In analoger Weise wie in den Beispielen 1-77 beschrieben, kann man ferner herstellen:

(1R,2S)-1-Hydroxy-1-phenyl-11-(4-acetyl-3-hydroxy-2-propyl-phenoxy)-undeca-3(E),5(Z)-dien-2-yl-7-thio-4-oxo-4H-1-benzoypran-2-carbonsäure-Natriumsalz;
(1R,2S)-1-Hydroxy-1-phenyl-10-(4-acetyl-3-hydroxy-2-propylphenoxy)-deca-3(E),5(Z)-dien-2-yl-7-thio-4-oxo-4H-1-benzopyran-2-carbonsäure-Natriumsalz;
(1R,2S)-1-Hydroxy-1-phenyl-9-(4-acetyl-3-hydroxy-2-propyl-phenoxy)-nona-3(E),5(Z)-dien-2-yl-7-thio-4-oxo-4H-1-benzopyran-2-carbonsäure-Natriumsalz;
(1R,2S)-1-Hydroxy-1-(3-chlorphenyl)-9-(4-acetyl-3-hydroxy-2-propyl-phenoxy)-nona-3(E),5(Z)-dien-2-yl-7-thio-4-oxo-4H-1-benzopyran-2-carbonsäure-Natriumsalz;
(1R,2S)-1-Hydroxy-1-(3-chlorphenyl)-11-(4-acetyl-3-hydroxy-2-propyl-phenoxy)-undeca-3(E),5(Z)-dien-2-yl-7-thio-4-oxo-4H-1-benzopyran-2-carbonsäure-Natriumsalz;
(1R,2S)-1-Hydroxy-1-(3-fluorphenyl)-8-(4-acetyl-3-hydroxy-2-propyl-phenoxy)-octa-3(E),5(Z)-dien-2-yl-7-thio-4-oxo-4H-1-benzopyran-2-carbonsäure-Natriumsalz;
(1R,2S)-1-Hydroxy-1-(3-fluorphenyl)-9-(4-acetyl-3-hydroxy-2-propyl-phenoxy)-nona-3(E),5(Z)-dien-2-yl-7-thio-4-oxo-4H-1-benzopyran-2-carbonsäure-Natriumsalz;
(1R,2S)-1-Hydroxy-1-(3-fluorphenyl)-10-(4-acetyl-3-hydroxy-2-propyl-phenoxy)-deca-3(E),5(Z)-dien-2-yl-7-thio-4-oxo-4H-1-benzopyran-2-carbonsäure-Natriumsalz;
(1R,2S)-1-Hydroxy-1-(3-fluorphenyl)-11-(4-acetyl-3-hydroxy-2-propyl-phenoxy)-undeca-3(E),5(Z)-dien-2-yl-7-thio-4-oxo-4H-1-benzopyran-2-carbonsäure-Natriumsalz;
(1R,2S)-1-Hydroxy-1-(3-methoxyphenyl)-9-(4-acetyl-3-hydroxy-2-propyl-phenoxy)-nona-3(E),5(Z)-dien-2-yl-7-thio-4-oxo-4H-1-benzopyran-2-carbonsäure-Natriumsalz;
(1R,2S)-1-Hydroxy-1-(3-methoxyphenyl)-11-(4-acetyl-3-hydroxy-2-propyl-phenoxy)-undeca-3(E),5(Z)-dien-2-yl-7-thio-4-oxo-4H-1-benzopyran-2-carbonsäure-Natriumsalz;
(1R,2S)-1-Hydroxy-1-(3-carboxyphenyl)-8-(4-acetyl-3-hydroxy-2-propyl-phenoxy)-octa-3(E),5(Z)-dien-2-yl-7-thio-4-oxo-4H-1-benzopyran-2-carbonsäure-di-Natriumsalz;
(1R,2S)-1-Hydroxy-1-(3-carboxyphenyl)-9-(4-acetyl-3-hydroxy-2-propylphenoxy)-nona-3(E),5(Z)-dien-2-yl-7-thio-4-oxo-4H-1-benzopyran-2-carbonsäure-di-Natriumsalz;
(1R,2S)-1-Hydroxy-1-(3-carboxyphenyl)-10-(4-acetyl-3-hydroxy-2-propylphenoxy)-deca-3(E),5(Z)-dien-2-yl-7-thio-4-oxo-4H-1-benzopyran-2-carbonsäure-di-Natriumsalz;
(1R,2S)-1-Hydroxy-1-(3-carboxyphenyl)-11-(4-acetyl-3-hydroxy-2-propylphenoxy)-undeca-3(E),5(Z)-dien-2-yl-7-thio-4-oxo-4H-1-benzopyran-2-carbonsäure-di-Natriumsalz;
(1R,2S)-1-Hydroxy-1-(3-methoxycarbonylphenyl)-9-(4-acetyl-3-hydroxy-2-propyl-phenoxy)-nona-3(E),5(Z)-dien-2-yl-7-thio-4-oxo-4H-1-benzopyran-2-carbonsäure-Natriumsalz;
(1R,2S)-1-Hydroxy-1-(3-methoxycarbonylphenyl)-10-(4-acetyl-3-hydroxy-2-propyl-phenoxy)-deca-3(E),5(Z)-dien-2-yl-7-thio-4-oxo-4H-1-benzopyran-2-carbonsäure-Natriumsalz;
(1R,2S)-1-Hydroxy-1-(3-methoxycarbonylphenyl)-11-(4-acetyl-3-hydroxy-2-propyl-phenoxy)-undeca-3(E),5-(Z)-dien-2-yl-7-thio-4-oxo-4H-1-benzopyran-2-carbonsäure-Natriumsalz;
(1R,2S)-1-Hydroxy-1-(3,4-dichlorphenyl)-10-(4-acetyl-3-hydroxy-2-propyl-phenoxy)-deca-3(E),5(Z)-dien-2-yl-7-thio-4-oxo-4H-1-benzopyran-2-carbon-säure-Natriumsalz;
(1R,2S)-1-Hydroxy-1-(2,4-dichlorphenyl)-10-(4-acetyl-3-hydroxy-2-propyl-phenoxy)-deca-3(E),5(Z)-dien-2-yl-7-thio-4-oxo-4H-1-benzopyran-2-carbonsäure-Natriumsalz;
(1R,2S)-1-Hydroxy-1-(3,4-dimethoxyphenyl)-10-(4-acetyl-3-hydroxy-2-propyl-phenoxy)-deca-3(E),5(Z)-dien-2-yl-7-thio-4-oxo-4H-1-benzopyran-2-carbonsäure-Hatriumsalz;
(1R,2S)-1-Hydroxy-1-(2,4-dimethoxyphenyl)-10-(4-acetyl-3-hydroxy-2-propyl-phenoxy)-deca-3(E),5(Z)-dien-2-yl-7-thio-4-oxo-4H-1-benzopyran-2-carbonsäure-Natriumsalz;
(1R,2S)-1-Hydroxy-1-(2,4-dimethylphenyl)-10-(4-acetyl-3-hydroxy-2-propyl-phenoxy)-deca-3(E),5(Z)-dien-2-yl-7-thio-4-oxo-4H-1-benzopyran-2-carbonsäure-Hatriumsalz;
(1R,2S)-1-Hydroxy-1-(3-dimethylaminophenyl)-10-(4-acetyl-3-hydroxy-2-propyl-phenoxy)-deca-3(E),5(Z)-dien-2-yl-7-thio-4-oxo-4H-1-benzopyran-2-carbonsäure-Natriumsalz;

Beispiel 103: In analoger Weise wie in den Beispielen 1-101 beschrieben, kann man ferner folgende Verbindungen herstellen:
(1R,2S)-1-Hydroxy-1-(3-bromphenyl)-8-(4-acetyl-3-hydroxy-2-propyl-phenoxy)-octa-3(E),5(Z)-dien-2-yl-7-thio-4-oxo-4H-1-benzopyran-2-carbonsäure-Natriumsalz;
(1R,2S)-1-Hydroxy-1-(3-bromphenyl)-9-(4-acetyl-3-hydroxy-2-propyl-phenoxy)-nona-3(E),5(Z)-dien-2-yl-7-thio-4-oxo-4H-1-benzopyran-2-carbonsäure-Natriumsalz;
(1R,2S)-1-Hydroxy-1-(3-bromphenyl)-10-(4-acetyl-3-hydroxy-2-propyl-phenoxy)-deca-3(E),5(Z)-dien-2-yl-7-thio-4-oxo-4H-1-benzopyran-2-carbonsäure-Natriumsalz;
(1R,2S)-1-Hydroxy-1-(3-bromphenyl)-11-(4-acetyl-3-hydroxy-2-propyl-phenoxy)-undeca-3(E),5(Z)-dien-2-yl-7-thio-4-oxo-4H-1-benzopyran-2-carbonsäure-Natriumsalz;
(5R,6S)-1,1,1-trifluor-5-hydroxy-13-(4-acetyl-3-hydroxy-2-propyl-phenoxy)-trideca-7(E),9(Z)-dien-6-yl-7-thio-4-oxo-4H-1-benzopyran-2-carbonsäure-Natriumsalz;
(5R,6S)-1,1,1-trifluor-5-hydroxy-15-(4-acetyl-3-hydroxy-2-propylphenoxy)-pentadeca-7(E),9(Z)-dien-6-yl-7-thio-4-oxo-4H-1-benzopyran-2-carbonsäure-Natriumsalz;
(5R,6S)-5-hydroxy-13-(4-acetyl-3-hydroxy-2-propylphenoxy)-trideca-7(E),9(Z)-dien-6-yl-7-thio-4-oxo-4H-1-benzopyran-2-carbonsäure-Natriumsalz;
(5R,6S)-5-hydroxy-15-(4-acetyl-3-hydroxy-2-propylphenoxy)-pentadeca-7(E),9(Z)-dien-6-yl-7-thio-4-oxo-4H-1-benzopyran-2-carbonsäure-Natriumsalz;
(4R,5S)-1-Carboxy-4-hydroxy-11-(4-acetyl-3-hydroxy-2-propylphenoxy)-undeca-6(E),8(Z)-dien-6-yl-7-thio-4-oxo-4H-1-benzopyran-2-carbonsäure-Dinatriumsalz;
(4R,5S)-1-Carboxy-4-hydroxy-12-(4-acetyl-3-hydroxy-2-propylphenoxy)-dodeca-6(E),8(Z)-dien-5-yl-7-thio-4-oxo-4H-1-benzopyran-2-carbonsäure-Dinatriumsalz;
(4R,5S)-1-Carboxy-4-hydroxy-13-(4-acetyl-3-hydroxy-2-propylphenoxy)-trideca-6(E),8(Z)-dien-5-yl-7-thio-4-oxo-4H-1-benzopyran-2-carbonsäure-Dinatriumsalz;
(4R,5S)-1-Carboxy-4-hydroxy-14-(4-acetyl-3-hydroxy-2-propylphenoxy)-tetradeca-6(E),8(Z)dien-5-yl-7-thio-4-oxo-4H-1-benzopyran-2-carbonsäure-Dinatriumsalz.

Beispiele der pharmazeutischen Zusammensetzungen
und entsprechender fertiger Arzneimittelformen

Unter dem Ausdruck "Wirkstoff" ist nachstend eine erfindungsgemässe Verbindung der Formel I zu verstehen, insbesondere eine solche, die als Produkt in Beispielen 1-9 beschrieben ist, wie z.B. (1R,2S)-1-Hydroxy-1-(3-trifluormethylphenyl)-8-(4-acetyl-3-hydroxy-2-propylphenoxy)-octa-3(E),5(Z)-dien-2-yl-7-thio-4-oxo-4H-1-benzopyran-2-carbonsäurenatriumsalz.

Beispiel A: Eine treibmittelhaltige, feststoffaerosolbildende Inhalationssuspension enthaltend 0,1 Gew.-% Wirkstoff

Zusammensetzung:

| | Gew.-% |
|---|---|
| Wirkstoff, mikronisiert | 0,1 |
| Sorbitantrioleat | 0,5 |
| Treibmittel A (Trichlortrifluorethan) | 4,4 |
| Treibmittel B (Dichlordifluormethan und 1,2-Dichlortetrafluorethan) | 15,0<br>80,0 |

Herstellung: Der Wirkstoff wird unter Feuchtigkeitsausschluss mit Hilfe eines üblichen Homogenisators unter Zusatz des Sorbitantrioleats im Trichlortrifluorethan suspendiert, die Suspension in einen mit Dosierventil versehenen Aerosolbehälter abgefüllt; dieser wird verschlossen und unter Druck mit dem Treibmittel B aufgefüllt.

Beispiel B: Eine zur Inhalation geeignete, etwa 2%ige wässrige Lösung eines Wirkstoffes in Form dessen Natrium- oder Kalium-salzes.

Zusammensetzung

| | |
|---|---|
| Wirkstoff (K- oder Na-Salz) | 2000 mg |
| Ethylendiamintetraessigsäure-Dinatriumsalz | 10 mg |
| Benzalkoniumchlorid | 10 mg |
| Wasser, frisch destilliert ad | 100 ml |

Herstellung: Der Wirkstoff wird in etwa 60 ml frisch destilliertem Wasser gelöst und der Stabilisator (Ethylendiamintetraessigsäure-Dinatriumsalz) und das Konservierungsmittel (Benzalkoniumchlorid) hinzugegeben. Nach vollständiger Auflösung aller Komponenten wird die erhaltene Lösung auf 100 ml aufgefüllt, in Druckfläschchen abgefüllt und diese gasdicht verschlossen. Das Treibmittel wird nach Bedarf gasförmig unter Druck oder in flüssiger Form zugegeben.

ANHANG - PHARMAKOLOGISCHE TESTMETHODEN

Meerschweinchen-Bronchokonstriktionstest (in vivo, Aerosol):

Man anästhetisiert männliche, 400-700 g schwere Meerschweinchen intraperitoneal mit 1.4 g/kg Urethan und führt eine Polyethylenkanüle in die Vena jugularis ein. Eine zweite Polyethylenkanüle wird in die Trachea eingeführt. Mittels einer in die Speiseröhre eingeführten Kanüle, welche mit einem Statham-Druck-Transduktor verbunden ist, wird der Druck in der Speiseröhre aufgezeichnet. Das Tier wird in eine luftdicht verschliessbare Plexiglaskammer gelegt, die mit einer Fleisch'schen Röhre Nr. 000 und einem Validyne-Transducer MP 45-1 verbunden ist. Mit dieser Anordnung wird der Flow gemessen.

Nach der chirurgischen Präparierung der Versuchstiere wartet man eine gewisse Zeit, damit die pulmonalen Funktionen sich stabilisieren können. Die zu testende Verbindung wird anschliessend gemäss dem nachfolgenden Protokoll verabreicht. Die Versuchstiere werden während einer Minute einer 1-prozentigen Aerosollösung der zu testenden Verbindung (Gew/Vol) oder destilliertem Wasser (zu Kontrollzwecken) ausgesetzt. Für alle Testverbindungen, welche durch Inhalation verabreicht werden, verwendet man ein Monaghan-Ultraschall-Sprühgerät (Modell 670) dessen Partikelgrösse sich zwischen 1 und 8 Mikron bewegt mit einem Hauptanteil von 3 Mikron.

Wässrige Lösungen werden jeweils frisch hergestellt und mit einem On-stream drug vial in die Kammer des Sprühgeräts eingeführt. Der produzierte Sprühnebel wird den Versuchstieren über eine Glaskammer von 65 ml Inhalt, die mit einer Kanüle mit der Trachea verbunden wird, verabreicht. Nach Ablauf der Behandlungszeit wird mit einem zweiten Monaghan-Ultraschall-Sprühgerät (Modell 670) und über eine gleiche Glaskammer $LTD_4$ (0,3 µg/ml) während 2 Minuten verabreicht.

Es wird die Abnahme der Compliance in der 3. Minute nach $LTD_4$-Applikation abgelesen und zwar wird der Mittelwert von drei Tieren mit dem Mittelwert von drei Kontrolltieren verglichen und die prozentuale Hemmung der Compliance nach folgender Formel errechnet:

$$\%\ \text{Hemmung} = 100 - \frac{(100 - \text{Compliance Präparat}) \cdot 100}{(100 - \text{Compliance Kontrolle})}$$

Werden unterschiedliche Wirkstoffkonzentrationen untersucht, so wird die prozentuale Hemmung für jede Konzentration aufgezeichnet, und zwar wird der log Konzentration auf der Abszisse gegen die prozentuale Hemmung auf der Ordinate aufgetragen. Die $IC_{50}$ wird dann durch lineare Regressions-analyse ermittelt.

In vitro-Test zur Bestimmung der Hemmung der Phospholipase $A_2$ aus menschlichen Leukozyten

Neutrophile polymorphkernige menschliche Leukozyten werden ausgehend von "Buffy coats" durch mehrstufige fraktionierte Sedimentation isoliert und tiefgefroren. Die Phospholipase $A_2$ wird aus der Zellsuspension durch Homogenisieren unter Zusatz von eiskalter 0.36 N $H_2SO_4$ in 2N NaCl extrahiert und der nach Zentrifugation bei 10'000 x g erhaltene Ueberstand gegen Natriumacetatpuffer pH 4.5 dialysiert
Für die Bestimmung der Enzymaktivität wird Enzym (10-30 ug Protein) in 0.1 M Tris/HCl-Puffer pH 7 unter

Zusatz von 1 mM $CaCl_2$ und Substrat, bestehend aus biosynthetisch mit $^{14}C$-Oelsäure radioaktiv markiertem Phospholipiden (2 µM) von Escherichia coli bei 37° während 1 Stunde inkubiert. Die Reaktion wird abgestoppt durch Zugabe von Dole-Reagens (Isopropanol/Heptan/1N $H_2SO_4$ 40:10:1, v/v) und die durch Phospholipase $A_2$ selektiv freigesetzte $^{14}C$-Oelsäure extrahiert. Ebenfalls mitextrahiertes Substrat wird durch Filtration des Extraktes durch eine Säule von Kieselgel vollständig entfernt. Die Bestimmung der $^{14}C$-Oelsäure im Eluat erfolgt durch Radiometrie.

Zur Ermittlung einer Hemmwirkung von Prüfsubstanzen auf die Phospholipase $A_2$ werden diese als Lösungen in Wasser, Dimethylsulfoxid (Endkonzentration im Ansatz bis 5 %, v/v) oder Ethanol (Endkonzentration im Ansatz bis 2.5 % v/v) dem Inkubationsumsatz zugesetzt. Die Wirkungsstärke der Prüfsubstanzen wird ausgedrückt durch die $IC_{50}$, d.h. die Konzentration, welche eine Hemmung von 50 % der Kontrollaktivität bewirkt. Die $IC_{50}$ wird graphisch ermittelt durch Auftragen der prozentualen Hemmung auf der Ordinate gegen den log der Konzentration (µM) auf der Abszisse.

Unter den beschriebenen Versuchsbedingungen hemmt Mepacrin die Phospholipase $A_2$ mit einer $IC_{50}$ von 1600 µM.

In vitro-Test zur Bestimmung der Hemmung der Phospholipase C aus menschlichen Thrombozyten

Menschliche Thrombozyten werden aus "Buffy coats" durch fraktionierte Zentrifugation gewonnen und anschliessend tiefgefroren. Die Phospholipase C wird durch Ultraschallbehandlung der Zellsuspension freigesetzt und findet sich nach Ultrazentrifugation (150'000 x g während 1 Stunde) in löslicher Form im Ueberstand.

Für die Bestimmung der Enzymaktivität wird Enzym (20-100 µg Protein) in 0.025 M Tris/Maleat-Puffer pH 6 unter Zusatz von 0.2 mM $CaCl_2$ und 0.02 mM radioaktiv markiertem Substrat, Phosphatidyl-[$^{14}C$]-inosit, bei 37° während 5 Minuten inkubiert. Die Reaktion wird abgestoppt durch Ausschütteln mit $CHCl_3/CH_3OH$ 2:1 (v/v). Dabei wird unverbrauchtes Substrat in die organische Phase extrahiert, während das Reaktionsprodukt $^{14}C$-Inositphophat in der wässrigen Phase verbleibt und durch Radiometrie eines Aliquots gemessen werden kann.

Zur Ermittlung einer Hemmwirkung von Prüfsubstanzen auf die Phospholipase C werden diese als Lösungen in Wasser, Dimethylsulfoxid (Endkonzentration im Ansatz bis 5 %, v/v) oder Ethanol (Endkonzentration im Ansatz bis 2.5 %, v/v) dem Inkubationsansatz zugesetzt. Die Wirkungsstärke der Prüfsubstanzen wird ausgedrückt durch die $IC_{50}$, d.h. die Konzentration, welche eine Hemmung von 50 % der Kontrollaktivität bewirkt. Die $IC_{50}$ wird graphisch ermittelt durch Auftragen der prozentualen Hemmung auf der Ordinate gegen den log der Konzentration (µM) auf der Abszisse.

Unter den beschriebenen Versuchsbedingungen hemmt Mepacrin die Phospholipase C mit einer $IC_{50}$ von 20 µM.

**Patentansprüche**

**Patentansprüche für die folgende Vertragsstaaten : AT, BE, CH, DE, FR, GB, IT, LI, LU, NL, SE**

**1.** Verbindungen der Formel I,

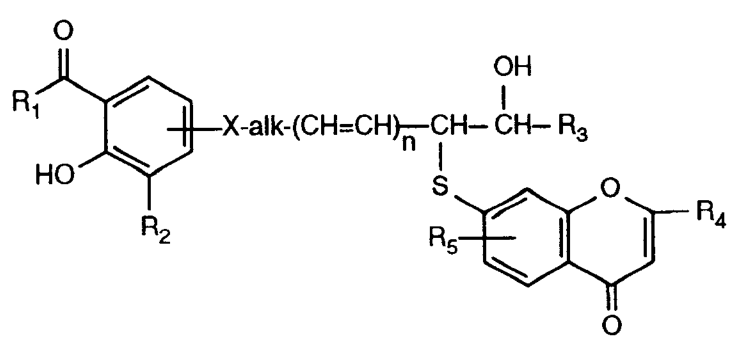

(I)

worin $R_1$ Niederalkyl oder Mono-, Di- oder Polyfluorniederalkyl bedeutet, $R_2$ Wasserstoff, Niederalkyl, Niederalkenyl oder Mono-, Di- oder Polyfluorniederalkyl darstellt, X $C_1$-$C_3$-Alkylen, Oxy oder Thio darstellt, alk Niederalkylen bedeutet, n für 1 oder 2 steht, $R_3$ für unsubstituiertes oder durch Niederalkyl, Niederalkoxy, Halogen, Carboxy, Niederalkoxycarbonyl, Amino, N-Mono- oder N,N-Diniederalkylamino, Carbamoyl, N-Mono- oder N,N-Diniederalkylcarbamoyl und/oder Trifluormethyl substituiertes Phenyl; oder für Niederalkyl, Mono-, Di- oder Trifluorniederalkyl, Carboxyniederalkyl, Niederalkoxycarbonyl-niederalkyl, Carbamoyl-niederalkyl oder N-Mono- oder N,N-Diniederalkylcarbamoyl-niederalkyl steht, $R_4$ Carboxy, Niederalkoxycarbonyl, 5-Tetrazolyl, Carbamoyl, N-Mono- oder N,N-Diniederalkylcarbamoyl oder unsubstituiertes oder im Phenylteil durch Niederalkyl, Niederalkoxy, Halogen und/oder Trifluormethyl substituiertes N-(Benzolsulphonyl)carbarmoyl bedeutet, und $R_5$ für Wasserstoff oder Niederalkyl steht; wobei mit "nieder" bezeichnete Reste nicht mehr als 7 Kohlenstoffatome aufweisen; und ihre Salze.

**2.** Verbindungen gemäss Anspruch 1 der Formel I, Worin $R_1$ $C_1$-$C_4$-Alkyl oder $\omega,\omega,\omega$-Trifluor-$C_1$-$C_4$-alkyl bedeutet, $R_2$ Wasserstoff, $C_1$-$C_4$-Alkyl, $C_2$-$C_4$-Alkenyl oder $\omega,\omega,\omega$-Trifluor-$C_1$-$C_4$-alkyl darstellt, X $C_1$-$C_3$-Alkylen, Oxy oder Thio darstellt, alk geradkettiges $C_2$-$C_6$-Alkylen darstellt, n für 1 oder 2 steht, $R_3$ für unsubstituiertes oder durch $C_1$-$C_4$-Alkyl, $C_1$-$C_4$-Alkoxy, Halogen der Atomnummer bis und mit 35, Trifluormethyl, Carboxy und/oder $C_1$-$C_4$-Alkoxycarbonyl substituiertes Phenyl; oder für $C_1$-$C_7$-Alkyl, $\omega,\omega,\omega$-Trifluor-$C_2$-$C_5$-alkyl, Carboxy-$C_2$-$C_5$-alkyl oder $C_1$-$C_4$-Alkoxycarbonyl-$C_2$-$C_5$-alkyl steht, $R_4$ Carboxy oder N-(Benzolsulphonyl)carbamoyl bedeutet, und $R_5$ für Wasserstoff steht, und ihre Salze.

**3.** Verbindungen gemäss einem der Ansprüche 1 bis 2 der Formel I, worin $R_1$ $C_1$-$C_4$-Alkyl oder $\omega,\omega,\omega$-Trifluor-$C_1$-$C_4$-alkyl bedeutet, $R_2$ $C_1$-$C_4$-Alkyl, $C_2$-$C_4$-Alkenyl oder $\omega,\omega,\omega$-Trifluor-$C_1$-$C_4$-alkyl bedeutet, und die Gruppe X in <u>para</u>-Stellung zur $R_1$-C(=O)-Gruppe gebunden ist, und ihre Salze.

**4.** Verbindungen der Formel Ia,

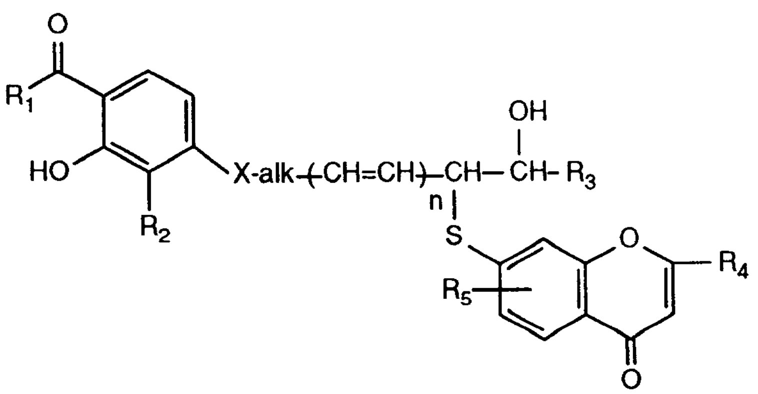

(Ia)

worin $R_1$ $C_1$-$C_4$-Alkyl bedeutet, $R_2$ $C_1$-$C_4$-Alkyl, $C_2$-$C_4$-Alkenyl oder $\omega,\omega,\omega$-Trifluor-$C_1$-$C_4$-alkyl darstellt, X $C_1$-$C_3$-Alkylen, Oxy oder Thio darstellt, alk geradkettiges $C_2$-$C_6$-Alkylen darstellt, n für 1 oder 2 steht, $R_3$ eine Gruppe der Formel - A-$R_3$' darstellt, in der -A- für $C_1$-$C_4$-Alkylen, Phenylen oder eine direkte Bindung steht und $R_3$' $C_1$-$C_4$-Alkyl, Trifluormethyl, Carboxy oder $C_1$-$C_4$-Alkoxycarbonyl bedeutet, $R_4$ Carboxy oder N-(Benzolsulphonyl)carbamoyl bedeutet, und $R_5$ für Wasserstoff steht, und ihre Salze.

**5.** Verbindungen gemäss Anspruch 4 der Formel Ia, worin $R_1$ $C_1$-$C_4$-Alkyl bedeutet, $R_2$ $C_1$-$C_4$-Alkyl darstellt, X für Oxy steht, alk $C_2$-$C_6$-Alkylen darstellt, n für 1 oder 2 steht, $R_3$ für durch $C_1$-$C_4$-Alkyl, $C_1$-$C_4$-Alkoxy, Halogen der Atomnummer bis und mit 35, Trifluormethyl oder $C_1$-$C_4$-Alkoxycarbonyl substituiertes Phenyl; oder für $C_2$-$C_8$-Alkyl, $\omega,\omega,\omega$-Trifluor-$C_3$-$C_5$-alkyl oder $C_1$-$C_4$-Alkoxycarbonyl-$C_1$-$C_4$-alkyl steht, $R_4$ Carboxy bedeutet, und $R_5$ Wasserstoff ist, und ihre Salze.

**6.** Verbindungen gemäss Anspruch 4 der Formel Ia, worin $R_1$ $C_1$-$C_4$-Alkyl bedeutet, $R_2$$C_1$-$C_4$-Alkyl darstellt, X für Oxy steht, alk $C_2$-$C_6$-Alkylen darstellt, n für 2 steht, $R_3$ eine Gruppe der Formel -A-$R_3$' darstellt, in der -A- für $C_1$-$C_4$-Alkylen oder Phenylen steht und $R_3$' $C_1$-$C_4$-Alkyl, Trifluormethyl oder $C_1$-$C_4$-Alkoxycarbonyl bedeutet, $R_4$ Carboxy oder N-(Benzolsulphonyl)carbamoyl bedeutet, und $R_5$ Wasserstoff ist, und ihre Salze.

**7.** Verbindungen gemäss einem der Ansprüche 1 bis 4 der Formel I bzw. Ia, worin X für Oxy steht, und ihre Salze.

**8.** Verbindungen gemäss einem der Ansprüche 1 bis 5 der Formel I bzw. Ia, worin n für 2 steht, und ihre Salze.

**9.** Verbindungen gemäss einem der Ansprüche 1 bis 8 der Formel I bzw. Ia, worin $R_3$ m-$C_1$-$C_4$-Alkylphenyl oder m-Trifluormethylphenyl, $R_4$ Carboxy und $R_5$ Wasserstoff bedeuten, und ihre Salze.

**10.** Eine Verbindung gemäss einem der Ansprüche 1 bis 9 der Formel I bzw. Ia, worin die dem Rest alk benachbarte Doppelbindung des Restes $-(CH=CH)_n-$ in (Z)-, d.h. cis-Konfiguration, und die gegebenenfalls vorhandene zusätzliche Doppelbindung des Restes $-(CH=CH)_n-$ in (E)-, d.h. trans-Konfiguration, vorliegt, und ihre Salze.

**11.** Eine Verbindung gemäss einem der Ansprüche 1 bis 10 der Formel I bzw. Ia, worin das mit dem Schwefelatom verbundene Ketten-C-Atom (S)-Konfiguration und das die Hydroxygruppe tragende Ketten-C-Atom (R)-Konfiguration besitzt, und ihre Salze.

**12.** (1R,2S)-1-Hydroxy-1-(3-trifluormethylphenyl)-8-(4-acetyl-3-hydroxy-2-propyl-phenoxy)-octa-3(E),5(Z)-dien-2-yl-7-thio-4-oxo-4H-1-benzopyran-2-carbonsäure oder das Natriumsalz davon.

**13.** (1R,2S)-1-Hydroxy-1-(3-methylphenyl)-8-(4-acetyl-3-hydroxy-2-propylphenoxy)-octa-3(E),5(Z)-dien-2-yl-7-thio-4-oxo-4H-1-benzopyran-2-carbonsäure oder das Natriumsalz davon.

**14.** (1R,2S)-1-Hydroxy-1-phenyl-10-(4-acetyl-3-hydroxy-2-propylphenoxy)-deca-3(E),5(Z)-dien-2-yl-7-thio-4-oxo-4H-1-benzopyran-2-carbonsäure oder das Natriumsalz davon.

**15.** (4R,5S)-1,1,1-Trifluor-4-hydroxy-11-(4-acetyl-3-hydroxy-2-propylphenoxy)-undeca-6(E),8(Z)-dien-5-yl-7-thio-4-oxo-4H-1-benzopyran-2-carbonsäure oder das Natriumsalz davon.

**16.** (1R,2S)-1-Hydroxy- 1-(3-fluorphenyl)-10-(4-acetyl-3-hydroxy-2-propylphenoxy)-deca-3(E),5(Z)-dien-2-yl-7-thio-4-oxo-4H-1-benzopyran-2-carbonsäure oder das Natriumsalz davon.

**17.** (1R,2S)-1-Hydroxy-1-(3-bromophenyl)-10-(4-acetyl-3-hydroxy-2-propylphenoxy)-deca-3(E),5(Z)-dien-2-yl-thio-4-oxo-4H-1-benzopyran-2-carbonsäure oder das Natriumsalz davon.

**18.** (1R,2S)-1-Hydroxy-1-(3-trifluormethylphenyl)-10-(4-acetyl-3-hydroxy-2-propyl-phenoxy)-deca-3(E),5(Z)-dien-2-yl-7-thio-4-oxo-4H-1-benzopyran-2-carbonsäure oder das Natriumsalz davon.

**19.** (1R,2S)-1-Hydroxy-1-(3-methylphenyl)-10-(4-acetyl-3-hydroxy-2-propylphenoxy)-deca-3(E),5(Z)-dien-2-yl-7-thio-4-oxo-4H-1-benzopyran-2-carbonsäure oder das Natriumsalz davon.

**20.** (1R,2S)-1-Hydroxy-1-(3-trifluormethylphenyl)-9-(4-acetyl-3-hydroxy-2-propyl-phenoxy)-nona-3(E),5(Z)-dien-2-yl-7-thio-4-oxo-4H-1-benzopyran-2-carbonsäure oder das Natriumsalz davon.

**21.** (1R,2S)-1-Hydroxy-1-(3-trifluormethylphenyl)-11-(4-acetyl-3-hydroxy-2-propyl-phenoxy)-undeca-3(E),5-(Z)-dien-2-yl-7-thio-4-oxo-4H-1-benzopyran-2-carbonsäure oder das Natriumsalz davon.

**22.** (1R,2S)-1-Hydroxy-1-(3-chlorphenyl)-10-(4-acetyl-3-hydroxy-2-propylphenoxy)-deca-3(E),5(Z)-dien-2-yl-7-thio-4-oxo-4H-1-benzopyran-2-carbonsäure oder das Natriumsalz davon.

**23.** (1R,2S)-1-Hydroxy-1-(3-methoxyphenyl)-10-(4-acetyl-3-hydroxy-2-propyl-phenoxy)-deca-3(E),5(Z)-dien-2-yl-7-thio-4-oxo-4H-1-benzopyran-2-carbonsäure oder das Natriumsalz davon.

**24.** (1R,2S)-1-Hydroxy-1-(3-methoxycarbonylphenyl)-10-(4-acetyl-3-hydroxy-2-propyl-phenoxy)-deca-3(E),5-(Z)-dien-2-yl-7-thio-4-oxo-4H-1-benzopyran-2-carbonsäure oder das Natriumsalz davon.

**25.** Eine Verbindung gemäss einem der Ansprüche 1 bis 24 zur Anwendung in einem Verfahren zur therapeutischen Behandlung des menschlichen oder tierischen Körpers.

**26.** Pharmazeutische Präparate enthaltend eine Verbindung gemäss einem der Ansprüche 1 bis 24 und mindestens ein pharmazeutisch verwendbares Trägermaterial.

**27.** Pharmazeutische Präparate enthaltend eine Verbindung gemäss Anspruch 14 und mindestens ein pharmazeutisch verwendbares Trägermaterial.

**28.** Pharmazeutische Präparate enthaltend eine Verbindung gemäss Anspruch 18 und mindestens ein pharmazeutisch verwendbares Trägermaterial.

**29.** Verfahren zur Herstellung von Verbindungen der Formel I gemäss Anspruch 1, oder Salzen davon, dadurch gekennzeichnet, dass man ein Epoxid der Formel II,

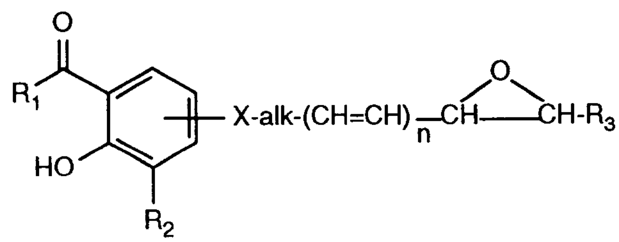

(II)

worin $R_1$, $R_2$, X, alk, n und $R_3$ wie unter Formel I definiert sind, mit einem Thiol der Formel III,

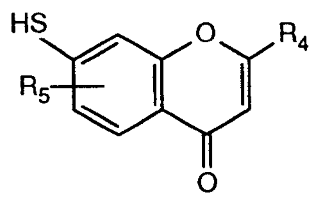

(III)

worin $R_4$ und $R_5$ wie unter Formel I definiert sind, oder einem Salz davon, umsetzt; und gewünschtenfalls eine verfahrensgemäss erhältliche Verbindung in eine andere Verbindung der Formel I umwandelt, ein verfahrensgemäss erhältliches Stereoisomeren-gemisch in die Komponenten auftrennt, und/oder eine verfahrensgemäss erhältliche freie Verbindung in ein Salz oder ein verfahrensgemäss erhältliches Salz in die freie Verbindung oder in ein anderes Salz überführt.

**30.** Verwendung einer Verbindung gemäss einem der Ansprüche 1-24 zur Herstellung pharmazeutischer Präparate.

**31.** Verwendung einer Verbindung gemäss einem der Ansprüche 1-24 zur Herstellung eines antiallergischen Arzneimittels.

**Patentansprüche für folgende Vertragsstaaten : ES, GR**

1. Verfahren zur Herstellung von Verbindungen der Formel I,

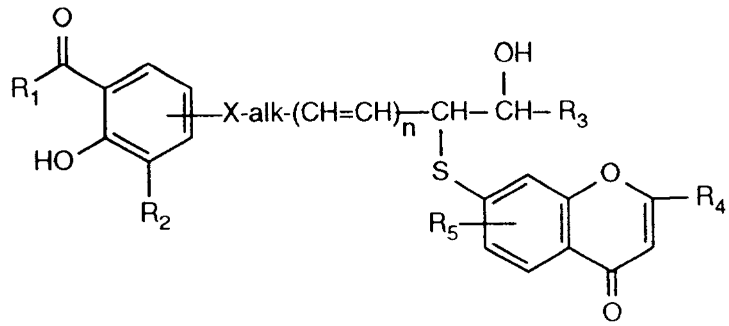

(I)

worin $R_1$ Niederalkyl oder Mono-, Di- oder Polyfluorniederalkyl bedeutet, $R_2$ Wasserstoff, Niederalkyl, Niederalkenyl oder Mono-, Di- oder Polyfluorniederalkyl darstellt, X $C_1$-$C_3$-Alkylen, Oxy oder Thio darstellt, alk Niederalkylen bedeutet, n für 1 oder 2 steht, $R_3$ für unsubstituiertes oder durch Niederalkyl, Niederalkoxy, Halogen, Carboxy, Niederalkoxycarbonyl, Amino N-Mono- oder N,N-Diniederalkylamino, Carbamoyl, N-Mono- oder N,N-Diniederalkylcarbomoyl und/oder Trifluormethyl substituiertes Phenyl; oder für Niederalkyl, Mono-, Di- oder Trifluorniederalkyl, Carboxyniederalkyl, Niederalkoxycarbonylniederalkyl, Carbamoyl-niederalkyl oder N-Mono- oder N,N-Diniederalkylcarbomoyl-niederalkyl steht, $R_4$ Carboxy, Niederalkoxycarbonyl, 5-Tetrazolyl, Carbamoyl, N-Mono- oder N,N-Diniederalkylcarbamoyl oder unsubstituiertes oder im Phenylteil durch Niederalkyl, Niederalkoxy, Halogen und/oder Trifluormethyl substituiertes N-(Benzolsulphonyl)carbamoyl bedeutet, und $R_5$ für Wasserstoff oder Niederalkyl steht; wobei mit "nieder" bezeichnete Reste nicht mehr als 7 Kohlenstoffatome aufweisen; und ihren Salzen,
dadurch gekennzeichnet, dass man ein Epoxid der Formel II,

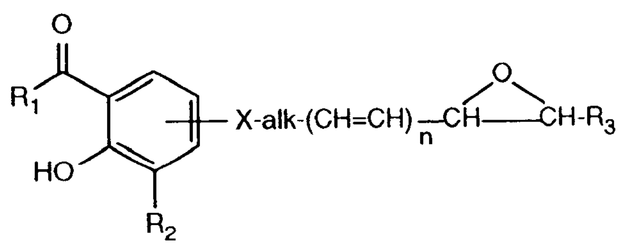

(II)

worin $R_1$,$R_2$,X, alk, n und $R_3$ wie unter Formel I definiert sind, mit einem Thiol der Formel III,

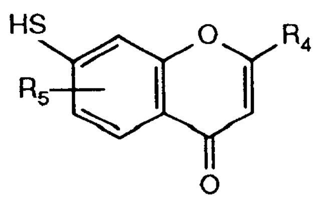

(III)

worin $R_4$ und $R_5$ wie unter Formel I definiert sind, oder einem Salz davon, umsetzt; und gewünschtenfalls eine verfahrensgemäss erhältliche Verbindung in eine andere Verbindung der Formel I umwandelt, ein verfahrensgemäss erhältliches Stereoisomeren-gemisch in die Komponenten auftrennt, und/oder eine verfahrensgemäss erhältliche freie Verbindung in ein Salz oder ein verfahrensgemäss erhältliches Salz in die freie Verbindung oder in ein anderes Salz überführt.

**2.** Verfahren gemäss Anspruch 1 zur Herstellung von Verbindungen der Formel I, worin $R_1$ $C_1$-$C_4$-Alkyl oder ω,ω,ω-Trifluor-$C_1$-$C_4$-alkyl bedeutet, $R_2$ Wasserstoff, $C_1$-$C_4$-Alkyl, $C_2$-$C_4$-Alkenyl oder ω,ω,ω-Trifluor-$C_1$-$C_4$-alkyl darstellt, X $C_1$-$C_3$-Alkylen, Oxy oder Thio darstellt, alk geradkettiges $C_2$-$C_6$-Alkylen darstellt, n für 1 oder 2 steht, $R_3$ für unsubstituiertes oder durch $C_1$-$C_4$-Alkyl, $C_1$-$C_4$-Alkoxy. Halogen der Atomnummer bis und mit 35. Trifluormethyl, Carboxy und/oder $C_1$-$C_4$-Alkoxycarbonyl substituiertes Phenyl: oder für $C_1$-$C_7$-Alkyl, ω,ω,ω-Trifluor-$C_2$-$C_5$-alkyl, Carboxy-$C_2$-$C_5$-alkyl oder $C_1$-$C_4$-Alkoxycarbonyl-$C_2$-$C_5$-alkyl steht, $R_4$ Carboxy oder N-(Benzolsulphonyl)carbamoyl bedeutet, und $R_5$ für Wasserstoff steht, und ihren Salzen.

**3.** Verfahren gemäss einem der Ansprüche 1 bis 2 zur Herstellung von Verbindungen der Formel I worin $R_1$ $C_1$-$C_4$-Alkyl oder ω,ω,ω-Trifluor-$C_1$-$C_4$-alkyl bedeutet, $R_2$ $C_1$-$C_4$-Alkyl, $C_2$-$C_4$-Alkenyl oder ω,ω,ω-Trifluor-$C_1$-$C_4$-alkyl bedeutet, und die Gruppe X in <u>para</u>-Stellung zur $R_1$-C(=O)-Gruppe gebunden ist, und ihren Salzen.

**4.** Verfahren analog Anspruch 1 zur Herstellung von Verbindungen der Formel Ia,

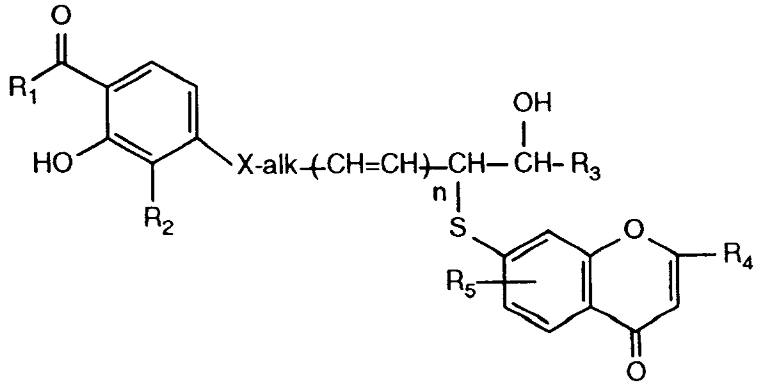

(Ia)

worin $R_1$ $C_1$-$C_4$-Alkyl bedeutet, $R_2$ $C_1$-$C_4$-Alkyl, $C_2$-$C_4$-Alkenyl oder ω,ω,ω-Trifluor-$C_1$-$C_4$-alkyl darstellt, X $C_1$-$C_3$-Alkylen, Oxy oder Thio darstellt, alk geradkettiges $C_2$-$C_6$-Alkylen darstellt, n für 1 oder 2 steht, $R_3$ eine Gruppe der Formel - A-$R_3$' darstellt, in der -A- für $C_1$-$C_4$-Alkylen, Phenylen oder eine direkte Bindung steht und $R_1$' $C_1$-$C_4$-Alkyl, Trifluormethyl, Carboxy oder $C_1$-$C_4$-Alkoxycarbonyl bedeutet, $R_4$ Carboxy oder N-(Benzolsulphonyl)carbamoyl bedeutet, und $R_5$ für Wasserstoff steht, und ihren Salzen.

**5.** Verfahren gemäss Anspruch 4 zur Herstellung von Verbindungen der Formel Ia, worin $R_1$ $C_1$-$C_4$-Alkyl bedeutet, $R_2$ $C_1$-$C_4$-Alkyl darstellt X für Oxy steht, alk $C_2$-$C_6$-Alkylen darstellt, n für 1 oder 2 steht, $R_3$ für durch $C_1$-$C_4$-Alkyl, $C_1$-$C_4$-Alkoxy, Halogen der Atomnummer bis und mit 35, Trifluormethyl oder $C_1$-$C_4$-Alkoxycarbonyl substituiertes Phenyl; oder für $C_2$-$C_8$-Alkyl, ω,ω,ω-Trifluor-$C_3$-$C_5$-alkyl oder $C_1$-$C_4$-Alkoxycarbonyl-$C_1$-$C_4$-alkyl steht, $R_4$ Carboxy bedeutet, und $R_5$ Wasserstoff ist, und ihren Salzen.

**6.** Verfahren gemäss Anspruch 4 zur Herstellung von Verbindungen der Formel Ia, worin $R_1$ $C_1$-$C_4$-Alkyl bedeutet, $R_2 C_1$-$C_4$-Alkyl darstellt, X für Oxy steht, alk $C_2$-$C_6$-Alkylen darstellt, n für 2 steht, $R_3$ eine Gruppe der Formel -A-$R_3$' darstellt, in der -A- für $C_1$-$C_4$-Alkylen oder Phenylen steht und $R_3$' $C_1$-$C_4$-Alkyl, Trifluormethyl oder $C_1$-$C_4$-Alkoxycarbonyl bedeutet, $R_4$ Carboxy oder N-(Benzolsulphonyl)-carbamoyl bedeutet, und $R_5$ Wasserstoff ist, und ihren Salzen.

**7.** Verfahren gemäss einem der Ansprüche 1 bis 4 zur Herstellung von Verbindungen der Formel I bzw. Ia, worin X für Oxy steht, und ihren Salzen.

**8.** Verfahren gemäss einem der Ansprüche 1 bis 5 zur Herstellung von Verbindungen der Formel I bzw. Ia, worin n für 2 steht, und ihren Salzen.

**9.** Verfahren gemäss einem der Ansprüche 1 bis 8 zur Herstellung von Verbindungen der Formel I bzw. Ia, worin $R_3$ m-$C_1$-$C_4$-Alkylphenyl oder m-Trifluormethylphenyl, $R_4$ Carboxy und $R_5$ Wasserstoff bedeuten, und ihren Salzen.

10. Verfahren gemäss einem der Ansprüche 1 bis 9 zur Herstellung von Verbindungen der Formel I bzw. Ia, worin die dem Rest alk benachbarte Doppelbindung des Restes $-(CH=CH)_n-$ in (Z)-, d.h. cis-Konfiguration, und die gegebenenfalls vorhandene zusätzliche Doppelbindung des Restes $-(CH=CH)_n-$ in (E)-, d.h. trans-Konfiguration, vorliegt, und ihren Salzen.

11. Verfahren gemäss einem der Ansprüche 1 bis 10 zur Herstellung von Verbindungen der Formel I bzw. Ia, worin das mit dem Schwefelatom verbundene Ketten-C-Atom (S)-Konfiguration und das die Hydroxygruppe tragende Ketten-C-Atom (R)-Konfiguration besitzt, und ihren Salzen.

12. Verfahren gemäss Anspruch 1 zur Herstellung von (1R,2S)-1-Hydroxy-1-(3-trifluormethylphenyl) -8-(4-acetyl-3-hydroxy-2-propyl-phenoxy)-octa-3(E),5(Z)-dien-2-yl-7-thio-4-oxo-4H-1-benzopyran-2-carbonsäure oder dem Natriumsalz davon.

13. Verfahren gemäss Anspruch 1 zur Herstellung von (1R,2S)-1-Hydroxy-1-(3-methylphenyl) -8-(4-acetyl-1-hydroxy-2-propylphenoxy)-octa-3(E),5(Z)-dien-2-yl-7-thio-4-oxo-4H-1-benzopyran-2-carbonsäure oder dem Natriumsalz davon.

14. Verfahren gemäss Anspruch 1 zur Herstellung von (1R,2S)-1-Hydroxy-1-phenyl-10-(4-acetyl-3-hydrox-2-propylphenoxy)-deca-3(E),5(Z)-dien-2-yl-7-thio-4-oxo-4H-1-benzopyran-2-carbonsäure oder dem Natriumsalz davon.

15. Verfahren gemäss Anspruch 1 zur Herstellung von (4R,5S)-1,1,1-Trifluor-4-hydroxy-11-4-acetyl -3-hydroxy-2-propylphenoxy)-undeca-6(E),8(Z)-dien-5-yl-7-thio-4-oxo-4H-1-benzopyran-2-carbonsäure oder dem Natriumsalz davon.

16. Verfahren gemäss Anspruch 1 zur Herstellung von (1R,2S)-1-Hydroxy-1-(3-fluorphenyl)-10-(4-acetyl-3-hydroxy-2-propylphenoxy)-deca-3(E),5(Z)-dien-2-yl-7-thio-4-oxo-4H-1-benzopyran-2-carbonsäure oder dem Natriumsalz davon.

17. Verfahren gemäss Anspruch 1 zur Herstellung von (1R,2S)-1-Hydroxy-1-(3-bromophenyl) -10-(4-acetyl-3-hydroxy-2-propylphenoxy)-deca-3(E),5(Z)-dien-2-yl-7-thio-4-oxo-4H-1-benzopyran-2-carbonsäure oder dem Natriumsalz davon.

18. Verfahren gemäss Anspruch 1 zur Herstellung von (1R,2S)-1-Hydroxy-1-(3-trifluormethylphenyl)-10-(4-acetyl-3-hydroxy-2-propylphenoxy)-deca-3(E),5(Z)-dien-2-yl-7-thio-4-oxo-4H-1-benzopyran-2-carbonsäure oder dem Natriumsalz davon.

19. Verfahren gemäss Anspruch 1 zur Herstellung von (1R,2S)-1-Hydroxy-1-(3-methylphenyl)-10-(4-acetyl-3-hydroxy-2-propylphenoxy)-deca-3(E),5(Z)-dien-2-yl-7-thio-4-oxo-4H-1-benzopyran-2-carbonsäure oder dem Natriumsalz davon.

20. Verfahren gemäss Anspruch 1 zur Herstellung von (1R,2S)-1-Hydroxy-1-(3-trifluormethylphenyl)-9-(4-acetyl-3-hydroxy-2-propyl-phenoxy)-nona-3(E),5(Z)-dien-2-yl-7-thio-4-oxo-4H-1-benzopyran-2-carbonsäure oder dem Natriumsalz davon.

21. Verfahren gemäss Anspruch 1 zur Herstellung von (1R,2S)-1-Hydroxy-1-(3-trifluormethylphenyl)-11-(4-acetyl-3-hydroxy-2-propyl-phenoxy)-undeca-3(E),5(Z)-dien-2-yl-7-thio-4-oxo-4H-1-benzopyran-2-carbonsäure oder dem Natriumsalz davon.

22. Verfahren gemäss Anspruch 1 zur Herstellung von (1R,2S)-1-Hydroxy-1-(3-chlorphenyl)-10-(4-acetyl-3-hydroxy-2-propylphenoxy)-deca-3(E),5(Z)-dien-2-yl-7-thio-4-oxo-4H-1-benzopyran-2-carbonsäure oder dem Natriumsalz davon.

23. Verfahren gemäss Anspruch 1 zur Herstellung von (1R,2S)-1-Hydroxy-1-(3-methoxyphenyl)-10-(4-acetyl-3-hydroxy-2-propyl-phenoxy)-deca-3(E),5(Z)-dien-2-yl-7-thio-4-oxo-4H-1-benzopyran-2-carbonsäure oder dem Natriumsalz davon.

**24.** Verfahren gemäss Anspruch 1 zur Herstellung von (1R,2S)-1-Hydroxy-1-(3-methoxycarbonylphenyl) -10-(4-acetyl-3-hydroxy-2-propyl-phenoxy)-deca-3(E),5(Z)-dien-2-yl-7-thio-4-oxo-4H-1-benzopyran-2-carbonsäure oder dem Natriumsalz davon.

**25.** Verfahren zur Herstellung pharmazeutischer Präparate, dadurch gekennzeichnet, dass man eine Verbindung, erhältlich gemäss einem der Ansprüche 1 bis 24, mit üblichen pharmazeutischen Hilfsstoffen vermischt.

**Claims**

**Claims for the following Contracting States : AT, BE, CH, DE, FR, GB, IT, LI, LU, NL, SE**

**1.** A compound of formula I

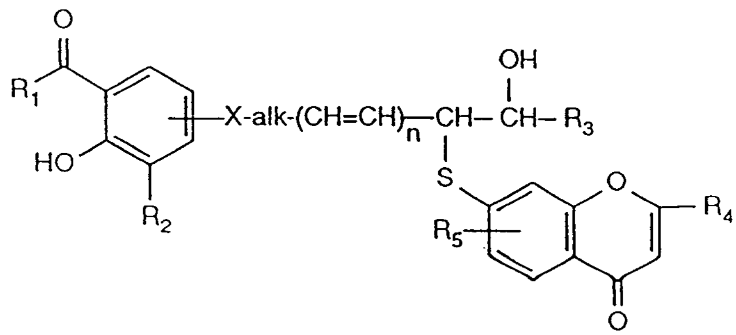

(I),

in which $R_1$ is lower alkyl or mono-, di- or poly-fluoro-lower alkyl, $R_2$ is hydrogen, lower alkyl, lower alkenyl or mono-, di- or poly-fluoro-lower alkyl, x is $C_1$-$C_3$-alkylene, oxy or thio, <u>alk</u> is lower alkylene, <u>n</u> is 1 or 2, $R_3$ is phenyl that is unsubstituted or is substituted by lower alkyl, lower alkoxy, halogen, carboxy, lower alkoxycarbonyl, amino, N-mono- or N,N-di-lower alkylamino, carbamoyl, N-mono- or N,N-di-lower alkylcarbamoyl and/or by trifluoromethyl, or is lower alkyl, mono-, di-or tri-fluoro-lower alkyl, carboxy-lower alkyl, lower alkoxycarbonyl-lower alkyl, carbamoyl-lower alkyl or N-mono- or N,N-di-lower alkylcarbamoyl-lower alkyl, $R_4$ is carboxy, lower alkoxycarbonyl, 5-tetrazolyl, carbamoyl, N-mono- or N,N-di-lower alkylcarbamoyl, or N-(benzene-sulfonyl)-carbamoylthat is unsubstituted or is substituted in the phenyl moiety by lower alkyl, lower alkoxy, halogen and/or by trifluoromethyl, and $R_5$ is hydrogen or lower alkyl; radicals designated "lower" having not more than 7 carbon atoms; or a salt thereof.

**2.** A compound according to claim 1, of formula I, in which $R_1$ is $C_1$-$C_4$alkyl or ω,ω,ω-trifluoro-$C_1$-$C_4$alkyl, $R_2$ is hydrogen, $C_1$-$C_4$alkyl, $C_2$-$C_4$alkenyl or ω,ω,ω-trifluoro-$C_1$-$C_4$alkyl, X is $C_1$-$C_3$alkylene, oxy or thio, <u>alk</u> is straight-chain $C_2$-$C_6$alkylene, <u>n</u> is 1 or 2, $R_3$ is phenyl that is unsubstituted or is substituted by $C_1$-$C_4$alkyl, $C_1$-$C_4$alkoxy, halogen having an atomic number of up to and including 35, trifluoromethyl, carboxy and/or by $C_1$-$C_4$alkoxycarbonyl, or is $C_1$-$C_7$alkyl, ω,ω,ω-trifluoro-$C_2$-$C_5$alkyl, carboxy-$C_2$-$C_5$alkyl or $C_1$-$C_4$alkoxy-carbonyl-$C_2$-$C_5$alkyl, $R_4$ is carboxy or N-(bensenesulfonyl)-carbamoyl, and $R_5$ is hydrogen, or a salt thereof.

**3.** A compound according to either claim 1 or claim 2, of formula I, in which $R_1$ is $C_1$-$C_4$alkyl or ω,ω,ω - trifluoro-$C_1$-$C_4$alkyl, $R_2$ is $C_1$-$C_4$alkyl, $C_2$-$C_4$alkenyl or ω,ω,ω-trifluoro$C_1$-$C_4$alkyl, and the group X is bonded in the <u>para</u>-position with respect to the $R_1$-C(=O) group, or a salt thereof.

**4.** A compound of formula Ia

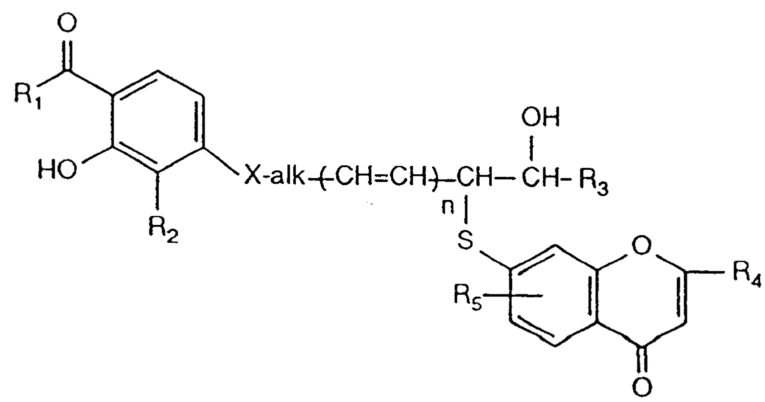

(Ia),

in which $R_1$ is $C_1$-$C_4$alkyl, $R_2$ is $C_1$-$C_4$alkyl, $C_2$-$C_4$-alkenyl or ω,ω,ω-trifluoro-$C_1$-$C_4$alkyl, X is $C_1$-$C_3$-alkylene, oxy or thio, alk is straight-chain $C_2$-$C_6$-alkylene, n is 1 or 2, $R_3$ is a group of formula -A-$R_3$' in which -A- is $C_1$-$C_4$alkylene, phenylene or a direct bond and $R_3$' is $C_1$-$C_4$alkyl, trifluoromethyl, carboxy or $C_1$-$C_4$alkoxycarbonyl, $R_4$ is carboxy or N-(bensenesulfonyl)-carbamoyl, and $R_5$ is hydrogen, or a salt thereof.

**5.** A compound according to claim 4, of formula Ia, in which $R_1$ is $C_1$-$C_4$alkyl, $R_2$ is $C_1$-$C_4$alkyl, X is oxy, alk is $C_2$-$C_6$alkylene, n is 1 or 2, $R_3$ is phenyl substituted by $C_1$-$C_4$alkyl, $C_1$-$C_4$alkoxy, halogen having an atomic number of up to and including 35, trifluoromethyl or by $C_1$-$C_4$alkoxycarbonyl, or is $C_2$-$C_8$alkyl, ω,ω,ω-trifluoro$C_3$-$C_5$alkyl or $C_1$-$C_4$alkoxycarbonyl-$C_1$-$C_4$alkyl, $R_4$ is carboxy, and $R_5$ is hydrogen, or a salt thereof.

**6.** A compound according to claim 4, of formula Ia, in which $R_1$ is $C_1$-$C_4$alkyl, $R_2$ is $C_1$-$C_4$alkyl, X is oxy, alk is $C_2$-$C_6$alkylene n is 2, $R_3$ is a group of formula -A-$R_3$' in which -A- is $C_1$-$C_4$alkylene or phenylene, and $R_3$' is $C_1$-$C_4$alkyl, trifluoromethyl or $C_1$-$C_4$alkoxy-carbonyl, $R_4$ is carboxy or N-(bensenesulfonyl)-carbamoyl, and $R_5$ is hydrogen, or a salt thereof.

**7.** A compound according to any one of claims 1 to 4, of formula I or Ia, in which X is oxy, or a salt thereof.

**8.** A compound according to any one of claims 1 to 5, of formula I or Ia, in which n is 2, or a salt thereof.

**9.** A compound according to any one of claims 1 to 8, of formula I or Ia, in which $R_3$ is m-$C_1$-$C_4$alkylphenyl or m-trifluoromethylphenyl, $R_4$ is carboxy and $R_5$ is hydrogen, or a salt thereof.

**10.** A compound according to any one of claims 1 to 9, of formula I or Ia, in which in the radical $-(CH=CH)_n-$the double bond adjacent to the radical alk is in the (Z)-, that is to say the cis-configuration, and the additional double bond which may be present in the radical $-(CH=CH)_n-$ is in the (E)-, that is to say the trans-configuration, or a salt thereof.

**11.** A compound according to any one of claims 1 to 10, of formula I or Ia, in which the chain carbon atom bonded to the sulfur atom has the (S)-configuration and the chain carbon atom carrying the hydroxy group has the (R)-configuration, or a salt thereof.

**12.** (1R,2S)-1-hydroxy-1-(3-trifluoromethylphenyl)-8-(4-acetyl-3-hydroxy-2-propylphenoxy)-octa-3(E),5(Z)-dien-2-yl-7-thio-4-oxo-4H-1-benzopyrane-2-carboxylic acid or the sodium salt thereof.

**13.** (1R,2S)-1-hydroxy-1-(3-methylphenyl)-8-(4-acetyl-3-hydroxy-2-propylphenoxy)-octa-3(E),5(Z)-dien-2-yl-7-thio-4-oxo-4H-1-benzopyrane-2-carboxylic acid or the sodium salt thereof.

**14.** (1R,2S)-1-hydroxy-1-phenyl-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**15.** (4R,5S)-1,1,1-trifluoro-4-hydroxy-11-(4-acetyl-3-hydroxy-2-propylphenoxy)-undeca-6(E),8(Z)-dien-5-yl-7-thio-4-oxo-4H-1-benzopyrane-2-carboxylic acid or the sodium salt thereof.

**16.** (1R,2S)-1-hydroxy-1-(3-fluorophenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**17.** (1R,2S)-1-hydroxy-1-(3-bromophenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**18.** (1R,2S)-1-hydroxy-1-(3-trifluoromethylphenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**19.** (1R,2S)-1-hydroxy-1-(3-methylphenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**20.** (1R,2S)-1-hydroxy-1-(3-trifluoromethylphenyl)-9-(4-acetyl-3-hydroxy-2-propylphenoxy)-nona-3(E),5(Z)-dien-2-yl-7-thio-4-oxo-4H-1-benzopyrane-2-carboxylic acid or the sodium salt thereof.

**21.** (1R,2S)-1-hydroxy-1-(3-trifluoromethylphenyl)-11-(4-acetyl-3-hydroxy-2-propylphenoxy)-undeca-3(E),5-(Z)-dien-2-yl-7-thio-4-oxo-4H-1-benzopyrane-2-carboxylic acid or the sodium salt thereof.

**22.** (1R,2S)-1-hydroxy-1-(3-chlorophenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**23.** (1R,2S)-1-hydroxy-1-(3-methoxyphenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**24.** (1R,2S)-1-hydroxy-1-(3-methoxycarbonylphenyl)-10-(4-acetyl-3-hydroxy-2-propylphenoxy)-deca-3(E),5-(Z)-dien-2-yl-7-thio-4-oxo-4H-1-benzopyrane-2-carboxylic acid or the sodium salt thereof.

**25.** A compound according to any one of claims 1 to 24 for use in a method for the therapeutic treatment of the human or animal body.

**26.** A pharmaceutical composition comprising a compound according to any one of claims 1 to 24 and at least one pharmaceutically acceptable carrier.

**27.** A pharmaceutical composition comprising a compound according to claim 14 and at least one pharmaceutically acceptable carrier.

**28.** A pharmaceutical composition comprising a compound according to claim 18 and at least one pharmaceutically acceptable carrier.

**29.** A process for the preparation of a compound of formula I according to claim 1, or a salt thereof, which process comprises reacting an epoxide of formula II

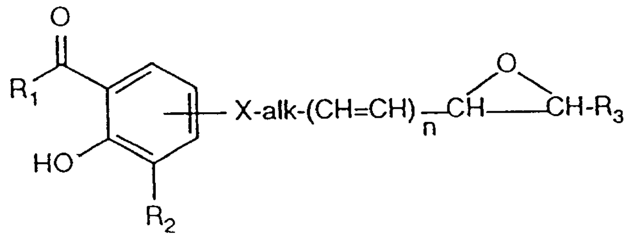

(II),

in which $R_1$, $R_2$, X, alk, n and $R_3$ are as defined for formula I, with a thiol of formula III

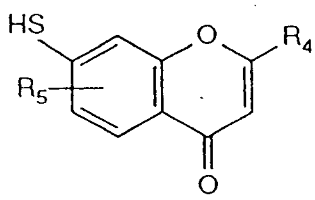

(III),

in which $R_4$ and $R_5$ are as defined for formula I, or with a salt thereof, and, if desired, converting a compound obtainable in accordance with the process into a different compound of formula I, separating a stereoisomeric mixture obtainable in accordance with the process into the components and/or converting a free compound obtainable in accordance with the process into a salt, or converting a salt obtainable in accordance with the process into the free compound or into a different salt.

**30.** The use of a compound according to any one of claims 1 to 24 for the preparation of a pharmaceutical composition.

**31.** The use of a compound according to any one of claims 1 to 24 for the preparation of an anti-allergic medicament.

**Claims for the following Contracting States : ES, GR**

**1.** A process for the preparation of a compound of formula I

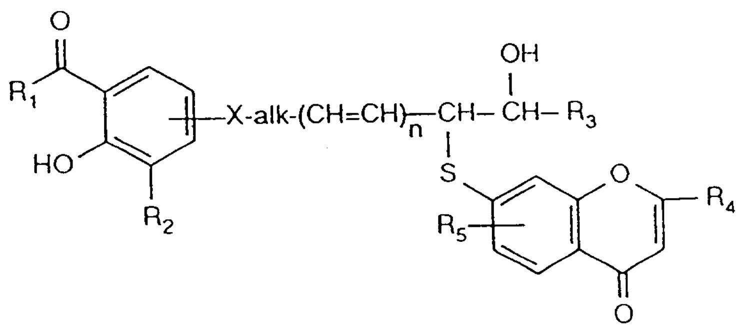

(I),

in which $R_1$ is lower alkyl or mono-, di- or poly-fluoro-lower alkyl, $R_2$ is hydrogen, lower alkyl, lower alkenyl or mono-, di- or poly-fluoro-lower alkyl, X is $C_1$-$C_3$-alkylene, oxy or thio, alk is lower alkylene, n is 1 or 2, $R_3$ is phenyl that is unsubstituted or is substituted by lower alkyl, lower alkoxy, halogen, carboxy, lower alkoxycarbonyl, amino, N-mono- or N,N-di-lower alkylamino, carbamoyl, N-mono- or N,N-di-lower alkylcarbamoyl and/or by trifluoromethyl, or is lower alkyl, mono-, di-or tri-fluoro-lower alkyl, carboxy-lower alkyl, lower alkoxycarbonyl-lower alkyl, carbamoyl-lower alkyl or N-mono- or N,N-di-lower alkylcarbamoyl-lower alkyl, $R_4$ is carboxy, lower alkoxycarbonyl, 5-tetrazolyl, carbamoyl, N-mono- or N,N-di-lower alkylcarbamoyl, or N-(bensene-sulfonyl)-carbamoylthat is unsubstituted or is substituted in the phenyl moiety by lower alkyl, lower alkoxy, halogen and/or by trifluoromethyl, and $R_5$ is hydrogen or lower alkyl; radicals designated "lower" having not more than 7 carbon atoms; or a salt thereof, which process comprises reacting an epoxide of formula II

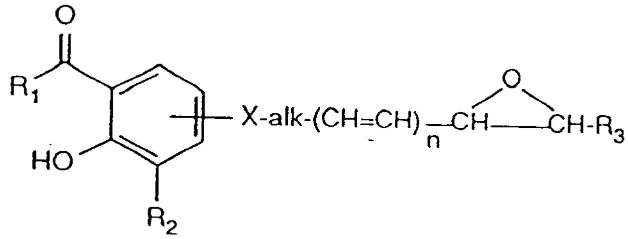

(II),

in which $R_1$, $R_2$, X, alk, n and $R_3$ are as defined for formula I, with a thiol of formula III

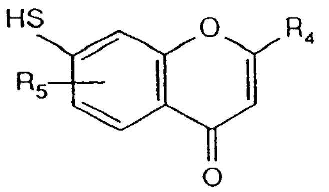

(III),

in which $R_4$ and $R_5$ are as defined for formula I, or with a salt thereof, and, if desired, converting a compound obtainable in accordance with the process into a different compound of formula I, separating a stereoisomeric mixture obtainable in accordance with the process into the components and/or converting a free compound obtainable in accordance with the process into a salt, or converting a salt obtainable in accordance with the process into the free compound or into a different salt.

**2.** A process according to claim 1 for the preparation of a compound of formula I, in which $R_1$ is $C_1$-$C_4$alkyl or ω,ω,ω-trifluoro-$C_1$-$C_4$alkyl, $R_2$ is hydrogen, $C_1$-$C_4$alkyl, $C_2$-$C_4$alkenyl or ω,ω,ω-trifluoro-$C_1$-$C_4$alkyl, X is $C_1$-$C_3$alkylene, oxy or thio, alk is straight-chain $C_2$-$C_6$-alkylene, n is 1 or 2, $R_3$ is phenyl that is unsubstituted or is substituted by $C_1$-$C_4$alkyl, $C_1$-$C_4$alkoxy, halogen having an atomic number of up to and including 35, trifluoromethyl, carboxy and/or by $C_1$-$C_4$alkoxy-carbonyl, or is $C_1$-$C_7$alkyl, ω,ω,ω-trifluoro-$C_2$-$C_5$alkyl, carboxy-$C_2$-$C_5$alkyl or $C_1$-$C_4$alkoxycarbonyl-$C_2$-$C_5$alkyl, $R_4$ is carboxy or N-(benzenesulfonyl)-carbamoyl, and $R_5$ is hydrogen, or a salt thereof.

**3.** A process according to either claim 1 or claim 2 for the preparation of a compound of formula I, in which $R_1$ is $C_1$-$C_4$alkyl or ω,ω,ω-trifluoro-$C_1$-$C_4$alkyl, $R_2$ is $C_1$-$C_4$alkyl, $C_2$-$C_4$alkenyl or ω,ω,ω-trifluoro-$C_1$-$C_4$-alkyl, and the group X is bonded in the para-position with respect to the $R_1$-C(=O) group, or a salt thereof.

**4.** A process analogous to claim 1 for the preparation of a compound of formula Ia

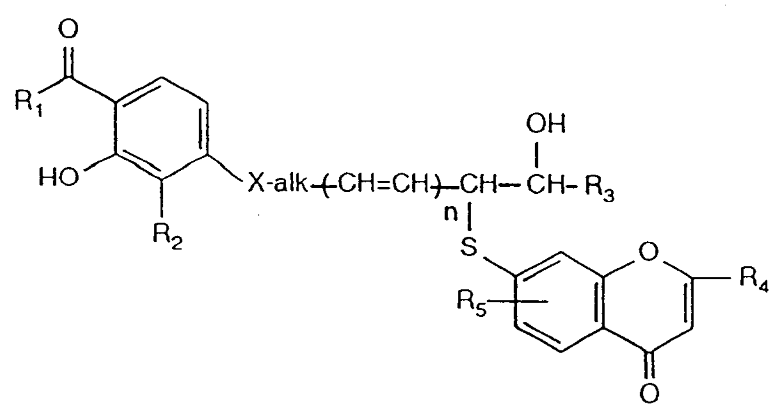

(Ia),

in which $R_1$ is $C_1$-$C_4$alkyl, $R_2$ is $C_1$-$C_4$alkyl, $C_2$-$C_4$-alkenyl or ω,ω,ω-trifluoro-$C_1$-$C_4$alkyl, X is $C_1$-$C_3$-alkylene, oxy or thio, alk is straight-chain $C_2$-$C_6$-alkylene, n is 1 or 2, $R_3$ is a group of formula -A-$R_3$' in which -A- is $C_1$-$C_4$alkylene, phenylene or a direct bond and $R_3$' is $C_1$-$C_4$alkyl, trifluoromethyl, carboxy or $C_1$-$C_4$alkoxycarbonyl, $R_4$ is carboxy or N-(benzenesulfonyl)-carbamoyl, and $R_5$ is hydrogen, or a salt thereof.

**5.** A process according to claim 4 for the preparation of a compound of formula Ia, in which $R_1$ is $C_1$-$C_4$alkyl, $R_2$ is $C_1$-$C_4$alkyl, X is oxy, alk is $C_2$-$C_6$alkylene n is 1 or 2, $R_3$ is phenyl substituted by $C_1$-$C_4$alkyl, $C_1$-$C_4$alkoxy, halogen having an atomic number of up to and including 35, trifluoromethyl or by $C_1$-$C_4$alkoxycarbonyl, or is $C_2$-$C_8$alkyl, ω,ω,ω-trifluoro-$C_3$-$C_5$alkyl or $C_1$-$C_4$alkoxy-carbonyl-$C_1$-$C_4$alkyl, $R_4$ is carboxy, and $R_5$ is hydrogen, or a salt thereof.

**6.** A process according to claim 4 for the preparation of a compound of formula Ia in which R1 is $C_1$-$C_4$alkyl, $R_2$ is $C_1$-$C_4$alkyl, X is oxy, alk is $C_2$-$C_6$alkylene n is 2, $R_3$ is a group of formula -A-$R_3$' in which -A- is $C_1$-$C_4$alkylene or phenylene, and $R_3$' is $C_1$-$C_4$alkyl, trifluoromethyl or $C_1$-$C_4$alkoxycarbonyl, $R_4$ is carboxy or N-(benzenesulfonyl)-carbamoyl, and $R_5$ is hydrogen, or a salt thereof.

**7.** A process according to any one of claims 1 to 4 for the preparation of a compound of formula I or Ia in which X is oxy, or a salt thereof.

**8.** A process according to any one of claims 1 to 5 for the preparation of a compound of formula I or Ia in which n is 2, or a salt thereof.

**9.** A process according to any one of claims 1 to 8 for the preparation of a compound of formula I or Ia in which $R_3$ is m-$C_1$-$C_4$alkylphenyl or m-trifluoromethylphenyl, $R_4$ is carboxy and $R_5$ is hydrogen, or a salt thereof.

**10.** A process according to any one of claims 1 to 9 for the preparation of a compound of formula I or Ia in which in the radical $-(CH=CH)_n-$ the double bond adjacent to the radical alk is in the (Z)-, that is to say the cis-configuration, and the additional double bond which may be present in the radical $-(CH=CH)_n-$ is in the (E)-, that is to say the trans-configuration, or a salt thereof.

**11.** A process according to any one of claims 1 to 10 for the preparation of a compound of formula I or Ia in which the chain carbon atom bonded to the sulfur atom has the (S)-configuration and the chain carbon atom carrying the hydroxy group has the (R)-configuration, or a salt thereof.

**12.** A process according to claim 1 for the preparation of (1R,2S)-1-hydroxy-1-(3-trifluoromethylphenyl)-8-(4-acetyl-3-hydroxy-2-propylphenoxy)-octa-3(E),5(Z)-dien-2-yl-7-thio-4-oxo-4H-1-benzopyrane-2-carboxylic acid or the sodium salt thereof.

**13.** A process according to claim 1 for the preparation of (1R,2S)-1-hydroxy-1-(3-methylphenyl)-8-(4-acetyl-3-hydroxy-2-propylphenoxy)-octa-3(E),5(Z)-dien-2-yl-7-thio-4-oxo-4H-1-benzopyrane-2-carboxylic acid or the sodium salt thereof.

**14.** A process according to claim 1 for the preparation of (1R,2S)-1-hydroxy-1-phenyl-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**15.** A process according to claim 1 for the preparation of (4R,5S)-1,1,1-trifluoro-4-hydroxy-11-(4-acetyl-3-hydroxy-2-propylphenoxy)-undeca-6(E),8(Z)-dien-5-yl-7-thio-4-oxo-4H-1-bensopyrane-2-carboxylic acid or the sodium salt thereof.

**16.** A process according to claim 1 for the preparation of (1R,2S)-1-hydroxy-1-(3-fluorophenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**17.** A process according to claim 1 for the preparation of (1R,2S)-1-hydroxy-1-(3-bromophenyl)-10-(4-acetyl-3-hydroxy-2-propylphenoxy)-deca-3(E),5(Z)-dien-2-yl-7-thio-4-oxo-4H-1-bensopyrane-2-carboxylic acid or the sodium salt thereof.

**18.** A process according to claim 1 for the preparation of (1E,2S)-1-hydroxy-1-(3-trifluoromethylphenyl)-10-(4-acetyl-3-hydroxy-2-propylphenoxy)-deca-3(E),5(Z)-dien-2-yl-7-thio-4-oxo-4H-1-bensopyrane-2-carboxylic acid or the sodium salt thereof.

**19.** A process according to claim 1 for the preparation of (1E,2S)-1-hydroxy-1-(3-methylphenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**20.** A process according to claim 1 for the preparation of (1R,2S)-1-hydroxy-1-(3-trifluoromethylphenyl)-9-(4-acetyl-3-hydroxy-2-propylphenoxy)-nona-3(E),5(Z)-dien-2-yl-7-thio-4-oxo-4H-1-benzopyrane-2-carboxylic acid or the sodium salt thereof.

**21.** A process according to claim 1 for the preparation of (1R,2S)-1-hydroxy-1-(3-trifluoromethylphenyl)-11-(4-acetyl-3-hydroxy-2-propylphenoxy)-undeca-3(E),5(Z)-dien-2-yl-7-thio-4-oxo-4H-1-benzopyrane-2-carboxylic acid or the sodium salt thereof.

**22.** A process according to claim 1 for the preparation of (1R,2S)-1-hydroxy-1-(3-chlorophenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**23.** A process according to claim 1 for the preparation of (1R,2S)-1-hydroxy-1-(3-methoxyphenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**24.** A process according to claim 1 for the preparation of (1R,2S)-1-hydroxy-1-(3-methoxycarbonylphenyl)-10-(4-acetyl-3-hydroxy-2-propylphenoxy)-deca-3(E),5(Z)-dien-2-yl-7-thio-4-oxo-4H-1-benzopyrane-2-carboxylic acid or the sodium salt thereof.

**25.** A process for the preparation of a pharmaceutical composition, which comprises mixing a compound obtainable in accordance with any one of claims 1 to 24 with a customary pharmaceutical excipient.

**Revendications**

**Revendications pour les Etats contractants suivants : AT, BE, CH, DE, FR, GB, IT, LI, LU, NL, SE**

**1.** Composés de formule I

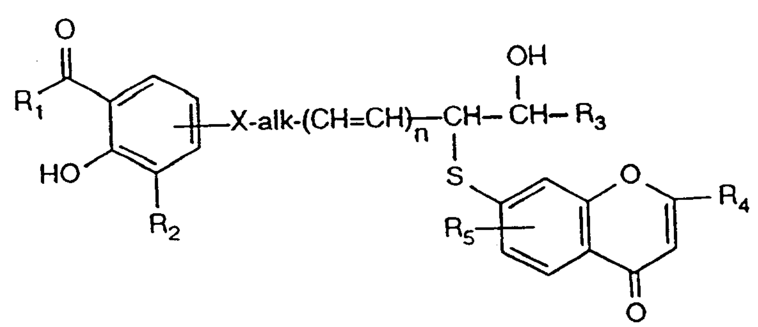

(I)

où $R_1$ représente un alkyle inférieur ou un alkyle inférieur mono-, di- ou polyfluoré, $R_2$ représente l'hydrogène, un alkyle inférieur, un alcényle inférieur ou un alkyle inférieur mono-, di- ou polyfluoré, X représente un alkylène en $C_1$-$C_3$, un oxy ou un thio, alk représente un alkylène inférieur, n est égal à 1 ou 2, $R_3$ représente un phényle non substitué ou substitué par un alkyle inférieur, un alcoxy inférieur, un halogène, un carboxy, un alcoxy inférieur carbonyle, un amino, un N-mono- ou un N,N-dialkyle inférieur amino, un carbamoyle, un N-mono-ou un N,N-dialkyle inférieur carbamoyle et/ou le trifluorométhyle; ou représente un alkyle inférieur, un alkyle inférieur mono-, di- ou trifluoré, un carboxyalkyle inférieur, un alcoxy inférieur carbonylalkyle inférieur, un carbamoylalkyle inférieur ou un N-mono- ou un N,N-dialkyle inférieur carbamoylalkyle inférieur, $R_4$ représente un carboxy, un alcoxy inférieur carbonyle, le 5-tétrazolyle, un carbamoyle, un N-mono- ou un N,N-dialkyle inférieur carbamoyle ou un N-(benzènesulfonyl)carbamoyle non substitué ou substitué dans la partie phényle par un alkyle inférieur, un alcoxy inférieur, un halogène et/ou le trifluorométhyle et $R_5$ représente l'hydrogène ou un alkyle inférieur, les restes désignés par 'inférieurs" ne comportant pas plus de 7 atomes de carbone et leurs sels.

**2.** Composés selon la revendication 1 de formule I où $R_1$ représente un alkyle en $C_1$-$C_4$ ou un $\omega,\omega,\omega$-trifluoroalkyle en $C_1$-$C_4$, $R_2$ représente l'hydrogène, un alkyle en $C_1$ -$C_4$, un alcényle en $C_2$-$C_4$ ou un $\omega,\omega,\omega$-trifluoroalkyle en $C_1$-$C_4$, X représente un alkylène en $C_1$-$C_3$, un oxy ou un thio, alk représente un alkylène en $C_2$-$C_6$ à chaîne droite, n est égal à 1 ou 2, $R_3$ représente un phényle non substitué ou substitué par un alkyle en $C_1$-$C_4$, un alcoxy en $C_1$-$C_4$, un halogène ayant un numéro atomique allant

jusqu'à 35, le trifluorométhyle, le carboxy et/ou un alcoxy en $C_1$-$C_4$ carbonyle; ou un alkyle en $C_1$-$C_7$, un ω,ω,ω-trifluoroalkyle en $C_2$-$C_5$, un carboxyalkyle en $C_2$-$C_5$ ou un alcoxy en $C_1$-$C_4$ carbonylalkyle en $C_2$-$C_5$, $R_4$ représente un carboxy ou le N-(benzènesulfonyl) carbamoyle et $R_5$ représente l'hydrogène et leurs sels.

**3.** Composés selon l'une des revendications 1 et 2 de formule I où $R_1$ est un alkyle en $C_1$-$C_4$ ou un ω,ω,ω-trifluoroalkyle en $C_1$-$C_4$, $R_2$ représente un alkyle en $C_1$-$C_4$, un alcényle en $C_2$-$C_4$ ou un ω,ω,ω-trifluoroalkyle en $C_1$-$C_4$ et dans lequel le groupe X est lié au groupe $R_1$-C(=O) en position para et leurs sels.

**4.** Composés de formule Ia

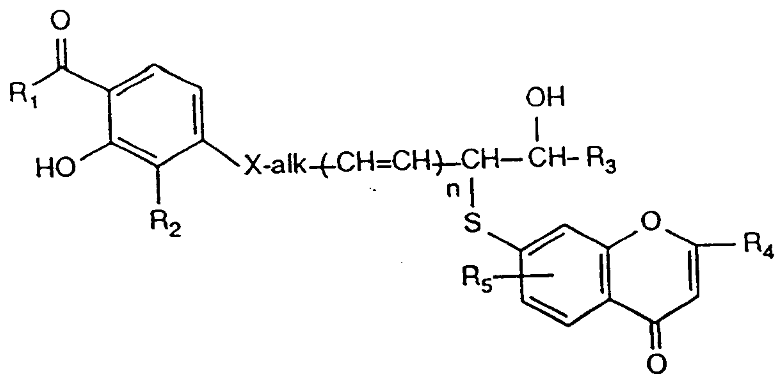

(Ia)

où $R_1$ représente un alkyle en $C_1$-$C_4$, $R_2$ représente un alkyle en $C_1$-$C_4$, un alcényle en $C_2$-$C_4$ ou un ω,ω,ω-trifluoroalkyle en $C_1$-$C_4$, X représente un alkylène en $C_1$-$C_3$, un oxy ou un thio, alk représente un alkylène en $C_2$-$C_6$ à chaîne droite, n est égal à 1 ou 2, $R_3$ représente un groupe de formule -A-$R_3'$, dans laquelle -A- représente un alkylène en $C_1$-$C_4$, le phénylène ou une liaison directe et $R_3'$ représente un alkyle en $C_1$-$C_4$, le trifluorométhyle, un carboxy ou un alcoxy en $C_1$-$C_4$ carbonyle, $R_4$ représente un carboxy ou le N-(benzènesulfonyl)carbamoyle et $R_5$ représente l'hydrogène et leurs sels.

**5.** Composés selon la revendication 4 de formule Ia où $R_1$ représente un alkyle en $C_1$-$C_4$, $R_2$ représente un alkyle en $C_1$-$C_4$, X représente un oxy, alk représente un alkylène en $C_2$-$C_6$, n est égal à 1 ou 2, $R_3$ représente un phényle substitué par un alkyle en $C_1$-$C_4$, un alcoxy en $C_1$-$C_4$, un halogène ayant un numéro atomique allant jusqu'à 35, le trifluorométhyle ou un alcoxy en $C_1$-$C_4$ carbonyle; ou représente un alkyle en $C_2$-$C_8$, un ω,ω,ω-trifluoroalkyle en $C_3$-$C_5$ ou un alcoxy en $C_1$-$C_4$ carbonylalkyle en $C_1$-$C_4$, $R_4$ représente un carboxy et $R_5$ est l'hydrogène et leurs sels.

**6.** Composés selon la revendication 4 de formule Ia où $R_1$ représente un alkyle en $C_1$-$C_4$, $R_2$ représente un alkyle en $C_1$-$C_4$, X représente un oxy, alk représente un alkylène en $C_2$-$C_6$, n est égal à 2, $R_3$ est un groupe de formule -A-$E_3'$ dans laquelle -A- représente un alkylène en $C_1$-$C_4$ ou un phénylène et $R_3'$ un alkyle en $C_1$-$C_4$, le trifluorométhyle ou un alcoxy en $C_1$-$C_4$ carbonyle, $R_4$ représente un carboxy ou le N-(benzènesulfonyl) carbamoyle et $R_5$ est l'hydrogène et leurs sels.

**7.** Composés selon l'une des revendications 1 à 4 de formule I ou Ia où X représente un oxy et leurs sels.

**8.** Composés selon l'une des revendications 1 à 5 de formule I ou Ia où n est égal à 2 et leurs sels.

**9.** Composés selon l'une des revendications 1 à 8 de formule I ou Ia où $R_3$ représente un m-alkyle en $C_1$-$C_4$ phényle ou le m-trifluorométhylphényle, $R_4$ représente le carboxy et $R_5$ l'hydrogène et leurs sels.

**10.** Un composé selon l'une des revendications 1 à 9 de formule I ou Ia où. la double liaison du reste $-(CH=CH)_n-$, voisine du reste alk, se présente dans la configuration (Z), c'est-à-dire dans la configuration cis et où l'autre double liaison du reste $-(CH=CH)_n-$ éventuellement présente se trouve dans la configuration (E), c'est-à-dire dans la configuration trans et son sel.

11. Un composé selon l'une des revendications 1 à 10 de formule I ou Ia où l'atome de C de la chaîne, lié à l'atome de soufre, présente la configuration (S) et l'atome de C de la chaîne portant le groupe hydroxy présente la configuration (R) et son sel.

12. L'acide (1R,2S)-1-hydroxy-1-(3-trifluorométhylphényl)-8-(4-acétyl-3-hydroxy-2-propylphénoxy)-octa-3(E)-,5(Z)-dién-2-yl-7-thio-4-oxo-4H-1-benzopyranne-2-carboxylique ou son sel de sodium.

13. L'acide (1R, 2S)-1-hydroxy-1-(3-méthyl-phényl)-8-(4-acétyl-3-hydroxy-2-propylphénoxy)-octa-3(E), 5(Z)-dién-2-yl-7-thio-4-oxo-4H-1-benzopyranne-2-carboxylique ou son sel de sodium.

14. L'acide (1R,2S)-1-hydroxy-1-phényl-10-(4-acétyl-3-hydroxy-2-propylphénoxy)-deca-3(E),5(Z)-dién-2-yl-7-thio-4-oxo-4H-1-benzopyranne-2-carboxylique ou son sel de sodium.

15. L'acide (4R,5S)-1,1,1-trifluoro-4-hydroxy-11- (4-acétyl-3-hydroxy-2-propylphénoxy)-undeca-6(E),8(Z)-dién-5-yl-7-thio-4-oxo-4H-1-benzopyranne-2-carboxylique ou son sel de sodium.

16. L'acide (1R,2S)-1-hydroxy-1-(3-fluoro-phényl)-10-(4-acétyl-3-hydroxy-2-propylphénoxy)-deca-3(E),5(Z)-dién-2-yl-7-thio-4-oxo-4H-1-benzopyranne-2-carboxylique ou son sel de sodium.

17. L'acide (1R,2S)-1-hydroxy-1-(3-bromophényl -10-(4-acétyl-3-hydroxy-2-propylphénoxy)-deca-3(E),5(Z)-dién-2-yl-7-thio-4-oxo-4H-1-benzopyranne-2-carboxylique ou son sel de sodium.

18. L'acide (1R,2S)-1-hydroxy-1-(3-trifluoro-méthylphényl)-10-(4-acétyl-3-hydroxy-2-propylphénoxy)-deca-3-(E),5(Z)-dién-2-yl-7-thio-4-oxo-4H-1-benzopyranne-2-carboxylique ou son sel de sodium.

19. L'acide (1R,2S)-1-hydroxy-1-(3-méthyl-phényl)-10-(4-acétyl-3-hydroxy-2-propylphénoxy)-deca-3(E),5(Z)-dién-2-yl-7-thio-4-oxo-4H-1-benzopyranne-2-carboxylique ou son sel de sodium.

20. L'acide (1R,2S)-1-hydroxy-1-(3-trifluoro-méthylphényl)-9-(4-acétyl-3-hydroxy-2-propylphénoxy)-nona-3-(E),5(Z)-dién-2-yl-7-thio-4-oxo-4H-1-benzopyranne-2-carboxylique ou son sel de sodium.

21. L'acide (1R,2S)-1-hydroxy-1-(3-trifluoro-méthylphényl)-11-(4-acétyl-3-hydroxy-2-propylphénoxy)-undeca-3(E),5(Z)-dién-2-yl-7-thio-4-oxo-4H-1-benzopyranne-2-carboxylique ou son sel de sodium.

22. L'acide (1R,2S)-1-hydroxy-1-(3-chlorophényl)-10-(4-acétyl-3-hydroxy-2-propylphénoxy)-deca-3(E), 5(Z)-dién-2-yl-7-thio-4-oxo-4H-1-benzopyranne-2-carboxylique ou son sel de sodium.

23. L'acide (1R,2S)-1-hydroxy-1-(3-méthoxyphényl)-10-(4-acétyl-3-hydroxy-2-propylphénoxy)-déca-3(E),5-(Z)-dién-2-yl-7-thio-4-oxo-4H-1-benzopyranne-2-carboxylique ou son sel de sodium.

24. L'acide (1R,2S)-1-hydroxy-1-(3-méthoxycarbonylphényl)-10-(4-acétyl-3-hydroxy-2-propylphénoxy)-deca-3(E),5(Z)-dién-2-yl-7-thio-4-oxo-4H-1-benzopyranne-2-carboxylique ou son sel de sodium.

25. Un composé selon l'une des revendications 1 à 24 destiné à être utilisé dans un procédé pour le traitement thérapeutique du corps humain ou animal.

26. Préparations pharmaceutiques contenant un composé selon l'une des revendications 1 à 24 et au moins un support pharmaceutiquement utilisable.

27. Préparations pharmaceutiques contenant un composé selon la revendication 14 et au moins un support pharmaceutiquement utilisable.

28. Préparations pharmaceutiques contenant un composé selon la revendication 18 et au moins un support pharmaceutiquement utilisable.

29. Procédé pour la préparation des composés de formule I selon la revendication 1 ou de leurs sels, caractérisé en ce que l'on fait réagir un époxyde de formule II

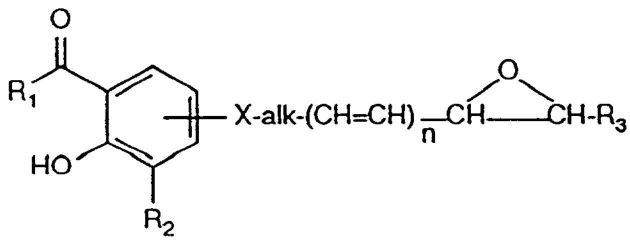

(II)

où $R_1$, $R_2$, X, alk, n et $R_3$ sont définis comme pour la formule I, avec un thiol de formule III

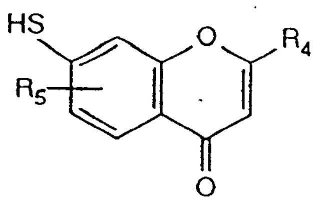

(III)

où $R_4$ et $R_5$ sont définis comme pour la formule I ou l'un de ses sels, et en ce que, si désiré, l'on transforme le composé obtenu par le procédé conforme à l'invention en un autre composé de formule I, en ce que l'on sépare le mélange de stéréoisomères obtenu par le procédé conforme à l'invention en ses composants et/ou en ce que l'on transforme le composé libre obtenu par le procédé conforme à l'invention en un sel ou le sel obtenu par le procédé conforme à l'invention en un composé libre ou en un autre sel.

**30.** Utilisation d'un composé selon l'une des revendications 1 à 24 pour la préparation de compositions pharmaceutiques.

**31.** Utilisation d'un composé selon l'une des revendications 1 à 24 pour la préparation d'un médicament antiallergique.

**Revendications pour les Etats contractants suivants : ES, GR**

**1.** Procédé pour la préparation des composés de formule I

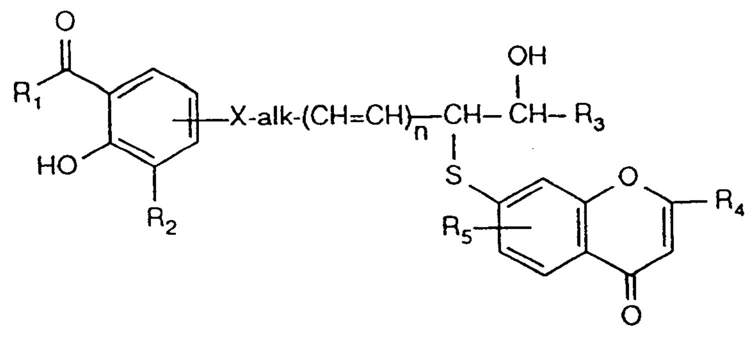

(I)

où $R_1$ représente un alkyle inférieur ou un alkyle inférieur mono-, di- ou polyfluoré, $R_2$ représente l'hydrogène, un alkyle inférieur, un alcényle inférieur ou un alkyle inférieur mono-, di- ou polyfluoré, X représente un alkylène en $C_1$-$C_3$, un oxy ou un thio, alk représente un alkylène inférieur, n est égal à 1 ou 2, $R_3$ représente un phényle non substitué ou substitué par un alkyle inférieur, un alcoxy inférieur, un halogène, un carboxy, un alcoxy inférieur carbonyle, un amino, un N-mono- ou un N,N-dialkyle inférieur amino, un carbamoyle, un N-mono-ou un N,N-dialkyle inférieur carbamoyle et/ou le trifluorométhyle; ou représente un alkyle inférieur, un alkyle inférieur mono-, di- ou trifluoré, un carboxyalkyle inférieur, un alcoxy inférieur carbonylalkyle inférieur, un carbamoylalkyle inférieur ou un N-mono- ou un

N,N-dialkyle inférieur carbamoylalkyle inférieur, $R_4$ représente un carboxy, un alcoxy inférieur carbonyle, le 5-tétrazolyle, un carbamoyle, un N-mono- ou un N,N-dialkyle inférieur carbamoyle ou un N-(benzènesulfonyl)carbamoyle non substitué ou substitué dans la partie phényle par un alkyle inférieur, un alcoxy inférieur, un halogène et/ou le trifluorométhyle et $R_5$ représente l'hydrogène ou un alkyle inférieur, les restes désignés par "inférieurs" ne comportant pas plus de 7 atomes de carbone et de leurs sels, caractérisé en ce que l'on fait réagir un époxyde de formule II

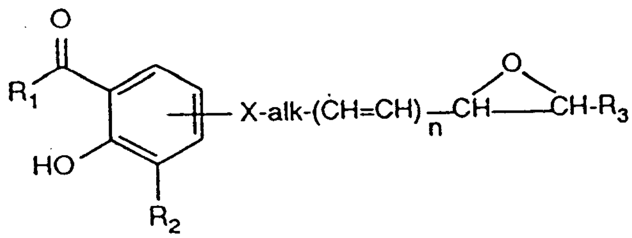

(II)

où $R_1$, $R_2$, X, alk, n et $R_3$ sont définis comme pour la formule I avec un thiol de formule III

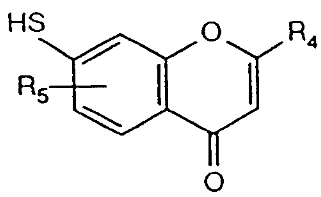

(III)

où $R_4$ et $R_5$ sont définis comme pour la formule I ou l'un de ses sels, et en ce que, si désiré, l'on transforme le composé obtenu par le procédé conforme à l'invention en un autre composé de formule I, en ce que l'on sépare le mélange de stéréoisomères obtenu par le procédé conforme à l'invention en ses composants et/ou en ce que l'on transforme le composé libre obtenu par le procédé conforme à l'invention en un sel ou le sel obtenu par le procédé conforme à l'invention en un composé libre ou en un autre sel.

**2.** Procédé selon la revendication 1 pour la préparation des composés de formule I où $R_1$ représente un alkyle en $C_1$-$C_4$ ou un $\omega,\omega,\omega$-trifluoroalkyle en $C_1$-$C_4$, $R_2$ représente l'hydrogène, un alkyle en $C_1$-$C_4$, un alcényle en $C_2$-$C_4$ ou un $\omega,\omega,\omega$-trifluoroalkyle en $C_1$-$C_4$, X représente un alkylène en $C_1$-$C_3$, un oxy ou un thio, alk représente un alkylène en $C_2$-$C_6$ à chaîne droite, n est égal à 1 ou 2, $R_3$ représente un phényle non substitué ou substitué par un alkyle en $C_1$-$C_4$, un alcoxy en $C_1$-$C_4$, un halogène ayant un numéro atomique allant jusqu'à 35, le trifluorométhyle, le carboxy et/ou un alcoxy en $C_1$-$C_4$ carbonyle; ou un alkyle en $C_1$-$C_7$, un $\omega,\omega,\omega$-trifluoroalkyle en $C_2$-$C_5$, un carboxyalkyle en $C_2$-$C_5$ ou un alcoxy en $C_1$-$C_4$ carbonylalkyle en $C_2$-$C_5$, $R_4$ représente un carboxy ou le N-(benzènesulfonyl)carbamoyle et $R_5$ représente l'hydrogène et de leurs sels.

**3.** Procédé selon l'une des revendications 1 et 2 pour la préparation des composés de formule I où $R_1$ est un alkyle en $C_1$-$C_4$ ou un $\omega,\omega,\omega$-trifluoroalkyle en $C_1$-$C_4$, $R_2$ représente un alkyle en $C_1$-$C_4$, un alcényle en $C_2$-$C_4$ ou un $\omega,\omega,\omega$-trifluoroalkyle en $C_1$-$C_4$ et dans lequel le groupe X est lié au groupe $R_1$-C(=O) en position <u>para</u> et de leurs sels.

**4.** Procédé analogue à la revendication 1 pour la préparation des composés de formule Ia

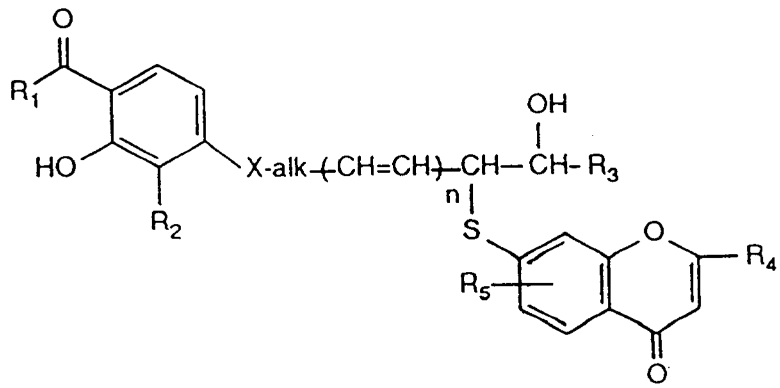

(Ia)

où $R_1$ représente un alkyle en $C_1$-$C_4$, $R_2$ représente un alkyle en $C_1$-$C_4$, un alcényle en $C_2$-$C_4$ ou un $\omega,\omega,\omega$-trifluoroalkyle en $C_1$-$C_4$, X représente un alkylène en $C_1$-$C_3$, un oxy ou un thio, alk représente un alkylène en $C_2$-$C_6$ à chaîne droite, n est égal à 1 ou 2, $R_3$ représente un groupe de formule -A-$R_3'$, dans laquelle -A- représente un alkylène en $C_1$-$C_4$, le phénylène ou une liaison directe et $R_3'$ représente un alkyle en $C_1$-$C_4$, le trifluorométhyle, un carboxy ou un alcoxy en $C_1$-$C_4$ carbonyle, $R_4$ représente un carboxy ou le N-(benzènesulfonyl)carbamoyle et $R_5$ représente l'hydrogène et de leurs sels.

**5.** Procédé selon la revendication 4 pour la préparation des composés de formule Ia où $R_1$ représente un alkyle en $C_1$-$C_4$, $R_2$ représente un alkyle en $C_1$-$C_4$, X représente un oxy, alk représente un alkylène en $C_2$-$C_6$,n est égal à 1 ou 2, $R_3$ représente un phényle substitué par un alkyle en $C_1$-$C_4$, un alcoxy en $C_1$-$C_4$, un halogène ayant un numéro atomique allant jusqu'à 35, le trifluorométhyle ou un alcoxy en $C_1$-$C_4$ carbonyle; ou représente un alkyle en $C_2$-$C_8$, un $\omega,\omega,\omega$-trifluoroalkyle en $C_3$-$C_5$ ou un alcoxy en $C_1$ -$C_4$ carbonylalkyle en $C_1$-$C_4$, $R_4$ représente un carboxy et $R_5$ est l'hydrogène et de leurs sels.

**6.** Procédé selon la revendication 4 pour la préparation des composés de formule Ia où $R_1$ représente un alkyle en $C_1$-$C_4$, $R_2$ représente un alkyle en $C_1$-$C_4$, X représente un oxy, alk représente un alkylène en $C_{2-6}$,n est égal à 2, $R_3$ est un groupe de formule -A-$R_3'$ dans laquelle -A- représente un alkylène en $C_1$-$C_4$ ou un phénylène et $R_3'$ un alkyle en $C_1$-$C_4$, le trifluorométhyle ou un alcoxy en $C_1$-$C_4$ carbonyle, $R_4$ représente un carboxy ou le N-(benzènesulfonyl)carbamoyle et $R_5$ est l'hydrogène et de leurs sels.

**7.** Procédé selon l'une des revendications 1 à 4 pour la préparation des composés de formule I ou Ia où X représente un oxy et de leurs sels.

**8.** Procédé selon l'une des revendications 1 à 5 pour la préparation des composés de formule I ou Ia où n est égal à 2 et de leurs sels.

**9.** Procédé selon l'une des revendications 1 à 8 pour la préparation des composés de formule I ou Ia où $R_3$ représente un m-alkyle en $C_1$-$C_4$ phényle ou le m-trifluorométhylphényle, $R_4$ représente le carboxy et $R_5$ l'hydrogène et de leurs sels.

**10.** Procédé selon l'une des revendications 1 à 9 pour la préparation des composés de formule I ou Ia la double liaison du reste $-(CH=CH)_n-$, voisine du reste alk, se présente dans la configuration (Z), c'est-à-dire dans la configuration cis et où l'autre double liaison du reste $-(CH=CH)_n-$ éventuellement présente se trouve dans la configuration (E), c'est-à-dire dans la configuration trans et de leurs sels.

**11.** Procédé selon l'une des revendications 1 à 10 pour la préparation des composés de formule I ou Ia où l'atome de C de la chaîne lié à l'atome de soufre présente la configuration (S) et l'atome de C de la chaîne portant le groupe hydroxy présente la configuration (R) et de leurs sels.

**12.** Procédé selon la revendication 1 pour la préparation de l'acide (1R,2S)-1-hydroxy-1-(3-trifluorométhylphényl)-8-(4-acéty-3-hydroxy-2-propylphénoxy)-octa-3 (E),5(Z)-dién-2-yl-7-thio-4-oxo-4H-1-benzopyrane-2-carboxylique ou de son sel de sodium.

**13.** Procédé selon la revendication 1 pour la préparation de l'acide (1R,2S)-1-hydroxy-1-(3-méthylphényl)-8-(4-acétyl-3-hydroxy-2-propylphénoxy)-octa-3(E),5(Z)-dién-2-yl-7-thio-4-oxo-4H-benzopyranne-2-carboxylique ou de son sel de sodium.

**14.** Procédé selon la revendication 1 pour la préparation de l'acide (1R,2S)-1-hydroxy-1-phényl-10-(4-acétyl-3-hydroxy-2-propylphénoxy)-deca-3(E),5(Z)-dién-2-yl-7-thio-4-oxo-4H-1-benzopyranne-2-carboxylique ou de son sel de sodium.

**15.** Procédé selon la revendication 1 pour la préparation de l'acide (4R,5S)-1,1,1-trifluoro-4-hydroxy-11-(4-acétyl-3-hydroxy-2-propylphénoxy)-undeca-6(E),8(Z)-dién-5-yl-7-thio-4-oxo-4H-1-benzopyranne-2-carboxylique ou de son sel de sodium.

**16.** Procédé selon la revendication 1 pour la préparation de l'acide (1R,2S)-1-hydroxy-1-(3-fluorophényl)-10-(4-acétyl-3-hydroxy-2-propylphénoxy)-deca-3(E),5(Z)-dién-2-yl-7-thio-4-oxo-4H-1-benzopyranne-2-carboxylique ou de son sel de sodium.

**17.** Procédé selon la revendication 1 pour la préparation de l'acide (1R,2S)-1-hydroxy-1-(3-bromophényl)-10-(4-acétyl-3-hydroxy-2-propylphénoxy)-deca-3(E),5(Z)-dién-2-yl-7-thio-4-oxo-4H-1-benzopyranne-2-carboxylique ou de son sel de sodium.

**18.** Procédé selon la revendication 1 pour la préparation de l'acide (1R,2S)-1-hydroxy-1-(3-trifluorométhylphényl)-10-(4-acétyl-3-hydroxy-2-propylphénoxy)-deca-3(E),5(Z)-dién-2-yl-7-thio-4-oxo-4H-1-benzopyranne-2-carboxylique ou de son sel de sodium.

**19.** Procédé selon la revendication 1 pour la préparation de l'acide (1R,2S)-1-hydroxy-1-(3-méthylphényl)-10-(4-acétyl-3-hydroxy-2-propylphénoxy)-deca-3(E), 5(Z)-dién-2-yl-7-thio-4-oxo-4H-1-benzopyranne-2-carboxylique ou de son sel de sodium.

**20.** Procédé selon la revendication 1 pour la préparation de l'acide (1R,2S)-1-hydroxy-1-(3-trifluorométhylphényl)-9-(4-acétyl-3-hydroxy-2-propylphénoxy)-nona-3(E),5(Z)-dién-2-yl-7-thio-4-oxo-4H-1-benzopyranne-2-carboxylique ou de son sel de sodium.

**21.** Procédé selon la revendication 1 pour la préparation de l'acide (1R,2S)-1-hydroxy-1-(3-trifluorométhylphényl)-11-(4-acétyl-3-hvdroxy-2-propylphénoxy)-undeca-3(E),5(Z)-dién-2-yl-7-thio-4-oxo-4H-1-benzopyranne-2-carboxylique ou de son sel de sodium.

**22.** Procédé selon la revendication 1 pour la préparation de l'acide (1R,2S)-1-hydroxy-1-(3-chloro-phényl)-10-(4-acétyl-3-hydroxy-2-propylphénoxy)-deca-3(E),5(Z)-dién-2-yl-7-thio-4-oxo-4H-1-benzopyranne-2-carboxylique ou de son sel de sodium.

**23.** Procédé selon la revendication 1 pour la préparation de l'acide (1R,2S)-1-hydroxy-1-(3-méthoxyphényl)-10-(4-acétyl-3-hydroxy-2-propylphénoxy)-deca-3(E), 5(Z)-dién-2-yl-7-thio-4-oxo-4H-1-benzopyranne-2-carboxylique ou de son sel de sodium.

**24.** Procédé selon la revendication 1 pour la préparation de l'acide (1R,2S)-1-hydroxy-1-(3-méthoxycarbonylphényl)-10-(4-acétyl-3-hydroxy-2-propylphénoxy)-deca-3(E),5(Z)-dién-2-yl-7-thio-4-oxo-4H-1-benzopyranne-2-carboxylique ou de son sel de sodium.

**25.** Procédé pour la préparation de compositions pharmaceutiques, caractérisé en ce que l'on mélange un composé obtenu selon l'une des revendications 1 à 24 avec des adjuvants pharmaceutiques usuels.